(19) Europäisches Patentamt
European Patent Office
Office européen des brevets

(11) Veröffentlichungsnummer: **0 008 421**
**B1**

(12) # EUROPÄISCHE PATENTSCHRIFT

(45) Veröffentlichungstag der Patentschrift: **08.02.84**

(51) Int. Cl.$^3$: **C 07 D 243/24,** C 07 C 127/19, A 61 K 31/55

(21) Anmeldenummer: **79102899.6**

(22) Anmeldetag: **10.08.79**

(54) **Benzodiazepin-Derivate, Verfahren zu deren Herstellung und diese enthaltende Arzneimittel.**

(30) Priorität: **11.08.78 CH 8563/78**
**06.06.79 CH 5270/79**
**05.07.79 CH 6296/79**

(43) Veröffentlichungstag der Anmeldung:
**05.03.80 Patentblatt 80/5**

(45) Bekanntmachung des Hinweises auf die Patenterteilung:
**08.02.84 Patentblatt 84/6**

(84) Benannte Vertragsstaaten:
**AT BE CH DE FR GB IT LU NL SE**

(56) Entgegenhaltungen:
**FR - A - 2 150 729**
**FR - A - 2 252 103**
**FR - M - 2 538**

(73) Patentinhaber: **F. HOFFMANN-LA ROCHE & CO.**
**Aktiengesellschaft**
**CH-4002 Basel (CH)**

(72) Erfinder: **Fischli, Albert Eduard, Prof. Dr.**
**Am Ausserberg 20**
**CH-4125 Riehen (CH)**
Erfinder: **Szente, André, Dr.**
**Burgstrasse 38**
**CH-4125 Riehen (CH)**

(74) Vertreter: **Lederer, Franz, Dr. et al,**
**Patentanwälte Dr. Franz Lederer Reiner F. Meyer**
**Lucile-Grahn-Strasse 22**
**D-8000 München 80 (DE)**

**EP 0 008 421 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Benzodiazepin-Derivate, Verfahren zu deren Herstellung und diese enthaltende Arzneimittel.

Die vorliegende Erfindung betrifft Benzodiazepin-Derivate. Im speziellen betrifft sie Benzodiazepin-Derivate der allgemeinen Formel (I)

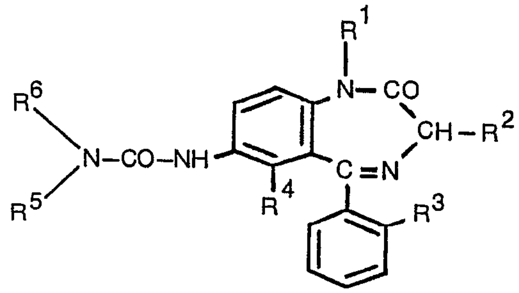

I

worin $R_1$ ($C_1$—$C_7$)-Alkyl, $R^2$ Wasserstoff oder ($C_1$—$C_7$)-Alkyl, $R^3$ Halogen und $R^4$ Wasserstoff oder Halogen bedeuten und entweder $R^5$ Wasserstoff oder ($C_1$—$C_7$)-Alkyl und $R^6$ ($C_1$—$C_7$)-Alkyl, Hydroxy-($C_2$—$C_7$)-alkyl oder ($C_1$—$C_7$)-Alkanoyloxy-($C_2$—$C_7$)-alkyl bedeuten oder $R^5$ Wasserstoff und $R^6$ einen gegebenenfalls durch Halogen oder ($C_1$—$C_7$)-Alkoxy monosubstituierten Phenyl- oder Phenyl-($C_1$—$C_7$)-alkylrest bedeuten oder $R^5$ und $R^6$ zusammen mit dem Stickstoffatom einen 3- bis 7-gliedrigen Heterocyclus ohne zusätzliches Heteroatom oder einen N-Methylpiperazin-, Thiazolindin-, Morpholin- oder Thiomorpholinrest bedeuten, und pharmazeutisch akzeptable Säureadditionssalze von Benzodiazepin-Derivaten der Formel I.

Gegenstand der vorliegenden Erfindung sind Benzodiazepin-Derivate der obigen allgemeinen Formel I und pharmazeutisch akzeptable Säureadditionssalze davon, die Herstellung dieser Verbindungen und Arzneimittel, enthaltend eine oder mehrere Verbindungen der allgemeinen Formel I oder pharmazeutisch akzeptable Säureadditionssalze davon. In den Dokumenten FR—A—2.150.729, FR—A—2.252.103 und FR—M—2.538 werden u.a. 1,4-Benzodiazepin-2-on-Derivate beschrieben, welche in der 7-Stellung einen stickstoffhaltigen Substituenten aufweisen und welche anticonvulsive, muskelrelaxierende und sedierende Eigenschaften besitzen.

Der Ausdruck "($C_1$—$C_7$)-Alkyl", für sich allein genommen oder in Kombinationen, bezeichnet geradkettige oder verzweigte gesättigte Kohlenwasserstoffreste mit 1 bis hochstens, 7, vorzugsweise mit 1 bis hochstens 4 Kohlenstoffatomen, wie Methyl, Aethyl, n-Propyl, Isopropyl, n-Butyl, tert.-Butyl. Der Ausdruck "Hydroxy-($C_2$—$C_7$)-alkyl" umfasst Reste wie 2-Hydroxyäthyl, 3-Hydroxy-2-propyl und dergleichen. Bei den ($C_1$—$C_7$)-Alkanoyloxy-($C_2$—$C_7$)-alkylgruppen handelt es sich um durch ($C_1$—$C_7$)-Alkanoyloxy substituierte ($C_2$—$C_7$)-Alkylgruppen, beispielsweise um Acetoxy-($C_2$—$C_7$)-alkylgruppen, wie 2-Acetoxyäthyl. Als durch Halogen oder ($C_1$—$C_7$)-Alkoxy monosubstituierte, Phenylreste kommen in erster Linie p-Chlorphenyl oder p-Methoxy-phenyl in Betracht. Der Ausdruck "Phenyl-($C_1$—$C_7$)-alkyl" bezeichnet einen durch Phenyl substituierten ($C_1$—$C_7$)-Alkylrest, wie z.B. Benzyl. Wenn $R^5$ und $R^6$ zusammen mit dem Stickstoffatom einen 3- bis 7-gliedrigen Heterocyclus ohne zusätzliches Heteroatom bedeuten, so kommen in erster Linie Aziridin- und Pyrrolidin reste in Betracht.

Unter den Verbindungen der Formel I sind diejenigen bevorzugt, worin $R^2$ Wasserstoff bedeutet. Die bevorzugte Bedeutungsmöglichkeit von $R^3$ in Formel I ist Fluor, und für das Symbol $R^4$ in Formel I sind die Bedeutungsmöglichkeiten Wasserstoff und Chlor bevorzugt.

Eine ganz besonders bevorzugte Verbindung im Rahmen der vorliegenden Erfindung ist der 1-[5-(o-Fluorphenyl)-2,3-dihydro-1-methyl-2-oxo-1H-1,4-benzodiazepin-7-yl]-3-(2-hydroxyäthyl)harnstoff. Weitere im Rahmen der vorliegenden Erfindung besonders bevorzugte, von der allgemeinen Formel I umfasste Verbindungen sind

1-[5-(o-Fluorphenyl)-2,3-dihydro-1-methyl-2-oxo-1H-1,4-benzodiazepin-7-yl]-3-methylharnstoff,

1-[5-(o-Fluorphenyl)-2,3-dihydro-1-methyl-2-oxo-1H-1,4-benzodiazepin-7-yl]-3-(2-hydroxy-1-methyläthyl)harnstoff,

N-[5-(o-Fluorphenyl)-2,3-dihydro-1-methyl-2-oxo-1H-1,4-benzodiazepin-7-yl]-1-pyrrolidincarboxamid,

1-[6-Chlor-5-(o-fluorphenyl)-2,3-dihydro-1-methyl-2-oxo-1H-1,4-benzodiazepin-7-yl]-3-methylharnstoff,

1-n-Butyl-3-[6-chlor-5-(o-fluorphenyl)-2,3-dihydro-1-methyl-2-oxo-1H-1,4-benzodiazepin-7-yl]harnstoff und

3-[6-Chlor-5-(o-fluorphenyl)-2,3-dihydro-1-methyl-2-oxo-1H-1,4-benzodiazepin-7-yl]-1-äthyl-1-methylharnstoff, ferner

1-tert.-Butyl-3-[5-(o-fluorphenyl)-2,3-dihydro-1-methyl-2-oxo-1H-1,4-benzodiazepin-7-yl]harnstoff,

1-[5-(o-Fluorphenyl)-2,3-dihydro-1-methyl-2-oxo-1H-1,4-benzodiazepin-7-yl]-3-(p-methoxyphenyl)harnstoff,

1-Benzyl-3-[5-(o-fluorphenyl)-2,3-dihydro-1-methyl-2-oxo-1H-1,4-benzodiazepin-7-yl]harnstoff,

3-[5-(o-Fluorphenyl)-2,3-dihydro-1-methyl-2-oxo-1H-1,4-benzodiazepin-7-yl]-1,1-dimethylharnstoff,

1-Aethyl-3-[5-(o-fluorphenyl)-2,3-dihydro-1-methyl-2-oxo-1H-1,4-benzodiazepin-7-yl]-1-methylharnstoff,

N-[5-(o-Fluorphenyl)-2,3-dihydro-1-methyl-2-oxo-1H-1,4-benzodiazepin-7-yl]-1-aziridincarboxamid,

1-[6-Chlor-5-(o-fluorphenyl)-2,3-dihydro-1-methyl-2-oxo-1H-1,4-benzodiazepin-7-yl]-3-äthylharnstoff,

1-[6-Chlor-5-(o-fluorphenyl)-2,3-dihydro-1-methyl-2-oxo-1H-1,4-benzodiazepin-7-yl]-3-(2-hydroxyäthyl)harnstoff und

3-[6-Chlor-5-(o-fluorphenyl)-2,3-dihydro-1-methyl-2-oxo-1H-1,4-benzodiazepin-7-yl]-1,1-diäthylharnstoff.

Die Benzodiazepin-Derivate der allgemeinen Formel I und deren pharmazeutisch akzeptable Säureadditionssalze können erfindungsgemäss dadurch heregestellt werden, dass man

a) ein in situ hergestelltes Benzodiazepin-Derivat der allgemeinen Formel (II)

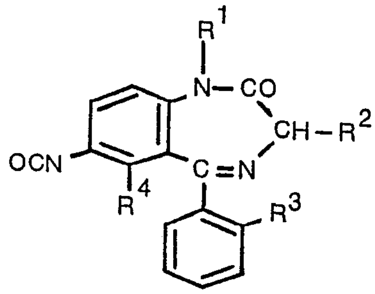

II

worin $R^1$, $R^2$, $R^3$ und $R^4$ oblige Bedeutung besitzen, mit einer Aminoverbindung der allgemeinen Formel (III)

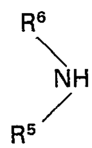

III

worin $R^5$ und $R^6$ obige Bedeutung besitzen, umsetzt, oder

b) eine in situ hergestellte Verbindung der allgemeinen Formel IV

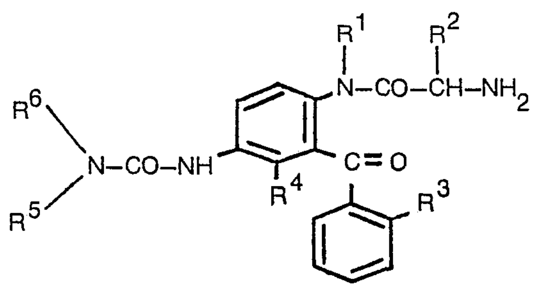

IV

worin $R^1$, $R^2$, $R^3$, $R^4$, $R^5$ und $R^6$ obige Bedeutung besitzen, in Lösung cyclisiert, oder

c) ein Benzodiazepin-Derivat der allgemeinen Formel (V)

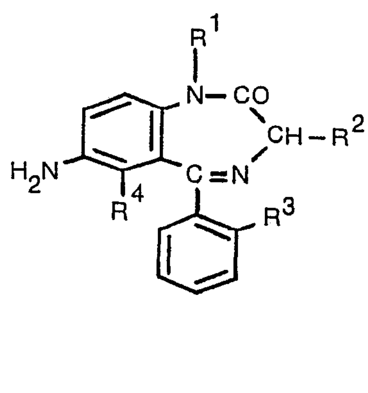

V

worin $R^1$, $R^2$, $R^3$ und $R^4$ obige Bedeutung besitzen, mit einem Halogenid der allgemeinen Formel VI

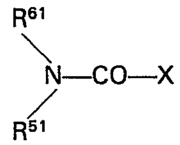

VI

worin X Halogen bedeutet und entweder $R^{51}$ ($C_1$—$C_7$)-Alkyl und $R^{61}$ ($C_1$—$C_7$)-Alkyl oder ($C_1$—$C_7$)-Alkanoyloxy-($C_2$—$C_7$)-alkyl bedeuten oder $R^{51}$ und $R^{61}$ zusammen mit dem Stickstoffatom eine 3- bis 7-gliedrigen Heterocyclus ohne zusätzliches Heteroatom oder einen N-Methylpiperazin-, Thiazolidin-, Morpholin-, oder Thiomorpholinrest bedeuten, umsetzt, oder

d) ein Benzodiazepin-Derivat der obigen allgemeinen Formel (V) mit einem Isocyanat der allgemeinen Formel VII

$$R^{62}\text{—NCO} \qquad \text{VII}$$

worin $R^{62}$ ($C_1$—$C_7$)-Alkyl, ($C_1$—$C_7$)-Alkanoyloxy-($C_2$—$C_7$)-alkyl, oder einen gegebenenfalls durch Halogen oder ($C_1$—$C_7$)-Alkoxy monosubstituierten Phenyl-oder Phenyl-($C_1$—$C_7$)-alkylrest bedeutet, umsetzt, oder

e) aus einem Benzodiazepin-Derivat der allgemeinen Formel (VIII)

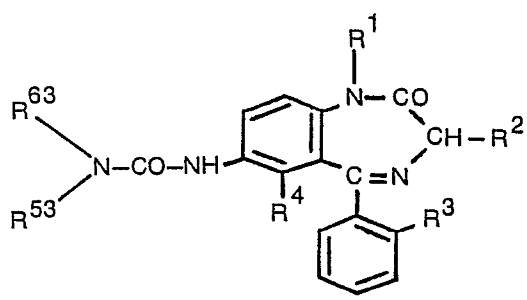

VIII

worin $R^1$, $R^2$, $R^3$ und $R^4$ obige Bedeutung besitzen und entweder $R^{53}$ eine Schutzgruppe und $R^{63}$ ($C_1$—$C_7$)-Alkyl, einen gegebenenfalls durch Halogen oder ($C_1$—$C_7$)-Alkoxy monosubstituierten Phenyl- oder Phenyl-($C_1$—$C_7$)-alkylrest oder einen Rest der Formel (IX)

$$\text{—A—O—Y} \qquad \text{IX}$$

bedeuten, wobei A ($C_2$—$C_7$)-Alkylen und Y eine Schutzgruppe bedeuten, oder $R^{53}$ Wasserstoff oder ($C_1$—$C_7$)-Alkyl und $R^{63}$ einen Rest der obigen Formel IX bedeuten, die Schutzgruppe (n) entfernt, oder

f) ein Benzodiazepin-Derivat der allgemeinen Formel (X)

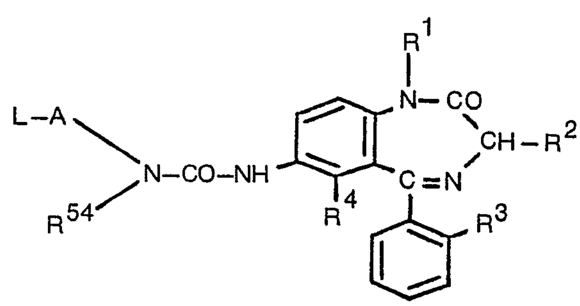

X

worin $R^1$, $R^2$, $R^3$, $R^4$ und A obige Bedeutung besitzen und $R^{54}$ Wasserstoff oder ($C_1$—$C_7$)-Alkyl und L eine Abgangsgruppe bedeuten, in die entsprechende Hydroxy- oder ($C_1$—$C_7$)-Alkanoyloxy verbindung überführt, oder

g) ein Benzodiazepin-Derivat der allgemeinen Formel (Ia)

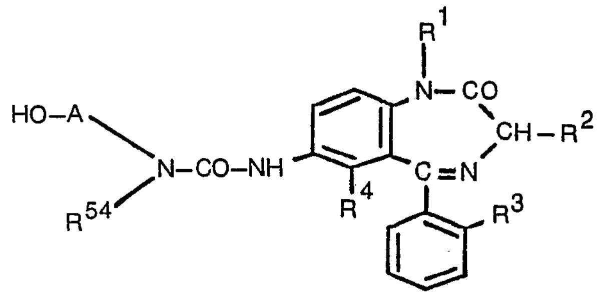

Ia

worin $R^1$, $R^2$, $R^3$, $R^4$, $R^{54}$ und A obige Bedeutung besitzen, $(C_1—C_7)$-alkanoyliest, oder

h) In einem Benzodiazepin-Derivat der allgemeinen Formel (Ib)

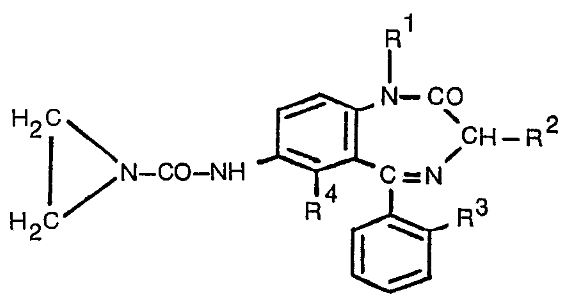

Ib

worin $R^1$, $R^2$, $R^3$ und $R^4$ obige Bedeutung besitzen, den Aziridinring hydrolytisch öffnet, oder

i) ein Benzodiazepin-Derivat der allgemeinen Formel (I) in ein pharmazeutisch akzeptables Säureadditionssalz überführt.

Nach einem ersten Verfahrensaspekt können die Benzodiazepin-Derivate der allgemeinen Formel (I) demnach aus Benzodiazepin-Derivaten der allgemeinen Formel (II) und Aminoverbindungen der allgemeinen Formel (III) hergestellt werden. Zweckmässigerweise geht man dabei so vor, dass man das zum Einsatz vorgesehene Benzodiazepin-Derivat der allgemeinen Formel (II) kurz oder unmittelbar vor der Umsetzung mit der Aminoverbindung der allgemeinen Formel (III) auf die weiter unten beschriebene Weise aus einem entsprechenden Benzodiazepin-Derivat der allgemeinen Formel (V) herstellt und das betreffende Benzodiazepin-Derivat der allgemeinen Formel (II) nicht in isolierter Form in die Reaktion einbringt, sondern in der Lösung, in welcher es vorgängig aus dem entsprechenden Benzodiazepin-Derivat der allgemeinen Formel (V) hergestellt worden ist.

Zu der erwähnten, das Benzodiazepin-Derivat der allgemeinen Formel (II) enthaltenden Lösung kann man dann eine Aminoverbindung der allgemeinen Formel (III) geben. Hierbei kann die Aminoverbindung der allgemeinen Formel (III) in Form einer Lösung oder auch ohne Lösungsmittel verwendet werden; falls es sich um eine bei Raumtemperatur gasförmige Aminoverbindung handelt (was beispielsweise für Methylamin zutrifft) kann man sie als Gas in die erwähnte, das Benzodiazepin-Derivat der allgemeinen Formel (II) enthaltende Lösung einleiten.

Andererseits ist es auch möglich, die Aminoverbindung der allgemeinen Formel (III) vorzulegen, zweckmässigerweise in Form einer Lösung, und dann die erwähnte, das Benzodiazepin-Derivat der allgemeinen Formel II enthaltende Lösung zuzugeben.

In vielen Fällen ist es zweckmässig, die Aminoverbindung der allgemeinen Formel III im Ueberschuss einzusetzen.

Als Lösungsmittel für den vorliegenden Verfahrensaspekt eignen sich verschiedene, unter den Reaktionsbedingungen inerte organische Lösungsmittel, beispielsweise halogenierte Kohlenwasserstoffe, wie Dichloräthan, Methylenchlorid, Chloroform, o-Dichlorbenzol; Aether, wie Tetrahydrofuran, Dioxan, Dimethoxyäthan, Diäthylenglykoldimethyläther.

Die Umsetzung der Verbindungen der Formeln (II) und (III) erfolgt zweckmässigerweise bei Raumtemperatur oder Temperaturen unterhalb davon. Es ist noch zu beachten, dass man dann, wenn man eine Lösung des Benzodiazepin-Derivats der allgemeinen Formel (II) vorlegt, die Aminoverbindung der allgemeinen Formel (III) innerhalb kurzer Zeit zugeben sollte, wogegen im umgekehrten Fall, d.h. wenn man die Aminoverbindung der allgemeinen Formel (III) vorlegt und dann die Lösung des Benzodiazepin-Derivats der allgemeinen Formel (II) zugibt, die Geschwindigkeit der Zugabe keine wesentliche Rolle spielt.

Nach einem zweiten Verfahrensaspekt können die Benzodiazepin-Derivate der allgemeinen Formel (I) dadurch hergestellt werden, dass man eine in situ hergestellte Verbindung der allgemeinen Formel (IV) in Lösung cyclisiert. Die Cyclisation einer Verbindung der allgemeinen Formel (IV) erfolgt ziemlich leicht; sie kann gegebenenfalls durch längeres stehenlassen und/oder durch Anwendung von

Wärme beschleunigt werden. Man kann in neutralem, alkalischem oder in saurem Milieu arbeiten; die alkalische Verfahrensführung ist jedoch bevorzugt. Die Cyclisation erfolgt zweckmässigerweise in einem inerten organischen Lösungsmittel, z.B. in Kohlenwasserstoffen, wie Benzol, Toluol, in chlorierten Kohlenwasserstoffen, wie Chloroform, Methylenchlorid, in Aether, wie Dioxan. Für die Cyclisation der Verbindungen der allgemeinen Formel IV eignen sich Temperaturen im Bereich zwischen Raumtemperatur und 150°C, natürlich unter Berücksichtigung des eingesetzten Lösungsmittels.

Nach einem weiteren Verfahrensaspekt können Benzodiazepin-Derivate der allgemeinen Formel (I) erfindungsgemäss dadurch hergestellt weden, dass man ein Benzodiazepin-Derivat der allgemeinen Formel (V) mit einem Halogenid der allgemeinen Formel (VI) umsetzt. Die Umsetzung der Verbindungen der Formeln (V) und (VI) erfolgt in Gegenwart eines säurebindenden Mittels, beispielsweise einer anorganischen Base, wie Kaliumcarbonat, Natriumcarbonat, oder einer organischen Base, z.B. einer tertiären Aminoverbindung, wie Triäthylamin, N-Aethyl-diisopropylamin, Chinuclidin.

Die Umsetzung der Verbindungen der Formeln (V) und (VI) erfolgt zweckmässigerweise bei Raumtemperatur oder unterhalb davon; sie verläuft ziemlich langsam und dauert in der Regel mehrere Tage.

Nach einem weiteren Verfahrensaspekt können Benzodiazepin-Derivate der allgemeinen Formel (I) erfindungsgemäss dadurch hergestellt werden, dass man ein Benzodiazepin-Derivat der allgemeinen Formel (V) mit einem Isocyanat der allgemeinen Formel (VII) umsetzt. Diese Umsetzung erfolgt zweckmässigerweise in einem unter den Reaktionsbedingungen inerten organischen Lösungsmittel, beispielsweise in einem halogenierten Kohlenwasserstoff, wie Methylenchlorid, Dichloräthan, Chloroform, o-Dichlorbenzol; in einem Aether, wie Tetrahydrofuran, Dioxan, Dimethoxyäthan, Diäthylenglykoldimethyläther. In manchen Fällen erweist es sich als günstig, die Reaktion in Gegenwart einer Katalytisch wirkenden geringen Menge einer Base durchzuführen, beispielsweise in Gegenwart einer tertiären Aminoverbindung, wie Triäthylamin, N-Aethyl-diisopropylamin, Chinuclidin. Die Temperatur ist für die Umsetzung der Verbindungen der Formlen (V) und (VII) nicht kritisch, und man kann demnach bei Raumtemperatur, unterhalb der Raumtemperatur oder oberhalb der Raumtemperatur arbeiten, beispielsweise bei Rückflusstemperatur.

Nach einem weiteren Verfahrensaspekt können Benzodaizepin-Derivate der allgemeinen Formel (I) erfindungsgemäss dadurch hergestellt werden, dass man aus einem Benzodiazepin-Derivat der allgemeinen Formel (VIII) die Schutzgruppe bzw. die Schutzgruppen entfernt. Als Stickstoff-Schutzgruppen eignen sich für die Zwecke der vorliegenden Erfindung in erster Linie Acylgruppen, vorzugsweise leicht abspaltbare Alkoxycarbonyl- oder Aralkoxycarbonylgruppen, insbesondere die tert. -Butoxycarbonylgruppe, die Benzyloxycarbonylgruppe, ferner auch leicht abspaltbare Aralkylgruppen, wie die Benzylgruppe. Als Sauerstoff-Schutzgruppen eignen sich einerseits Acylgruppen oder Aralkylgruppen, wie sie soeban als Stickstoff-Schutzgruppen erwähnt worden sind, andererseits aber auch Ketal-Schutzgruppen, wie Tetrahydropyranyl, 2-Methoxy-2-propyl, Methoxymethyl, $\beta$-Methoxy-äthoxymethyl, leicht abspaltbare Alkylgruppen, wie tert. -Butyl, oder Alkanoylgruppen, wie Acetyl.

Die Entfernung der Schutzgruppe bzw. der Schutzgruppen aus den Benzodiazepin-Derivaten der allgemeinen Formel (VIII) erfolgt nach an sich bekannten Methoden, wobei natürlich die Natur der zu entfernenden Schutzgruppe bzw. Schutzgruppen für die Wahl der zur Anwendung gelangenden Methode bzw. Methoden in Betracht, gezogen werden muss. Ebenfalls zu beachten ist natürlich, dass nur solche Methoden verwendet werden können, welche die Schutzgruppe bzw. Schutzgruppen selektiv entfernen, ohne dass andere im Molekül vorhandene Struckturelemente in Mitleidenschaft gezogen werden.

Die weiter oben als Beispiele für in Betracht kommende Schutzgruppen erwähnten Reste können je nach ihrer Natur hydrogenolytisch und/oder hydrolytisch abgespalten werden. So können z.B. die Benzyloxycarbonylgruppe und die tert.-Butoxycarbonylgruppe unter selektiven sauren Bedingungen abgespalten werden, z.B. durch Behandeln mit einem Gemisch von Bromwasserstoff und Eisessig, ebenso durch Behandlung mit Bortrifluorid oder Bortribromid in einem inerten organischen Lösungsmittel, wie Dichlormethan. Die tert.-Butoxycarbonylgruppe kann auch durch Behandeln mit Chlorwasserstoff in einem inerten organischen Lösungsmittel, wie Dioxan, Tetrahydrofuran, oder durch Behandeln mit Trifluoressigsäure abgespalten werden. Die Tetrahydropyranylgruppe lässt sich unter milden sauren wässrigen Bedingungen abspalten, beispielsweise durch Behandlung mit verdünnter wässriger Mineralsäure unter milden Bedingungen. Die tert.-Butylgruppe kann z.B. mittels Trifluoressigsäure abgespalten werden. Die Benzylgruppe kann durch katalytische Hydrierung, z.B. über Palladium/Kohle entfernt werden. Die Acetylgruppe kann unter milden alkalischen Bedingungen abgespalten werden, z.B. mit einer Lösung eines Natriumalkoholats im entsprechenden Alkohol (wie methanolisches Natriummethylat).

Nach einem weiteren Verfahrensaspekt können Benzodiazepin-Derivate der allgemeinen Formel (I) erfindungsgemäss dadurch hergestellt werden, dass man Benzodiazepin-Derivate der allgemeinen Formel (X) in entsprechende Hydroxy- oder ($C_1$—$C_7$)-Alkanoyloxy- Verbindungen überführt. Die in Formel X durch das Symbol L wiedergegebene Abgangsgruppe ist ein Halogenatom, insbesondere Chlor, Brom oder Jod; eine Arylsulfonyloxyrest wie Tosyloxy, ein Alkylsulfonyloxyrest, wie Mesyloxy, oder eine quartäre Ammoniumgruppen wie der Trimethylammoniumrest.

Die Ueberführung eines Benzodiazepin-Derivats der allgemeinen Formel (X) in die entsprechende Hydroxyverbindung kann z.B. durch Solvolyse in einem wasserhaltigen System erfolgen,

zweckmässigerweise in einem Gemisch eines aromatischen Kohlenwasserstoffes, wie Benzol, und Wasser, in Gegenwart eines quartären Ammoniumsalzes, wie Tetrabutylammoniumbromid, un bei einer Temperatur zwischen Raumtemperatur und Rückflusstemperatur des Reaktionsgemisches.

Die Ueberführung eines Benzodiazepin-Derivats der allgemeinen Formel X in eine entsprechende ($C_1$—$C_7$)-Alkanoyloxyverbindung erfolgt durch Umsetzung mit einem Alkali- oder Erdalkalimetallsalz der der einzuführenden ($C_1$—$C_7$)-Alkanoyloxygruppe entsprechenden organischen Säure, beispielsweise mit einem Alkalimetallsalz einer ($C_1$—$C_7$)-Alkancarbonsäure, wie Kaliumacetat. Die Umsetzung erfolgt in einem unter den Reaktionsbedingungen inerten organischen Lösungsmittel oder Lösungsmittelgemisch, beispielsweise in Dimethylformamid, Dimethylsulfoxid, Hexamethylphosphorsäuretriamid; bei der Herstellung einer Acetoxyverbindung kann auch Eisessig als Lösungsmittel dienen. Besonders zweckmässig ist eine Lösung von 18-crown-6 in Acetonitril. Zweckmässigerweise arbeitet man bei Temperaturen zwischen Raumtemperatur und Rückflusstemperatur des Reaktionsgemisches.

Nach einem weiteren Verfahrensaspekt können Benzodiazepin-Derivate der allgemeinen Formel (I) dadurch hergestellt werden, dass man Benzodiazepin-Derivate der allgemeinen Formel (Ia) ($C_1$—$C_7$)-alkanoyliert. Diese Reaktion kann mit jedem geeigneten ($C_1$—$C_7$)-Alkanoylierungsmittel durchgeführt werden, beispielsweise mit einem Säureanhydrid, wie Essigsäureanhydrid oder mit einem Säurehalogenid, wie Acetylchlorid. Die Reaktionsbedingungen können von jedem Fachmann je nach dem zum Einsatz vorgesehenen ($C_1$—$C_7$)-Alkanoyliertungsmittel leicht ausgewählt werden. Beispielsweise kann man bei Raumtemperatur oder oberhalb oder unterhalb der Raumtemperatur arbeiten. Die ($C_1$—$C_7$)-Alkanoylierung erfolgt zweckmässigerweise in einem unter den Reaktionsbedingungen inerten organischen Lösungsmittel, beispielsweise in Acetonitril, in Methylenchlorid, Dichloräthan, in Tetrahydrofuran, Dimethoxyäthan, und in Gegenwart eines säurebindenden Mittels, beispielsweise einer anorganischen Base, wie Kaliumcarbonat, Natriumcarbonat oder einer tertiären organischen Aminoverbindung, wie Triäthylamin, N-Aethyl-diisopropylamin, oder Chinuclidin.

Nach einem weiteren Verfahrensaspekt können Benzodiazepin-Derivat der allgemeiner Formel (I) dadurch erhalten werden, dass man in Benzodiazepin-Derivaten der allgemeiner Formel (Ib) den Aziridinring hydrolytisch öffnet. Diese hydrolytische Ringöffnung erfolgt unter sauren Bedingungen, wobei nur Säuren in Betracht kommen, deren Anion mit dem Aziridinring nicht reagiert. Man arbeitet zweckmässigerweise in Gegenwart eines geeigneten, unter den Reaktionsbedingungen inerten organischen Lösungsmittels und bei Raumtemperatur. Beispielsweise kann man so vorgehen, dass man die Verbindung der Formel (Ib) in Dioxan löst, wenig Mineralsäure (z.B. einige Tropfen 25%ige Schwefelsäure) zugibt und das Gemisch während nicht allzu langer Zeit (z.B. 15 bis 30 Minuten) stehen lässt.

Nach einem weiteren Verfahrensaspekt können die Benzodiazepin-Derivate der allgemeinen Formel (I) erfindungsgemäss in pharmazeutisch akzeptable Säureadditionssalze übergeführt werden. Die Herstellung von solchen pharmazeutisch akzeptablen Säureadditionssalzen erfolgt nach allgemein üblichen Methoden. Es kommen sowohl Salze mit anorganischen als auch Salze mit organischen Säuren in Betracht, beispielsweise Hydrochloride, Hydrobromide, Sulfate, Citrate, Acetate, Succinate, Methansulfonate, oder p-Toluolsulfonate.

Die als Ausgangsprodukte verwendeten Benzodiazepin-Derivate der allgemeinen Formel (II) können — wie bereits weiter oben erwähnt — aus entsprechenden Benzodiazepin-Derivaten der allgemeinen Formel (V) hergestellt werden, und zwar durch Umsetzung mit Phosgen. Dabei geht man zweckmässigerweise so vor, dass man eine Lösung von Phosgen in einem unter den Reaktionsbedingungen inerten organischen Lösungsmittel vorlegt und dann unter Külen eine Lösung eines Benzodiazepin-Derivats der allgemeinen Formel (V) zugibt, hierauf einige Zeit unter Rückfluss erhitzt, dann wieder abkühlt und schliesslich die erhaltene Lösung mit einer tertiären organischen Aminoverbindung, wie Triäthylamin, basisch oder mindestens neutral stellt. Die erhaltene Lösung, enthaltend ein Benzodiazepin-Derivat der allgemeinen Formel (II), kann unter Feuchtigkeitausschluss und in der Kälte während mehreren Stunden aufbewahrt werden; sie wird — wie weiter oben ausgeführt — ohne Isolierung des darin enthalten Benzodiazepin-Derivats der allgemeinen Formel (II) direkt weiterverarbeitet.

Verbindungen der allgemeinen Formel (IV) können nach an sich bekannten Methoden hergestellt werden, wobei man teilweise in Analogie zu Methoden arbeiten kann, welche weiter oben im Zusammenhang mit gewissen Verfahren zur Herstellung von Verbindungen der Formel (I) und weiter unten im Zusammenhang mit der Herstellung der Verbindungen der Formel (VIII) beschrieben sind. Als

Ausgangsprodukte zur Herstellung der Verbindungen der Formel (IV) dienen zweckmässigerweise Benzophenon-Derivate der allgemeinen formel (XI).

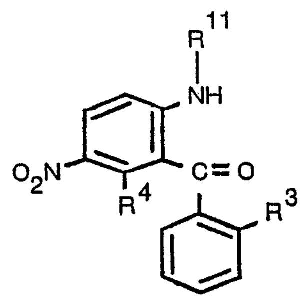

XI

worin $R^3$ und $R^4$ obige Bedeutung besitzen und $R^{11}$ Wasserstoff oder ($C_1$—$C_7$)-Alkyl bedeutet.

Beispielsweise kann man ein Benzophenon-Derivat der allgemeinen Formel (XI) zunächst in eine Verbindung der allgemeinen Formel XII

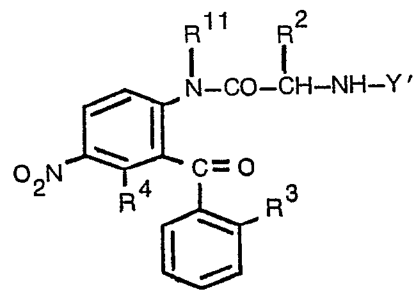

XII

worin $R^{11}$, $R^2$, $R^3$ und $R^4$ obige Bedeutung besitzen, und Y' eine Schutzgruppe bedeutet, überführen, worauf — falls $R^{11}$ in den Formeln (XI) und (XII) Wasserstoff bedeutet — das Stickstoffatom alkyliert, die Nitrogruppe zur Aminogruppe reduziert und — falls $R^4$ in den Formeln (XI) und (XII) Wasserstoff bedeutet — die erhaltene Aminoverbindung erwünschtenfalls halogeniert wird. Als Schutzgruppen (vgl. Symbol Y' in Formel XII) eignen sich in erster Linie Acylgruppen, vorzugsweise leicht abspaltbare Alkoxycarbonyl- oder Aralkoxycarbonylgruppen, insbesondere die Benzyloxycarbonylgruppe; zur Herstellung der Verbindungen der Formel (XII) aus den Benzophenonderivaten der Formel (XI) verwendet man demnach zweckmässigerweise entsprechende Acylaminoalkanoylhalogenide, wie Carbobenzoxyglycinchlorid, Carbobenzoxyalaninchlorid oder Carbobenzoxy-$\alpha$-aminobuttersäurechlorid. Falls eine N-Alkylierung durchgeführt werden muss, so erfolgt diese nach an sich bekannten Methoden, beispielsweise mittels Methyljodid in Gegenwart einer Base, wie Kaliumcarbonat, und in einem geeigneten, under der Reaktionsbedingungen inerten Lösungsmittel, wie Aceton. Die Reduktion der Nitrozur Aminogruppe erfolgt zweckmässigerweise mit Zinn (II)-chlorid. Eine fakultative Halogenierung von in 6-Stellung unsubstituierten 5-Aminobenzophenon-Derivaten erfolgt zweckmässigerweise mittels elementarem Halogen in saurer wässriger Lösung, wobei als Säure vorzungsweise der dem einzuführenden Halogen entsprechende Halogenwasserstoff verwendet wird.

Die auf die vorstehend beschriebene Weise erhaltenen 5-Aminobenzophenon-Derivate der allgemeinen Formel (XIII)

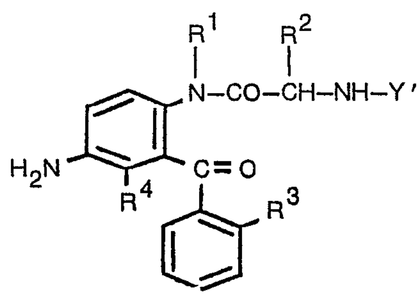

XIII

worin $R^1$, $R^2$, $R^3$, $R^4$ und Y' obige Bedeutung besitzen, werden anschliessend in entsprechende Verbindungen der allgemeinen Formel (XIV)

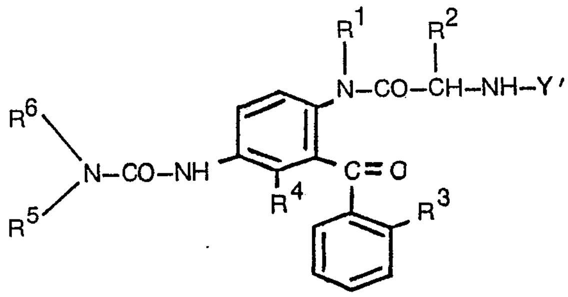

XIV

worin $R^1$, $R^2$, $R^3$, $R^4$, $R^5$, $R^6$ und Y' obige Bedeutung besitzen, übergeführt, was beispielsweise so bewerkstelligt werden kann, dass die Verbindung der Formel (XIII) in Analogie zu weiter oben beschriebenen Methoden mit einem Halogenid der allgemeinen Formel (VI) oder einem Isocyanat der allgemeinen Formel (VII) umgesetzt wird oder dass die Verbindung der allgemeinen Formel (XIII) — in Analogie zur weiter oben beschriebenen Methode für die Herstellung der Verbindungen der Formel (II) — in das entsprechende Isocyanat übergeführt wird, welches dann mit einer Aminoverbindung der allgemeinen Formel (III) umgesetzt wird, in Analogie zur weiter oben beschriebenen Methode zur Herstellung von Verbindungen der Formel (I) aus Verbindungen der Formel (II) und (III).

Durch Abspaltung der Schutzgruppe (Y') aus der Verbindung der Formel (XIV) erhält man dann die entsprechende Verbindung der Formel (IV).

Es ist auch möglich, die Verbindung der allgemeinen Formel (XIII) in eine Verbindung der allgemeinen Formel (XV)

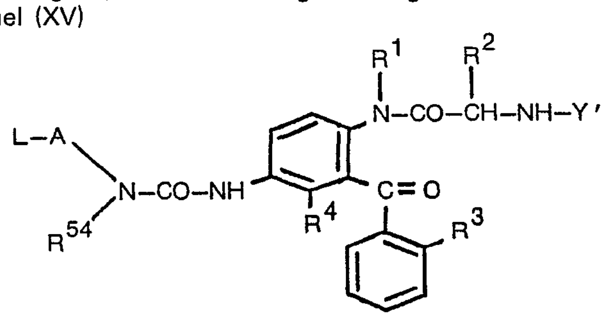

XV

worin $R^1$, $R^2$, $R^3$, $R^4$, $R^{54}$, L, A und Y' obige Bedeutung besitzen, überzuführen (in Analogie zur weiter unten beschriebenen Methode für die Herstellung der Verbindungen der allgemeinen Formel (X)), hierauf die Verbindung der Formel (XV) in die entsprechende Hydroxy- oder ($C_1$—$C_7$)-Alkanoyloxyverbindung überzuführen (in Analogie zur weiter oben beschriebenen Methode für die Herstellung von Verbindungen der Formel (I) aus Verbindungen der Formel (X)) und hierauf durch Abspaltung der Schutzgruppe (Y') zu einer entsprechenden Verbindung der Formel (IV) zu gelangen; weiterhin ist es möglich, die Verbindung der Formel (XIII) in eine Verbindung der allgemeinen Formel XIVa

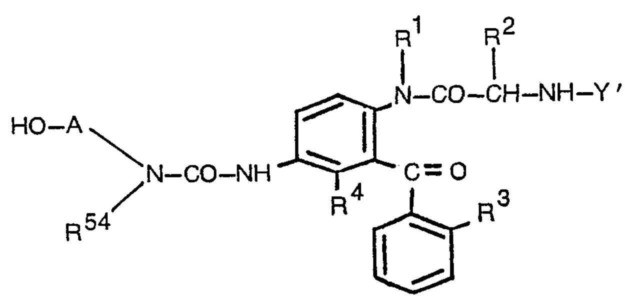

XIVa

worin $R^1$, $R^2$, $R^3$, $R^4$, $R^{54}$, A und Y' obige Bedeutung besitzen, überzuführen, worauf man dann durch ($C_1$—$C_7$)-Alkanoylierung (in Analogie zur weiter oben beschriebenen Methode für die Herstellung von Verbindungen der Formel (I) aus Verbindungen der Formel (Ia)) und anschliessende Abspaltung der Schutzgruppe (Y') zu einer entsprechenden Verbindung der Formel (IV) gelangt.

Weiterhin ist es möglich, die Verbindung der allgemeinen Formel (XIII) in eine Verbindung der allgemeinen Formel (XVI)

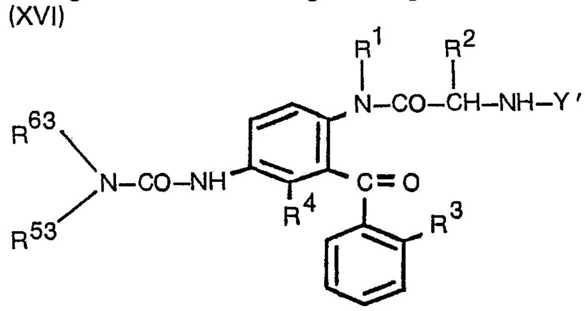

XVI

worin $R^1$, $R^2$, $R^3$, $R^4$, $R^{53}$, $R^{63}$ und Y′ obige Bedeutung besitzen, überzuführen, wobei man in Analogie zu den weiter unten beschriebenen Methoden zur Herstellung der Verbindungen der Formel (VIII) vorgehen kann; zu Verbindungen der allgemeinen Formel (IV) gelangt man aus Verbindungen der allgemeinen Formel (XVI) dadurch, dass man die Schutzgruppe Y′ und — vorgängig oder im gleichen Arbeitsgang — die weitere bzw. die weiteren im Molekül vorhandene bzw. vorhandenen Schutzgruppe bzw. Schutzgruppen abspaltet.

Eine weitere Möglichkeit zur Herstellung der Verbindungen der Formel (IV) besteht darin, dass man ein Nitrobenzophenon-Derivat der allgemeinen Formel (XI) in eine Verbindung der allgemeinen Formel (XVII)

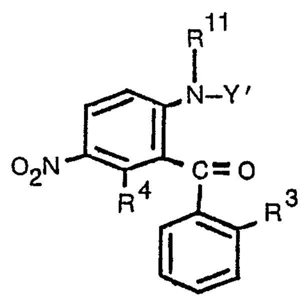

XVII

worin $R^{11}$, $R^3$, $R^4$ und Y′ obige Bedeutung besitzen, überführt, hierauf — falls $R^{11}$ in Formel (XVII) Wasserstoff bedeutet — das Stickstoffatom alkyliert, die Nitrogruppe reduziert und erwünschtenfalls eine erhaltenes in 6-Stellung unsubstituiertes 5-Aminobenzophenon-Derivat halogeniert. Die auf die soeben beschriebene Weise erhaltenen Verbindungen entsprechen der allgemeinen Formel (XVIII)

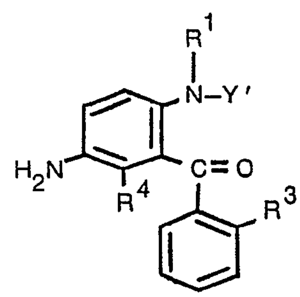

XVIII

worin $R^1$, $R^3$, $R^4$ und Y′ obige Bedeutung besitzen.

Eine Verbindung der obigen Formel (XVIII) kann sodann in eine Verbindung der Formel (XIX) oder (XIX′)

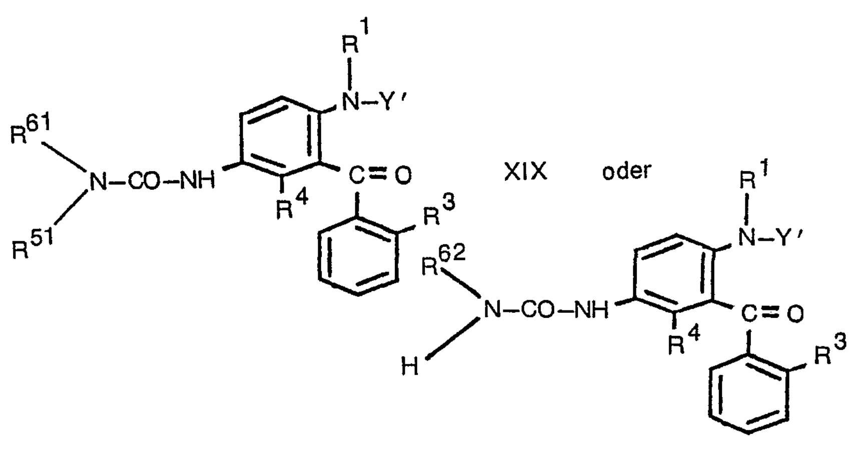

XIX oder XIX′

worin $R^1$, $R^3$, $R^4$, $R^{51}$, $R^{61}$, $R^{62}$ und Y′ obige Bedeutung besitzen, übergeführt werden, beispielsweise durch Umsetzung mit einem Halogenid der allgemeinen Formel (VI) bzw. einem Isocyanat der allgemeinen Formel (VII) (in Analogie zur oben beschriebenen Methode für die Herstellung von Verbindungen der Formel (I) aus Verbindungen der Formel (V)) oder durch Ueberführung in das entsprechende Isocyanat (in Analogie zum oben beschriebenen Verfahren für die Herstellung von Verbindungen der Formel (II) und anschliessende Umsetzung dieses Isocyanats mit einer Aminoverbindung der allgemeinen Formel (XX) oder (XX′)

XX 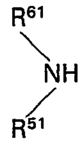 $R^{62}$—$NH_2$ XX′

worin $R^{51}$, $R^{61}$ und $R^{62}$ obige Bedeutung besitzen, (in Analogie zu dem weiter oben beschriebenen Verfahren für die Herstellung von Benzodiazepinderivaten der allgemeinen Formel (I) aus Verbindungen der allgemeinen Formel (II)). Durch Abspaltung der Schutzgruppe (Y′) aus der Verbindung der allgemeinen Formel (XIX) bzw. (XIX′) erhält man ein Benzophenon-Derivat der allgemeinen Formel (XXI) oder (XXI′)

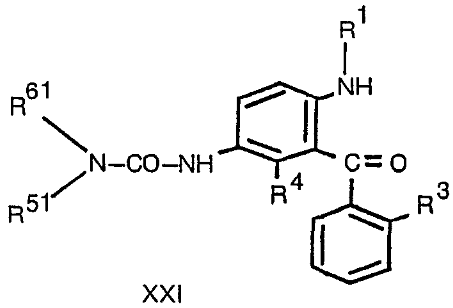

XXI

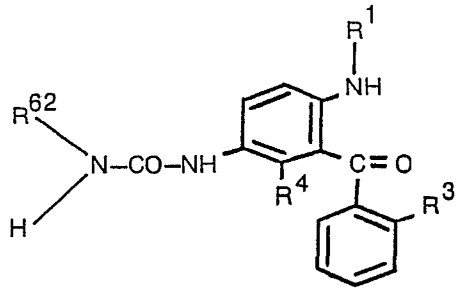

XXI′

worin $R^1$, $R^3$, $R^4$, $R^{51}$, $R^{61}$ und $R^{62}$ obige Bedeutung besitzen.

Verbindungen der allgemeinen Formel (XXI) bzw. (XXI′) können nach einer Vielzahl von verschiedenen, an sich bekannten Methoden in entsprechende Verbindungen der allgemeinen Formel (IV) übergeführt werden, beispielsweise durch Umsetzung mit einem entsprechenden $\alpha$-Halogenalkanoylhalogenid und Behandlung der erhaltenen Verbindung mit Ammoniak, durch Behandlung mit einem am Stickstoffatom eine geeignete Schutzgruppe tragenden entsprechenden $\alpha$-Aminoacylierungsmittel (beispielsweise mit einem entsprechenden $\alpha$-Benzyloxycarbonylaminoalkanoylhalogenid, wie Carbobenzoxyglycinchlorid) und anschliessende Abspaltung der Schutzgruppe, durch Ueberführung in ein entsprechendes $\alpha$-Azidoalkanoylderivat (beispielsweise in ein Azidoacetylderivat) und anschliessende Reduktion usw.

Verbindungen der Formel (XVIII) können andererseits auch in entsprechende Verbindungen der Formel (XXII)

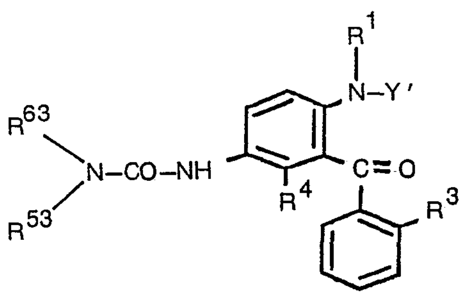 XXII

worin $R^1$, $R^3$, $R^4$, $R^{53}$, $R^{63}$ und Y′ obige Bedeutung besitzen, übergeführt werden (beispielsweise in Analogie zu den weiter unten beschriebenen Methoden für die Herstellung von Benzodiazepin-Derivaten der allgemeinen Formel (VIII)), worauf dann die Schutzgruppe Y′ entfernt wird; selbstverständlich müssen die verschiedenen Schutzgruppen in der Verbindung der Formel (XXII) so beschaffen sein, dass die Schutzgruppe Y′ entfernt werden kann, ohne dass eine Abspaltung der anderen Schutzgruppe bzw. Schutzgruppen im Molekül erfolgt. Die erhaltene Verbindung wird dann nach an sich bekannten Methoden in eine entsprechende Verbindung der obigen Formel (XVI) übergeführt, wobei man in Analogie zu der weiter oben beschriebenen Methode für die Herstellung der Verbindungen der Formel (XII) aus den Verbindungen der Formel (XI) verfahren kann; die Ueberführung der Verbindungen der Formel (XVI) in entsprechende Verbindungen der Formel (IV) ist bereits weiter oben erläutert worden.

Die Verbindungen der allgemeinen Formel V gehören einer an sich bekannten Verbindungsklasse an, und es sind bereits diverse spezifische Vertreter dieser Verbindungsklasse in der Literatur beschrieben worden. Noch nicht spezifisch vorbeschriebene Vertreter dieser Verbindungsklasse können nach an sich bekannten und jedem Fachmann geläufigen Methoden hergestellt werden. Zweckmässigerweise geht man von entsprechenden Nitroverbindungen der allgemeinen Formel (XXIII)

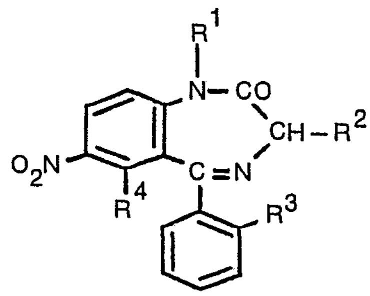

XXIII

worin $R^1$, $R^2$, $R^3$ und $R^4$ obige Bedeutung besitzen, aus; diese gehören ebenfalls einer an sich bekannten Verbindungsklasse an, von welcher verschiedene spezifische Vertreter in der Literatur beschrieben sind. Noch nicht spezifisch vorbeschriebene Vertreter dieser Verbindungsklasse können nach Methoden hergestellt werden, welche jedem Fachmann geläufig sind und in Analogie zu denjenigen Methoden durchgeführt werden können, welche für die Herstellung der spezifisch vorbekannten Verbindungen beschrieben sind; ausserdem enthalten verschiedene der weiter unten folgenden Beispiele detaillierte Angaben betreffend die Herstellung bestimmter Verbindungen der allgemeinen Formel (XXIII).

Die Ueberführung der Verbindugen der Formel (XXIII) in entsprechende Verbindungen der Formel (V) erfolgt durch Reduktion der Nitrogruppe, zweckmässigerweise mittels Zinn (II) chlorid, Zink, oder katalytisch angeregtem Wasserstoff. Falls das Symbol $R^4$ in Formel (XXIII) für Wasserstoff steht und eine Verbindung der Formel (V) gewünscht wird, worin $R^4$ Halogen bedeutet, muss anschliessend an die soeben erwähnte Reduktion noch eine Halogenierung durchgeführt werden; diese Halogenierung erfolgt zweckmässigerweise mittels elementarem Halogen in saurer wässriger Lösung, wobei als Säure zweckmässigerweise der dem einzuführenden Halogen entsprechende Halogenwasserstoff verwendet wird.

Verbindungen der allgemeinen Formel (VIII) können nach verschiedenen, an sich bekannten Methoden aus Verbindungen der Formeln (II)' oder (V)' hergestellt werden, wobei natürlich die Natur der Schutzgruppe bzw. Schutzgruppen, deren Anwesenheit in der herzustellenden Verbindung der Formel (VIII) erwünscht ist, für die Wahl der zur Anwendung gelangenden Methode bzw. Methoden in Betracht gezogen werden muss.

Zur Herstellung einer Verbindung der Formel (VIII), worin $R^{53}$ eine Schutzgruppe bedeutet, kann man ein Benzodiazepin-Derivat der Formel (V) mit einem entsprechenden Carbamoylhalogenid umsetzen (in Analogie zu der weiter oben beschriebenen Methode für die Herstellung von Verbindungen der Formel (I) aus Verbindungen der Formeln (V) und (VI).

Zur Herstellung von Verbindungen der Formel (VIII) worin $R^{53}$ Wasserstoff und $R^{63}$ eine Rest der Formel (IX) bedeuten, kann man ein Benzodiazepin-Derivat der allgemeinen Formel (V) mit einem entsprechenden Isocyanat umsetzen (in Analogie zur weiter oben beschriebenen Methode für die Herstellung von Verbindungen der Formel (I) aus Verbindungen der Formeln (V) und (VII)). Eine andere Möglichkeit besteht in der Umsetzung eines Benzodiazepin-Derivats der allgemeinen Formel (II) mit einem entsprechenden Amin (in Analogie zu der weiter oben beschriebenen Methode für die Herstellung von Verbindungen der Formel (I) aus Verbindungen der Formeln (II) und (III)); dabei ist jedoch zu beachten, dass in diesem Fall als Schutzgruppe (Y) eine Acylgruppe nicht in Betracht kommt. Weiterhin kann man in einem Benzodiazepin-Derivat der Formel (Ib) den Aziridinring durch saure Alkoholyse mit tert.-Butanol, oder Benzylalkohol öffnen, wobei man eine Verbindung der Formel (VIII) erhält, worin $R^{53}$ Wasserstoff und $R^{63}$ 2-tert.-Butoxyäthyl bzw. 2-Benzyloxyäthyl bedeuten.

Zur Herstellung von Verbindungen der Formel (VIII), worin $R^{53}$ ($C_1$—$C_7$)-Alkyl und $R^{63}$ einen Rest der Formel (IX) bedeuten, kann man ein Benzodiazepin der allgemeinen Formel (V) mit einem entsprechenden Carbamoylhalogenid umsetzen (in Analogie zu der weiter oben beschriebenen Methode für die Herstellung von Verbindungen der Formel (I) aus Verbindungen der Formeln (V) und (VI). Eine andere Möglichkeit besteht in der Umsetzung eines Benzodiazepin-Derivats der allgemeinen Formel (II) mit einem entsprechenden Amin (in Analogie zur weiter oben beschriebenen Methode für die Herstellung von Verbindungen der Formel (I) aus Verbindungen der Formeln (II) und (III)), wobei wiederum als Schutzgruppe (Y) eine Acylgruppe nicht in Betracht kommt.

Es ist zu beachten, dass diejenigen Verbindungen der Formel (VIII), worin $R^{53}$ Wasserstoff oder ($C_1$—$C_7$)-Alkyl und $R^{63}$ einen Rest der Formel (IX) bedeuten, worin die durch Y bezeichnete Schutzgruppe eine ($C_1$—$C_7$)-Alkanoylrest (wie Acetyl) ist, von der eingangs erwähnten Formel (I) umfasst werden; dabei handelt es sich nämlich um Verbindungen der Formel (I), worin $R^5$ Wasserstoff oder ($C_1$—$C_7$)-Alkyl und $R^6$ ($C_1$—$C_7$)-Alkenoyloxy-($C_2$—$C_7$)-alkyl (wie Acetoxy-($C_2$—$C_7$)-alkyl) bedeuten.

Unter den Verbindungen der allgemeinen Formel (VIII), worin $R^{53}$ eine Schutzgruppe bedeutet, falls $R^{63}$ einen Rest der Formel (IX) bedeutet und die durch Y bezeichnete Schutzgruppe eine ($C_1$—$C_7$)-Alkanoylrest ist, sind insbesondere diejenigen hervorzuheben, worin $R^1$ Methy, $R^2$ Wasserstoff, $R^3$ Fluor, $R^4$ Wasserstoff, $R^{53}$ Wasserstoff oder eine Schutzgruppe und $R^{63}$ einen Rest der obigen Formel (IX) bedeuten, wobei A für eine Dimethylengruppe steht, wie z.B. 1-(2-tert.-Butyoxyäthyl)-3-[5-(o-fluorphenyl)-2,3-dihydro-1-methyl-2-oxo-1H-1,4-benzodiazepin-7-yl]-harnstoff.

Verbindungen der Formel (X) können nach an sich bekannten Methoden aus Aminobenzodiazepin-Derivaten der allgemeinen Formel (V) hergestellt werden und zwar durch Umsetzung mit einem entsprechenden Carbamoylchlorid (in Analogie zur weiter oben beschriebenen Methode für die Herstellung von Verbindungen der Formel (I) aus Verbindungen der Formeln (V) und (VI) oder durch Umsetzung mit einem entsprechenden Isocyanat (in Analogie zur weiter oben beschriebenen Methode für die Herstellung von Verbindungen der Formel (I) aus Verbindungen der Formeln (V) und (VIII)).

Besonders erwähnenswert sind diejenigen Verbindungen der Formel (X), worin $R^1$ Methyl, $R^2$ Wasserstoff, $R^3$ Fluor, $R^4$ Wasserstoff, $R^{54}$ Wasserstoff und A eine Dimethylengruppe bedeuten, wie z.B. 1-(2-Chloräthyl)-3-[5-(o-fluorphenyl)-2,3-dihydro-1-methyl-2-oxo-1H-1,4-benzodiazepin-7-yl-]-harnstoff.

Ueberraschenderweise hat sich gezeigt, dass die Benzodiazepin-Derivate der eingangs definierten allgemeinen Formel (I) keine oder nur sehr geringe Wirkungen auf das zentrale Nervensystem entfalten, wogegen sie ausgeprägte aldosteronantagonistische Eigenschaften aufweisen. Diese aldosteronantagonistischen Eigenschaften lassen sich, wie nachstehend erläutert, an adrenalekomierten Ratten nachweisen.

Verabreicht man adrenalektomierten Ratten Aldosteron, so beobachtet man — im Vergleich zu unbehandelten Tieren — eine ausgeprägte Verminderung der Natrium-Ausscheidung (Natrium-Retention), eine erhöhte Kalium-Ausscheidung (Kalium-Exkretion) sowie eine Verminderung des ausgeschiedenen Harnvolumens. Gibt man den Tieren vor der Behandlung mit Aldosteron Verbindungen der Formel (I), so beobachtet man — im Vergleich zu nur mit Aldosteron behandelten Tieren (Kontrolltiere) — eine ausgeprägte Erhöhung der Natrium-Ausscheidung (das heisst: die durch Aldosteron bewirkte Natrium-Retention wird antagonisiert), wogegen die Kalium-Exkretion und das Harnvolumen in geringerem Ausmass beinflusst werden.

Der Standardversuch wird wie folgt durchgeführt:

Weibliche Holtzmann-Ratten (150—180 g) werden 70 bis 74 Stunden vor Versuchsbeginn bilateral adrenalektomiert. Nach der Operation erhalten die Tiere ein gebräuchliches Ratten-Trochenfutter und 0,9%-ige Kochsalzlösung zum Trinken. 16—17 Stunden vor Versuchsbeginn wird den Tieren das Futter entzogen, wogegen sie nach wie vor ad libitum 0,9%-ige Kochsalzlösung trinken können. Bei Versuchsbeginn wird den Tieren die auf Aldosteronantagonismus zu prüfende Substanz mittels einer Magensonde verabreicht. 30 Minuten später erhalten die Tiere eine subcutane Injektion von 4 mmg/kg Aldosteron. Nach weiteren 90 Minuten werden die Harnblasen der Tiere durch sorgfältiges suprapublisches Drücken entleert, worauf die Tiere ohne Nahrung und ohne Getränk einzeln in Metabolismuskäfige verbracht werden. Der Urin der Tiere wird dann während 3 Stunden gesammelt, worauf ihre Harnblasen nochmals entleert werden. Der spontan ausgeschiedene Harn und der am Schluss des Versuches durch Ausdrücken der Harnblasen erhaltene Restharn werden in graduierten Zentrifugengläsern gesammelt. Natrium- und Kalium-Konzentrationen des Harns werden mit einem Flammenphotometer bestimmt.

In der nachfolgenden Tabelle werden die Resultate dargestellt, welche mit repräsentativen Vertretern der durch die allgemeine Formel (I) definierten Verbindungsklasse in dem vorstehend geschilderten Versuch erhalten worden sind. Angegeben werden in dieser Tabelle für jede der darin figurierenden Verbindungen die verabreichte Dosis (in mg/kg p.o.) sowie die prozentuale Veränderung des Harnvolumens, der Natrium-Ausscheidung und der Kalium-Ausscheidung im Vergleich zu den Kontrolltieren (das heisst im Vergleich zu den nur mit Aldosteron behandelten Tieren). Ausserdem enthält die Tabelle Angaben über die akute Toxizität der untersuchten Verbindungen (DL 50 in mg/kg bei einmaliger oraler Verabreichung an Mäuse).

| $R^1$ | $R^2$ | $R^3$ | $R^4$ | $R^5$ | $R^6$ | Dosis mg/kg p.o. | Volumen | $[Na^+]$ | $[K^+]$ | DL 50 mg/kg p.o. |
|---|---|---|---|---|---|---|---|---|---|---|
| | | | | | | | in %, bezogen a. Kontrolltiere | | | |
| $CH_3$ | H | F | H | H | $CH_2CH_2OH$ | 0,1 | 85 | 247 | 121 | >5000 |
| $CH_3$ | H | F | H | H | $CH_3$ | 3 | 95 | 182 | 84 | >5000 |
| $CH_3$ | H | F | H | H | t.-$C_4H_9$ | 10<br>1 | 105<br>194 | 234<br>236 | 70<br>132 | 600–1200 |
| $CH_3$ | H | F | H | H | $CH(CH_3)CH_2OH$ | 10<br>1 | 200<br>73 | 452<br>127 | 129<br>80 | >5000 |
| $CH_3$ | H | F | H | H | p-$CH_3O-C_6H_4$ | 10 | 117 | 240 | 145 | >5000 |
| $CH_3$ | H | F | H | H | $CH_2C_6H_5$ | 1 | 139 | 255 | 153 | >5000 |
| $CH_3$ | H | F | H | $CH_3$ | $CH_3$ | 10 | 102 | 190 | 91 | >5000 |
| $CH_3$ | H | F | H | $CH_3$ | $C_2H_5$ | 1 | 100 | 291 | 97 | >5000 |
| $CH_3$ | H | F | H | $(CH_2)_2$ | | 10<br>1 | 129<br>192 | 237<br>294 | 108<br>146 | >5000 |
| $CH_3$ | H | F | H | $(CH_2)_4$ | | 1 | 184 | 308 | 112 | >5000 |
| $CH_3$ | H | F | Cl | H | $CH_3$ | 1 | 178 | 330 | 91 | 1250–2500 |
| $CH_3$ | H | F | Cl | H | $C_2H_5$ | 10 | 156 | 271 | 149 | 2500–5000 |
| $CH_3$ | H | F | Cl | H | n-$C_4H_9$ | 1 | 175 | 380 | 104 | >5000 |
| $CH_3$ | H | F | Cl | H | $CH_2CH_2OH$ | 1 | 140 | 242 | 110 | >5000 |
| $CH_3$ | H | F | Cl | $CH_3$ | $C_2H_5$ | 1 | 136 | 278 | 118 | 2500–5000 |
| $CH_3$ | H | F | Cl | $C_2H_5$ | $C_2H_5$ | 10 | 124 | 236 | 110 | >5000 |

Die Benzodiazepin-Derivate der allgemeinen Formel (I) und ihre pharmazeutisch akzeptablen Säureadditionssalze können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragées und Hartgelatinekapseln können die Benzodiazepin-Derivate der allgemeinen Formel (I) und ihre pharmazeutisch akzeptablen Säureadditionssalze mit pharmazeutisch inerten, anorganischen oder organischen Excipientien verarbeit werden. Als solche Excipientien kann man für Tabletten, Dragées und Hartgelatinekapseln z.B. Lactose, Maisstärke oder Derivate davon, Talk, Stearinsäure oder deren Salze verwenden.

Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich.

Zur Herstellung von Lösungen und Sirupen eignen sich als Excipientien z.B. Wasser, Polyole, Saccharose, Invertzucker oder Glukose.

Für Injektionslösungen eignen sich als Excipientien z.B. Wasser, Alkohole, Polyole, Glyzerin, vegetabile Oele.

Für Suppositorien eignen sich als Excipientien z.B. natürliche oder gehärtete Oele, Wachse, Fette, halbflüssige oder flüssige Polyole.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend eine oder mehrere Verbindungen der allgemeinen Formel (I) oder pharmazeutisch akzeptable Säureadditionssalze davon, ebenfalls Gegenstand der vorliegenden Erfindung. Solche Arzneimittel können hergestellt werden indem man eine oder mehrere Verbindungen der allgemeinen Formel (I) oder pharmazeutisch akzeptable Säureadditionssalze davon in eine galenische Darreichungsform bringt. Die Benzodiazepin-Derivate der allgemeinen Formel I und ihre pharmazeutisch akzeptablen Säureadditionssalzen können bei der Bekämpfung bzw. Verhütung von Krankheiten, insbesondere bei der Bekämpfung bzw. Verhütung von Herzversagen, von hepatitischer Aszites, von primärem Aldosteronismus und von essentieller Hypertonie verwendet werden. Die Dosierung kann innerhalb weiter Grenzen varieren und ist natürlich in jedem einzelnen Fall den individuellen Gegebenheiten anzupassen. Im Allgemeinen dürfte bei oraler Verabreichung eine Tagesdosis von etwa 20 mg bis 1500 mg angemessen sein.

Einige der Benzodiazepin-Derivate der eingangs definierten allgemeinen Formel I bewirken auch eine Hemmung der intestinalen Resorption von Cholesterin, und zwar insbesondere diejenigen, worin $R^1$, $R^2$, $R^3$ und $R^4$ die eingangs erwähnte Bedeutung besitzen und $R^5$ Wasserstoffs und $R^6$ $(C_2—C_7)$-Alkyl oder Hydroxy-$(C_2—C_7)$-alkyl bedeuten, sowie diejenigen, worin $R^1$, $R^2$ und $R^3$ die eingangs erwähnte Bedeutung besitzen, $R^4$ Halogen bedeutet und entweder $R^5$ und $R^6$ je $(C_1—C_7)$-Alkyl bedeuten oder $R^5$ und $R^6$ zusammen mit dem Stickstoffatomen einen 3- bis 7-gliedrigen Heterocyclus ohne zusätzliches Heteroatom oder einen N-Methylpiperazin-, Thiazolidin-, Morpholin- oder Thiomorpholin rest bedeuten. Unter diesen Verbindungen sind diejenigen bevorzugt, worin $R^3$ Fluor oder Chlor bedeutet und worin $R^4$ Halogen bedeutet, wobei Chlor und insbesondere Brom bevorzugt ist.

Eine ganz besonders bevorzugte die intestinale Resorption von Cholesterin hemmende Verbindung im Rahmen der vorliegenden Erfindung ist der 1-[6-Brom-5-(o-chlorphenyl)-2,3-dihydro-1,3-dimethyl-2-oxo-1H-1,4-benzodiazepin-7-yl]-3-(2-hydroxyäthyl)harnstoff. Diese Verbindung enthält ein Asymmetriezentrum und kann daher in racemischer oder in optisch aktiver Form vorliegen; dies trifft selbstverständlich für alle übrigen Verbindungen der eingangs definierten Formel I zu, welche ein Asymmetriezentrum enthalten. Verbindungen der eingangs definierten allgemeinen Formel I, welche mehr als ein Asymmetriezentrum enthalten, können in verschiedenen diastereoisomeren Formen vorliegen. Die vorliegende Erfindung umfasst sämtliche möglichen Stereoisomeren der Verbindungen der eingangs definierten Formel I mit einem oder mehreren Asymmetriezentren, ferner sämtliche möglichen Diastereoisomerengemische und Racemate, sowie die Auftrennung von Diastereoisomerengemischen und die Spaltung von Racematen, welche nach an sich bekannten Methoden erfolgen können.

Weitere besonders bevorzugte die intestinale Resorption von Cholesterin hemmende Verbindungen im Rahmen der vorliegenden Erfindung sind:

N-[6-Chlor-5-(o-fluorphenyl)-2,3-dihydro-1-methyl-2-oxo-1H-1,4-benzodiazepin-7-yl]-4-morpholincarboxamid;

1-[6-Brom-5-(o-fluorphenyl)-2,3-dihydro-1,3-dimethyl-2-oxo-1H-1,4-benzodiazepin-7-yl]-3-(2-hydroxyäthyl)harnstoff; und

1-[6-Chlor-5-(o-chlorphenyl)-2,3-dihydro-1,3-dimethyl-2-oxo-1H-1,4-benzodiazepin-7-yl]-3-(2-hydroxyäthyl)harnstoff.

Die Hemmung der intestinalen Resorption von Cholesterin lässt sich tierexperimentell wie folgt nachweisen:

Die zu untersuchenden Substanzen werden Gruppen von je 6 normalen weiblichen Füllinsdorfer Albino-Ratten (Körpergewicht etwa 70 g) mittels einer Magensonde verabreicht. Unmittelbar anschliessend erhalten die Tiere mittels einer Magensonde eine radioaktives Cholesterin enthaltende Testnahrung. Hierauf wird der Kot während drei Tagen gesammelt, gefriergetrocknet und pulverisiert, und ein Aliquot wird zwecks Ermittlung der Radioaktivität des Kots verbrannt. Unbehandelte Kontrolltiere scheiden etwa 40—50% des mit der Nahrung aufgenommenen radioaktiven Cholesterins aus; diese Ausscheidung wird als "cholesterol recovery" (CHOREC) bezeichnet und für die unbehandelten Tiere arbiträr als 100% festgesetzt. Tiere, welche mit einer die intestinale Resorption von Cholesterin hemmenden Substanz behandelt worden sind, zeigen eine höhere Ausscheidung von radioaktivem Cholesterin als unbehandelte Kontrolltiere. Die Cholesterinausscheidung (CHOREC) von behandelten Tieren wird in Prozenten der Cholesterinausscheidung von unbehandelten Kontrolltieren ausgedrückt.

In der nachfolgenden Tabelle werden die Resultate dargestellt, welche mit den weiter oben erwähnten besonders bevorzugten die intestinale Resorption von Cholesterin hemmenden Verbindungen erhalten worden sind. Angegeben werden für jede der darin figurierenden Verbindungen die verabreichte Dosis (in μMol/kg p.o.) sowie die jeweils ermittelte Cholesterinausscheidung (CHOREC) in Prozenten der Cholesterinausscheidung von unbehandelten Kontrolltieren. Ausserdem enthält die Tabelle Angaben über die akute Toxizität der untersuchten Verbindungen (DL 50 in mg/kg bei einmaliger oraler Verabreichung an Mäuse.

| $R^1$ | $R^2$ | $R^3$ | $R^4$ | $R^5$ | $R^6$ | Dosis µMol/kg p.o. | CHOREC % | DL 50 mg/kg p.o. |
|---|---|---|---|---|---|---|---|---|
| $CH_3$ | H | F | Cl | $(CH_2)_2O(CH_2)_2$ | | 1000 | 186 | 1250–2500 |
| $CH_3$ | $CH_3$ (rac.) | Cl | Cl | H | $CH_2CH_2OH$ | 1000 | 188 | >5000 |
| $CH_3$ | $CH_3$ (rac.) | Cl | Br | H | $CH_2CH_2OH$ | 1000<br>100<br>30<br>10 | 187<br>168<br>157<br>150 | >5000 |
| $CH_3$ | $CH_3$ (rac.) | F | Br | H | $CH_2CH_2OH$ | 1000<br>300<br>100<br>30<br>10 | 189<br>169<br>155<br>160<br>132 | >5000 |

Die weiter oben erwähnten, die intestinale Resorption von Cholesterin hemmenden Benzodiazepin-Derivate und pharmazeutisch akzeptable Säureadditionssalze davon können bei der Verhütung bzw. Bekämpfung der Atherosklerose verwendet werden. Die Dosierung kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 10 mg bis 3 g, vorzugsweise von etwa 100 bis 500 mg angemessen sein.

In den nachfolgenden Beispielen, welche die vorliegende Erfindung näher erläutern, sind sämtliche Temperaturen in Celsiusgraden angegeben.

Beispiel 1

a) 5 g (0,017M) 7-Amino-5-(o-fluorphenyl)-1,3-dihydro-1-methyl-2H-1,4-benzodiazepin-2-on werden in 60 ml 1,2-Dichloräthan unter Rückfluss in Lösung gebracht. In einem Sulfierkolben werden 2,5 g (0,025 M) Phosgen, gelöst in eisgekühltem 1,2-Dichloräthan, vorgelegt. Die heisse Dichloräthanlösung des 7-Amino-5-(o-fluorphenyl)-1,3-dihydro-1-methyl-2H-1,4-benzodiazepin-2-ons wird dann unter Eiskühlung und Rühren derart zugetropft, dass die Reaktions-temperatur 10° nicht übersteigt. Anschliessend wird das Reaktionsgemisch 1 Stunde unter Rühren am Rückfluss erhitzt, worauf die Lösung mit Eis auf 10—25° gekühlt und mit Triäthylamin basisch gestellt wird. Die erhaltene Dichloräthanlösung von [5-(o-Fluorphenyl)-2,3-dihydro-1-methyl-2-oxo-1H-1,4-benzodiazepin-7-yl]isocyanat kann unter Feuchtigkeitsausschluss mehrere Stunden im Eisschrank aufbewahrt werden. Sie wird ohne Isolierung des darin enthaltenen Isocyanates weiterverarbeitet.

b) In eine Dichloräthanlösung von [5-(o-Fluorphenyl)-2,3-dihydro-1-methyl-2-oxo-1H-1,4-benzodiazepin-7-yl]isocyanat, erhalten gemäss Angaben in Absatz a) aus 5,6 g (0,019 M) 7-Amino-5-(o-fluorphenyl)-1,3-dihydro-1-methyl-2H-1,4-benzodiazepin-2-on, wird überschüssiges gasförmiges Methylamin eingeleitet. Hierauf engt man das Reaktionsgemisch ein und nimmt den Rückstand in Methylenchlorid-Wasser auf, worauf die Methylenchloridlösung abgetrennt, mehrmals mit Wasser gewaschen, über Natriumsulfat getrocknet, filtriert und eingeengt wird. Aus Essigester kristallisiert man 1-[5-(o-Fluorphenyl)-2,3-dihydro-1-methyl-2-oxo-1H-1,4-benzodiazepin-7-yl]-3-methylharnstoff vom Schmelzpunkt 173°.

Beispiel 2

Eine Dichloräthanlösung von [5-(o-Fluorphenyl)-2,3-dihydro-1-methyl-2-oxo-1H-1,4-benzodiazepin-7-yl]isocyanat, erhalten gemäss Angaben in Absatz a) von Beispiel 1 aus 4,2 g (0,015 M) 7-Amino-5-(o-fluorphenyl)-1,3-dihydro-1-methyl-2H-1,4-benzodiazepin-2-on, wird mit überschüssigem tert. -Butylamin versetzt. Nach Einengen des Reaktionsgemisches wird der Rückstand in Methylenchlorid-Wasser aufgenommen, worauf die Methylenchloridlösung mehrmals mit Wasser gewaschen wird. Nach Trocknen der organischen Phase über Natriumsulfat wird das Natriumsulfat abfiltriert und das Methylenchlorid abgedampft. Der Rückstand wird an einer Kieselgelkolonne (150 g $SiO_2$) gereinigt (Laufmittel: 40% Essigester in Methylenchlorid) und aus Aethanol-Petroläther kristallisiert. Man erhält 1-tert.-Butyl-3-[5-(o-fluorphenyl)-2,3-dihydro-1-methyl-2-oxo-1H-1,4-benzodiazepin-7-yl]harnstoff, welcher bei 210° unter Zersetzung schmilzt.

Beispiel 3

Eine Dichloräthanlösung von [5-(o-Fluorphenyl)-2,3-dihydro-1-methyl-2-oxo-1H-1,4-benzodiazepin-7-yl]isocyanat, erhalten gemäss Angaben in Absatz a) von Beispiel 1 aus 5 g (0,018 M) 7-Amino-5-(o-fluorphenyl)-1,3-dihydro-1-methyl-2H-1,4-benzodiazepin-2-on, wird mit 5 ml Benzylamin versetzt und bei Raumtemperatur gerührt. Nach einer halben Stunde wird die Lösung eingeengt, der Rückstand in einem Gemisch von Methylenchlorid und 10%iger Natriumcarbonatlösung aufgenommen. Die organische Phase wird abgetrennt, mehrmals mit 10%iger Bicarbonatlösung gewaschen, über Natriumsulfat getrocknet, filtriert und eingeengt. Kristallisation des Rückstandes aus Essigester-Aether liefert 1-Benzyl-3-[5-(o-fluorphenyl)-2,3-dihydro-1-methyl-2-oxo-1H-1,4-benzodiazepin-7-yl]harnstoff, welcher bei 174—178° schmilzt.

Beispiel 4

Eine Dichloräthanlösung von [5-(o-Fluorphenyl)-2,3-dihydro-1-methyl-2-oxo-1H-1,4-benzodiazepin-7-yl]isocyanat, erhalten gemäss Angaben im Absatz a) von Beispiel 1 aus 6,7 g (0,024 M) 7-Amino-5-(o-fluorphenyl)-1,3-dihydro-1-methyl-2H-1,4-benzodiazepin-2-on, wird mit 8 ml Methyläthylamin versetzt, worauf eine halbe Stunde bie Raumtemperatur gerührt, eingeengt und der Rückstand in Methylenchlorid-Wasser aufgenommen wird. Die Methylenchloridphase wird abgetrennt, mit Wasser gewaschen über Natriumsulfat getrocknet, filtriert und eingeengt. Der Rückstand wird an einer 300 g-Kieselgelsäule mit Essigester als Eluierungsmittel gereinigt und aus Essigester-Aether kristallisiert. Man erhält 1-Aethyl-3-[5-(o-fluorphenyl)-2,3-dihydro-1-methyl-2-oxo-1H-1,4-benzodiazepin-7-yl]-1-methylharnstoff, welcher bei 165—168° schmilzt.

Beispiel 5

Eine Dichloräthanlösung von [5-(o-Fluorphenyl)-2,3-dihydro-1-methyl-2-oxo-1H-1,4-benzodiazepin-7-yl]isocyanat, erhalten gemäss Angaben im Absatz a) von Beispiel 1 aus 6 g (0,021 M) 7-Amino-5-(o-fluorphenyl)-1,3-dihydro-1-methyl-2H-1,4-benzodiazepin-2-on, wird mit einer Lösung von überschüssigem Aethylenimin in Dichloräthan versetzt, worauf man das Reaktionsgemisch auf Eis-Wasser giesst und mit Methylenchlorid extrahiert. Die organische Phase wird abgetrennt, mit Natriumsulfat getrocknet, filtriert und bis auf 50 ml eingeengt. Die zurückgebliebene Lösung wird an einer 250 g-Kieselgelsäule mit Essigester als Eluierungsmittel schnell gereinigt und aus Essigester-Aether kristallisiert. Man erhält N-[5-(o-Fluorphenyl)-2,3-dihydro-1-methyl-2-oxo-1H-1,4-benzodiazepin-7-yl]-1-aziridincarboxamid, welches bei 186—190° unter Zersetzung schmilzt.

Beispiel 6

Eine Dichloräthanlösung von [5-(o-Fluorphenyl)-2,3-dihydro-1-methyl-2-oxo-1H-1,4-benzodiazepin-7-yl]isocyanat, hergestellt gemäss Angaben in Absatz a) von Beispiel 1 aus 5 g (0,018 M) 7-Amino-5-(o-fluorphenyl)-1,3-dihydro-1-methyl-2H-1,4-benzodiazepin-2-on, wird mit 4,2 ml Pyrrolidin versetzt, worauf das Gemisch 20 Minuten gerührt und dann eingeengt wird. Der Rückstand wird an einer 250 g-Kieselgelsäule mit Methylenchlorid-Aceton (10:1) als Eluierungsmittel gereinigt und aus Aceton kristallisiert. Man erhält N-[5-(o-Fluorphenyl)-2,3-dihydro-1-methyl-2-oxo-1H-1,4-benzodiazepin-7-yl]-1-pyrrolidincarboxamid, welches bei 159—160° schmilzt.

Beispiel 7

Eine Dichloräthanlösung von [5-(o-Fluorphenyl)-2,3-dihydro-1-methyl-2-oxo-1H-1,4-benzodiazepin-7-yl]isocyanat, hergestellt gemäss Angaben in Absatz a) von Beispiel 1 aus 5 g (0,018 M) 7-Amino-5-(o-fluorphenyl)-1,3-dihydro-1-methyl-2H-1,4-benzodiazepin-2-on, wird mit 5 ml Aethanolamin versetzt, worauf das Gemisch 20 Minuten gerührt und dann eingeengt wird. Der Rückstand wird an einer 250 g-Kieselgelsäule mit Methylenchlorid-Aceton (10:1) als Eluierungsmittel gereinigt und aus Aceton kristallisiert. Man erhält 1-[5-(o-Fluorphenyl)-2,3-dihydro-1-methyl-2-oxo-1H-1,4-benzodiazepin-7-yl]-3-(2-hydroxyäthyl)harnstoff, welcher bei 156—160° unter Zersetzung schmilzt.

Beispiel 8

Eine Dichloräthanlösung von [5-(o-Fluorphenyl)-2,3-dihydro-1-methyl-2-oxo-1H-1,4-benzodiazepin-7-yl]isocyanat, hergestellt gemäss Angaben in Absatz a) von Beispiel 1 aus 4 g (0,014 M) 7-Amino-5-(o-fluorphenyl)-1,3-dihydro-1-methyl-2H-1,4-benzodiazepin-2-on, wird mit 2,2 ml DL-Alaninol in 50 ml 1,2-Dichloräthan versetzt, worauf das Gemisch 20 Minuten gerührt und dann eingeengt wird. Der Rückstand wird an einer 200 g-Kieselgelsäule mit Methylenchlorid-Aceton (4:1) als Eluierrungsmittel gereinigt und aus Aceton kristallisiert. Man erhält rac. 1-[5-(o-Fluorphenyl)-2,3-dihydro-1-methyl-2-oxo-1H-1,4-benzodiazepin-7-yl]-3-(2-hydroxy-1-methyläthyl)harnstoff, welcher bei 165—168° unter Zersetzung schmilzt.

Beispiel 9

a) Man löst 80 g (0,28 M) 7-Amino-5-(o-fluorphenyl)-1,3-dihydro-1-methyl-2H-1,4-benzodiazepin-2-on in 220 ml konz. Salzsäure und leitet bei —10° 4 Stunden lang langsam Chlorgas in die Lösung ein. Man giesst das Reaktionsgemisch langsam auf ein Gemisch von Eis und Sodalösung (250 g Soda in Wasser gelöst) und extrahiert mit Methylenchlorid, worauf der Extrakt über Natriumsulfat getrocknet, filtriert und eingeengt wird. Der Rückstand wird an einer 1,2 kg-Kieselgelsäule mit Methylenchlorid und dann Methylenchorid-Essigester (10:1) als Eluierungsmittel gereinigt und aus Essigester-Aether-n-Hexan kristallisiert. Man erhält 7-Amino-6-chlor-5-(o-fluorphenyl)-1,3-dihydro-1-methyl-2H-1,4-benzodiazepin-2-on, welches bei 240° schmilzt.

b) In eine Dichloräthanlösung von [6-Chlor-5-(o-fluorphenyl)-2,3-dihydro-1-methyl-2-oxo-1H-1,4-benzodiazepin-7-yl]isocyanat, erhalten in Analogue zu den Angaben in Absatz a) von Beispiel 1 aus 10 g (0,031 M) 7-Amino-6-chlor-5-(o-fluorphenyl)-1,3-dihydro-1-methyl-2H-1,4-benzodiazepin-2-on, wird überschüssiges gasförmiges Methylamin eingeleitet, worauf das Gemisch eingeengt wird. Den Rückstand löst man in Methylenchlorid-Wasser und extrahiert mit Methylenchlorid. Die organische Phase wird abgetrennt, mit Natriumsulfat getrocknet, filtriert und eingeengt. Nach der Reinigung an einer 500 g-Keiselgelsäule mit Essigester als Eluierungsmittel wird das Reaktionsprodukt aus Essigester-Aether kristallisiert. Man erhält 1-[6-Chlor-5-(o-fluorphenyl)-2,3-dihydro-1-methyl-2-oxo-1H-1,4-benzodiazepin-7-yl]-3-methylharnstoff, welcher bei 145—160° unter Zersetzung schmilzt.

Beispiel 10

Eine Dichloräthanlösung von [6-Chlor-5-(o-fluorphenyl)-2,3-dihydro-1-methyl-2-oxo-1H-1,4-benzodiazepin-7-yl]isocyanat, erhalten in Analogie zu den Angaben in Absatz a) von Beispiel 1 aus 4 g (0,0126 M) 7-Amino-6-chlor-5-(o-fluorphenyl)-1,3-dihydro-1-methyl-2H-1,4-benzodiazepin-2-on, wird mit 2,5 ml n-Butylamin in 40 ml 1,2-Dichloräthanversetzt. Man rührt 15 Minuten bei Raumtemperatur, engt die Dichloräthanlösung ein und reinigt den Rückstand an einer 250 g-Keiselgelkolonne mit Methylenchlorid und dann mit Methylenchorid-Essigester (5:1) als Eluierungsmittel. Man

erhält 1-Butyl-3-[6-chlor-5-(o-fluorphenyl)-2,3-dihydro-1-methyl-2-oxo-1H-1,4-benzodiazepin-7-yl]harnstoff, welcher nach Umkristallisieren aus Aether (mit sehr wenig Aceton) bei 196—198° schmilzt.

Beispiel 11

Eine Dichloräthanlösung von [6-Chlor-5-(o-fluorphenyl)-2,3-dihydro-1-methyl-2-oxo-1H-1,4-benzodiazepin-7-yl]isocyanat, erhalten in Analogie zu den Angaben in Absatz a) von Beispiel 1 aus 4 g (0,0126 M) 7-Amino-6-chlor-5-(o-fluorphenyl)-1,3-dihydro-1-methyl-2H-1,4-benzodiazepin-2-on, wird mit überschüssigem Diäthylamin versetzt, worauf das Gemisch eingeengt wird. Der Rückstand wird an einer 250 g-Kieselgelsäule mit Methylenchlorid-Aceton als Eluierungsmittel gereinigt und aus Essigester-Aether kristallisiert. Man erhält 3-[6-Chlor-5-(o-fluorphenyl)-2,3-dihydro-1-methyl-2-oxo-1H-1,4-benzodiazepin-7-yl]-1,1-diäthylharnstoff, welcher bei 190—191° schmilzt.

Beispiel 12

Eine Dichloräthanlösung von [6-Chlor-5-(o-fluorphenyl)-2,3-dihydro-1-methyl-2-oxo-1H-1,4-benzodiazepin-7-yl]isocyanat, erhalten in Analogie zu den Angaben in Absatz a) von Beispiel 1 aus 6 g (0,019 M) 7-Amino-6-chlor-5-(o-fluorphenyl)-1,3-dihydro-1-methyl-2H-1,4-benzodiazepin-2-on, wird mit 9 ml Methyläthylamin versetzt. Man rührt das Gemisch 20 Minuten bei Raumtemperatur, worauf die Dichloräthanlösung mehrmals mit Wasser gewaschen, mit Natriumsulfat getrocknet, filtriert und eingeengt wird. Der Rückstand wird an einer 200 g-Kieselgelsäule mit Methylenchlorid-Essigester (5:1) als Eluierungsmittel gereinigt und aus Essigester-Aether kristallisiert. Man erhält 3-[6-Chlor-5-(o-fluorphenyl)-2,3-dihydro-1-methyl-2-oxo-1H-1,4-benzodiazepin-7-yl]-1-äthyl-1-methylharnstoff, welcher bei 184—186°C schmilzt.

Beispiel 13

Eine Dichloräthanlösung von [6-Chlor-5-(o-fluorphenyl)-2,3-dihydro-1-methyl-2-oxo-1H-1,4-benzodiazepin-7-yl]isocyanat, erhalten in Analogie zu den Angaben in Absatz a) von Beispiel 1 aus 6 g (0,019 M) 7-Amino-6-chlor-5-(o-fluorphenyl)-1,3-dihydro-1-methyl-2H-1,4-benzodiazepin-2-on, wird mit 10 ml Aethanolamin in 30 ml 1,2-Dichloräthan versetzt. Das Gemisch wird 20 Minuten gerührt und dann eingeengt, worauf der Rückstand an einer 250 g-Kieselgelsäule mit Methylenchlorid-Aceton (10:1) als Eluierungsmittel gereinigt und aus Aethanol-Aether kristallisiert wird. Man erhält 1-[6-Chlor-5-(o-fluorphenyl)-2,3-dihydro-1-methyl-2-oxo-1H-1,4-benzodiazepin-7-yl]-3-(2-hydroxyäthyl)harnstoff, welcher bei 150—153° unter Zersetzung schmilzt.

Beispiel 14

Eine Dichloräthanlösung von [6-Chlor-5-(o-fluorphenyl)-2,3-dihydro-1-methyl-2-oxo-1H-1,4-benzodiazepin-7-yl]isocyanat, erhalten in Analogie zu den Angaben in Absatz a) von Beispiel 1 aus 4 g (0,012 M) 7-Amino-6-chlor-5-(o-fluorphenyl)-1,3-dihydro-1-methyl-2H-1,4-benzodiazepin-2-on, wird zu einer Lösung von 2,8 ml 2-Methylaminoäthanol in 50 ml Dichloräthan gegeben, worauf 20 Minuten gerührt und eingeengt wird. Der Rückstand wird an einer 200 g-Kieselgelsäule mit Methylenchlorid, Methylenchlorid-Essigester (3:1) und Methylenchlorid-Aceton (3:1) als Eluierungsmittel gereinigt. Nach Kristallisieren aus Aceton erhält man 3-[6-Chlor-5-(o-fluorphenyl)-2,3-dihydro-1-methyl-2-oxo-1H-1,4-benzodiazepin-7-yl]-1-(2-hydroxyäthyl)-1-methylharnstoff, welcher bei 218—220° schmilzt.

Beispiel 15

Eine Lösung von [6-Chlor-5-(o-fluorphenyl)-2,3-dihydro-1-methyl-2-oxo-1H-1,4-benzodiazepin-7-yl]isocyanat, erhalten in Analogie zu den Angaben in Absatz a) von Beispiel 1 aus 4 g (0,012 M) 7-Amino-6-chlor-5-(o-fluorphenyl)-1,3-dihydro-1-methyl-2H-1,4-benzodiazepin-2-on, wird zu einer Lösung von 3 ml Isopropylamin in 50 ml 1,2-Dichloräthan gegeben, worauf 20 Minuten gerührt und dann eingeengt wird. Der Rückstand wird an einer 260 g-Kieselgelsäule mit Methylenchlorid und dann Methylenchlorid-Essigester (5:1) als Eluierungsmittel gereinigt und aus Essigester-n-Hexan kristallisiert. Man erhält 1-[6-Chlor-5-(o-fluorphenyl)-2,3-dihydro-1-methyl-2-oxo-1H-1,4-benzodiazepin-7-yl]-3-isopropylharnstoff, welcher bei 130—140° schmilzt.

Beispiel 16

Eine Dichloräthanlösung von [6-Chlor-5-(o-fluorphenyl)-2,3-dihydro-1-methyl-2-oxo-1H-1,4-benzodiazepin-7-yl]isocyanat, erhalten in Analogie zu den Angaben in Absatz a) von Beispiel 1 aus 4 g (0,012 M) 7-Amino-6-chlor-5-(o-fluorphenyl)-1,3-dihydro-1-methyl-2H-1,4-benzodiazepin-2-on, wird zu einer Lösung von 2,5 ml tert.-Butylamin in 50 ml 1,2-Dichloräthan gegeben, worauf 15 Minuten gerührt und dann eingeengt wird. Der Rückstand wird an einer 240 g-Kieselgelsäule mit Methylenchlorid und dann Methylenchlorid-Essigester (3:1) als Eluierungsmittel gereinigt und aus Aether-n-Hexan kristallisiert. Man erhält 1-[6-Chlor-5-(o-fluorphenyl)-2,3-dihydro-1-methyl-2-oxo-1H-1,4-benzodiazepin-7-yl]-3-tert.-butylharnstoff, welcher bei 206—208° unter Zersetzung schmilzt.

Beispiel 17

Eine Dichloräthanlösung von [6-Chlor-5-(o-fluorphenyl)-2,3-dihydro-1-methyl-2-oxo-1H-1,4-benzodiazepin-7-yl]isocyanat, erhalten in Analogie zu den Angaben in Absatz a) von Beispiel 1 aus 6 g (0,019 M) 7-Amino-6-chlor-5-(o-fluorphenyl)-1,3-dihydro-1-methyl-2H-1,4-benzodiazepin-2-on, wird mit 9 ml N-Methylpiperazin versetzt. Hierauf wird das Reaktionsgemisch 20 Minuten bei Raumtemperatur gerührt, mit Wasser versetzt und mit Methylenchlorid extrahiert. Die organische Phase wird über Natriumsulfat getrocknet, filtriert und eingeengt. Der Rückstand wird an einer 250 g-Kieselgelsäule mit Methylenchlorid-Aethanol (10:1) als Eluierungsmittel gereinigt und aus Essigester-Aether-n-Hexan kristallisiert. Man erhält N-[6-Chlor-5-(o-fluorphenyl)-2,3-dihydro-1-methyl-2-oxo-1H-1,4-benzodiazepin-7-yl]-4-methyl-1-piperazincarboxamid, welches bei 154° unter Zersetzung schmilzt.

Beispiel 18

Eine Dichloräthanlösung von [6-Chlor-5-(o-fluorphenyl)-2,3-dihydro-1-methyl-2-oxo-1H-1,4-benzodiazepin-7-yl]isocyanat, erhalten in Analogue zu den Angaben in Absatz a) von Beispiel 1 aus, 6,66 g (0,021 M) 7-Amino-6-chlor-5-(o-fluorphenyl)-1,3-dihydro-1-methyl-2H-1,4-benzodiazepin-2-on, wird mit einer Lösung von 6,5 g p-Chloranilin in 50 ml 1,2-Dichloräthan versetzt. Hierauf wird 15 Minuten gerührt, wobei das Reaktionsprodukt schon teilweise auskristallisiert. Das Reaktionsgemisch wird auf Wasser gegossen, worauf man mit Eis kühlt, filtriert, das unlösliche Produkt in Methylenchlorid-Aethanol löst, filtriert und das Filtrat eingengt, bis das Produkt zu kristallisieren beginnt. Man erhält 1-[6-Chlor-5-(o-fluorphenyl)-2,3-dihydro-1-methyl-2-oxo-1H-1,4-benzodiazepin-7-yl]-3-(p-chlorphenyl)harnstoff, welcher bei 285° schmilzt.

Beispiel 19

Eine Dichloräthanlösung von [5-(o-Fluorphenyl)-2,3-dihydro-1-methyl-2-oxo-1H-1,4-benzodiazepin-7-yl]isocyanat, erhalten gemäss Angaben in Absatz a) von Beispiel 1 aus 8 g (0,028 M) 7-Amino-5-(o-fluorphenyl)-1,3-dihydro-1-methyl-2H-1,4-benzodiazepin-2-on, wird zu einer Lösung von 5,5 g Diäthylamin in 60 ml Acetonitril gegeben, worauf 20 Minuten gerührt und dann eingeengt wird. Der Rückstand wird an einer 400 g-Kieselgelsäule mit Methylenchlorid-Aceton (10:1) als Eluierungsmittel gereinigt und aus Aceton-Aether kristallisiert. Man erhält 1,1-Diäthyl-3-[5-(o-fluorphenyl)-2,3-dihydro-1-methyl-2-oxo-1H-1,4-benzodiazepin-7-yl]harnstoff, welcher bei 138—139° schmilzt.

Beispiel 20

Eine Dichloräthanlösung von [5-(o-Fluorphenyl)-2,3-dihydro-1-methyl-2-oxo-1H-1,4-benzodiazepin-7-yl]isocyanat, erhalten gemäss Angaben in Absatz a) von Beispiel 1 aus 4,2 g (0,015 M) 7-Amino-5-(o-fluorphenyl)-1,3-dihydro-1-methyl-2H-1,4-benzodiazepin-2-on, wird mit 3,5 ml Thiazolidin versetzt, worauf 20 Minuten bie Raumtemperatur gerührt, mit Wasser versetzt und mit Methylenchlorid extrahiert wird. Die Methylenchloridlösung wird über Natriumsulfat getrocknet, filtriert und eingeengt. Der Rückstand wird an einer 200 g-Kieselgelsäule mit Methylenchlorid-Essigester (5:1) als Eluierungsmittel gereinigt und als Methylenchlorid-Essigester kristallisiert. Man erhält N-[5-(o-Fluorphenyl)-2,3-dihydro-1-methyl-2-oxo-1H-1,4-benzodiazepin-7-yl]-3-thiazolidincarboxamid, welches bei 185° unter Zersetzung schmilzt.

Beispiel 21

Eine Dichloräthanlösung von [5-(o-Fluorphenyl)-2,3-dihydro-1-methyl-2-oxo-1H-1,4-benzodiazepin-7-yl]isocyanat, erhalten gemäss Angaben in Absatz a) von Beispiel 1 aus 4 g (0,014 M) 7-Amino-5-(o-fluorphenyl)-1,3-dihydro-1-methyl-2H-1,4-benzodiazepin-2-on, wird zu einer Lösung von 3 ml 2-Methylaminoäthanol in 50 ml 1,2-Dichloräthan gegeben, worauf 15 Minuten gerührt und dann eingeengt wird. Der Rückstand wird an einer 200 g-Kieselgelsäule mit Methylenchlorid und dann Methylenchlorid-Aceton (10:1) als Eluierungsmittel gereinigt und aus Aceton kristallisiert. Man erhält 3-[5-(o-Fluorphenyl)-2,3-dihydro-1-methyl-2-oxo-1H-1,4-benzodiazepin-7-yl]-1-(2-hydroxyäthyl)-1-methylharnstoff, welcher bei 196—198° unter Zersetzung schmilzt.

Beispiel 22

Eine Dichloräthanlösung von [5-(o-Fluorphenyl)-2,3-dihydro-1-methyl-2-oxo-1H-1,4-benzodiazepin-7-yl]isocyanat, erhalten gemäss Angaben in Absatz a) von Beispiel 1 aus 12,5 g (0,044 M) 7-Amino-5-(o-fluorphenyl)-1,3-dihydro-1-methyl-2H-1,4-benzodiazepin-2-on, wird mit einer Lösung von 8 g 3-Amino-1-propanol in 100 ml 1,2-Dichloräthan umgesetzt. Hierauf wird 15 Minuten gerührt, eingeengt und der Rückstand an einer 270 g-Kieselgelsäule mit Aceton als Eluierungsmittel gereinigt. Nach Kristallisieren aus Aceton erhält man 1-[5-(o-Fluorphenyl)-2,3-dihydro-1-methyl-2-oxo-1H-1,4-benzodiazepin-7-yl]-3-(3-hydroxypropyl)harnstoff, welcher bei 118—119° unter Zersetzung schmilzt.

## Beispiel 23

Eine Dichloräthanlösung von [5-(o-Fluorphenyl)-2,3-dihydro-1-methyl-2-oxo-1H-1,4-benzodiazepin-7-yl]isocyanat, erhalten gemäss Angaben in Absatz a) von Beispiel 1 aus 4 g (0,014 M) 7-Amino-5-(o-fluorphenyl)-1,3-dihydro-1-methyl-2H-1,4-benzodiazepin-2-on, wird zu einer Lösung von 2,5 ml 2-Amino-2-methyl-1-propanol in 50 ml 1,2-Dichloräthan gegeben, worauf 15 Minuten bei Raumtemperatur gerührt und dann eingeengt wird. Der Rückstand wird an einer 200 g-Kieselgelsäule mit Methylenchlorid und dann Methylenchlorid-Aceton (1:1) als Eluierungsmittel gereinigt. Man kristallisiert aus Aceton-Aether und erhält 1-[5-(o-Fluorphenyl)-2,3-dihydro-1-methyl-2-oxo-1H-1,4-benzodiazepin-7-yl]-3-(2-hydroxy-1,1-dimethyläthyl)harnstoff, welcher bei 162—176° unter Zersetzung schmilzt.

## Beispiel 24

Eine Dichloräthanlösung von [5-(o-Fluorphenyl)-2,3-dihydro-1-methyl-2-oxo-1H-1,4-benzodiazepin-7-yl]isocyanat, hergestellt gemäss Angaben in Absatz a) von Beispiel 1 aus 12,5 g (0,044 M) 7-Amino-5-(o-fluorphenyl)-1,3-dihydro-1-methyl-2H-1,4-benzodiazepin-2-on, wird zu einer Lösung von 8 g 1-Amino-2-propanol in 100 ml 1,2-Dichloräthan gegeben, worauf das Gemisch 15 Minuten bei Raumtemperatur gerührt und dann eingeengt wird. Der Rückstand wird an einer 300 g-Kieselgelsäule mit Methylenchlorid und dann Aceton als Eluierungsmittel gereinigt; diese Operation wird noch einmal mit Essigester als Eluierungsmittel wiederholt. Man kristallisiert hierauf aus Essigester und erhält rac. 1-[5 - (o - Fluorphenyl) - 2,3 - dihydro - 1 - methyl - 2 - oxo - 1H - 1,4 - benzodiazepin - 7 - yl] - 3 - (2-hydroxypropyl)harnstoff, welcher bei 149—151° unter Zersetzung schmilzt.

## Beispiel 25

a) 50 g Carbobenzoxy-DL-alanin werden in 400 ml abs. Tetrahydrofuran gelöst, unter Eiskühlung tropft wird. Anschliessend wird bei Raumtemperatur 18 Stunden gerührt, worauf die Lösung eingeengt Aufschlämmung von 50 g 2-Amino-5-nitro-2'-fluorbenzophenon in 200 ml abs. Tetrahydrofuran zugetropft wird. Aschliessend wird bei Raumtemperatur 18 Stunden gerührt, worauf die Lösung eingeengt und der Rückstand mit Eis und 10%iger Natriumbicarbonatlösung versetzt wird. Man extrahiert mit Methylenchlorid, trocknet die organische Lösung mit Natriumsulfat, filtriert und engt ein. Der Rückstand wird aus Aethanol kristallisiert und liefert rac. Benzyl-{1-/[2-(o-fluorbenzoyl)-4-nitrophenyl]carbamoyl/äthyl}carbamat, welches als Rohprodukt direkt wieterverarbeitet wird.

b) 70 g (0,15 M) des obigen Rohproduktes werden mit 200 ml 30—33%iger Bromwasserstofflösung in Eisessig versetzt, worauf eine halbe Stunde bei Raumtemperatur gerührt, eingeengt, mit Wasser versetzt und 2—3mal mit Aether extrahiert wird. Die wässerige Lösung wird in Eis gekühlt, mit festem Natriumbicarbonat neturalisiert und mit Methylenchlorid extrahiert, worauf der Methylenchloridextrakt mit Natriumsulfat getrocknet, filtriert und eingeengt wird. Der Rückstand wird mit 40 ml Eisessig und 400 ml Toluol versetzt, worauf 20 Minuten zum Rückfluss erhitzt und eingedampft wird. Das verbleibende Oel löst man in Methylenchlorid, wäscht mit gesättigter Natriumbicarbonatlösung, trocknet über Natriumsulfat, filtriert und engt ein. Man kristallisiert aus Essigester-Petroläther und erhält rac. 5-(o-Fluorphenyl)-1,3-dihydro-3-methyl-7-nitro-2H-1,4-benzodiazepin-2-on, welches bei 230—234° schmilzt.

c) 95 g (0,30 M) rac. 5-(o-Fluorphenyl)-1,3-dihydro-3-methyl-7-nitro-2H-1,4-benzodiazepin-2-on werden in 1 l abs. Aceton gelöst, mit 60 g Kaliumcarbonat (gemahlen) und 43 g Dimethylsulfat versetzt, worauf man das Gemisch 4 Stunden bei Raumtemperatur rührt und 48 Stunden im Eisschrank stehen lässt. Hierauf wird eingeengt, mit Eis-Wasser versetzt und mehrmals mit Methylenchlorid extrahiert. Die organische Lösung wird über Natriumsulfat getrocknet, filtriert und eingeengt. Der Rückstand wird aus Essigester-Petroläther kristallisiert, wobei man rac. 5-(o-Fluorphenyl)-1,3-dihydro-1,3-dimethyl-7-nitro-2H-1,4-benzodiazepin-2-on erhält, welches bei 154—156° schmilzt.

d) 85 g (0,26 M) rac. 5-(o-Fluorphenyl)-1,3-dihydro-1,3-dimethyl-7-nitro-2H-1,4-benzodiazepin-2-on werden in 700 ml konz. Salzsäure gelöst und langsam mit 180 g Zinn (II) chlorid versetzt. Nach etwa 15 Minuten wird mit Eis gekühlt und dann am Rotationsverdampfer eingeengt. Der Rückstand wird in Eis-Wasser gelöst, mit 200 g Soda in 1 l Wasser langsam alkalisch gestellt und mehrmals mit Methylenchlorid extrahiert. Die organische Lösung wird über Natriumsulfat getrocknet, filtriert und eingeengt. Man kristallisiert den Rückstand aus Essigester und erhält rac. 7-Amino-5-(o-fluorphenyl)-1,3-dihydro-1,3-dimethyl-2H-1,4-benzodiazepin-2-on, welches bei 178—180° schmilzt.

e) Eine Dichloräthanlösung von rac. [5-(o-Fluorphenyl)-2,3-dihydro-1,3-dimethyl-2-oxo-1H-1,4-benzodiazepin-7-yl]isocyanat, erhalten in Analogie zu den Angaben in Absatz a) von Beispiel 1 aus 5 g (0,017 M) rac. 7-Amino-5-(o-fluorphenyl)-1,3-dihydro-1,3-dimethyl-2H-1,4-benzodiazepin-2-on, wird unter Eiskühlung schnell mit überschüssigem gasförmigem Methylamin versetzt, worauf 15 Minuten bei Raumtemperatur gerührt wird. Das Reaktionsgemisch wird mit Wasser versetzt und mit Methylenchlorid extrahiert. Die organische Phase wird über Natriumsulfat getrocknet, filtriert und eingeengt. Der Rückstand wird an einer 250 g-Kieselgelsäule mit Methylenchlorid-Essigester und dann mit Essigester als Eluierungsmittel gereinigt und aus Aether-n-Hexan kristallisiert. Man erhält rac. 1-[5-(o-Fluorphenyl)-2,3-dihydro-1,3-dimethyl-2-oxo-1H-1,4-benzodiazepin-7-yl]-3-methylharnstoff, welcher bei 109—111° unter Zersetzung schmilzt.

Beispiel 26

a) 120 g Carbobenzoxy-L-alanin werden in 800 ml abs. Tetrahydrofuran gelöst, unter Eiskühlung tropfenweise mit 70 g Thionylchlorid versetzt und 40 Minuten unter Eiskühlung gerührt. Hierzu wird eine Aufschlämmung von 100 g (0,38 M) 2-Amino-5-nitro-2′-fluorbenzophenon in 400 ml abs. Tetrahydrofuran schnell zugetropft und darauf bei Raumtemperatur 24 Stunden gerührt. Die erhaltene Lösung wird eingeengt, und der Rückstand wird mit Eis und 10%iger Natriumbicarbonatlösung versetzt und mit Methylenchlorid extrahiert. Die organische Lösung wird über Natriumsulfat getrocknet, filtriert und eingeengt. Der Rückstand wird in wenig Methylenchlorid gelöst und mit Aether versetzt. Man erhält (S)-Benzyl-{1-/[2-(o-fluorbenzoyl)-4-nitrophenyl]carbamoyl/äthyl}carbamat. Eine zusätzliche Umkristallisation aus Methylenchlorid-Aether liefert ein Produkt, welches bei 158—160° schmilzt und eine Drehung von $[\alpha]^{D}_{25}$ = —23,4° (in Methylenchlorid; 1%ig) aufweist. Die Mutterlaugen werden an einer 600 g-Kieselgelsäule mit Methylenchlorid als Eluierungsmittel gereinigt, wobei noch eine zusätzliche Menge des obigen Produktes gewonnen wird.

b) Man löst 110 g (0,24 M) (S)-Benzyl{1-/[2-(o-fluorbenzoyl)-4nitrophenyl]carbamoyl/äthyl}carbamat in 400 ml 30—33%iger Bromwasserstofflösung in Eisessig, gibt 30 ml Methylenchlorid dazu, rührt 45 Minuten bei Raumtemperatur, engt ein, versetzt mit Wasser und extrahiert 2—3mal mit Aether. Die wässerige Lösung wird in Eis gekühlt, mit festem Natriumbicarbonat neutralisiert, mit Methylenchlorid extrahiert, über Natriumsulfat getrocknet, filtriert und eingeengt. Der Rückstand wird mit 50 ml Eisessig und 500 ml Toluol versetzt, worauf 15 bis 20 Minuten zum Rückfluss erhitzt und eingedampft wird. Das zurückbleibende Oel wird in Methylenchlorid gelöst, und die erhaltene Lösung wird mit gesättigter Natriumbicarbonatlösung gewaschen, über Natriumsulfat getrocknet, filtriert und eingeengt. Der Rückstand wird in 400 ml Benzol gelöst und mit racemischem 5-(o-Fluorphenyl)-1,3-dihydro-3-methyl-7-nitro-2H-1,4-benzodiazepin-2-on angeimpft. Man lässt über Nacht bei Raumtemperatur stehen, wobei 2 g Racemat auskristallisieren. Die Mutterlauge wird eingeengt und mit Aether versetzt. Durch Kristallisation aus Aether erhält man (S)-5-(o-Fluorphenyl)-1,3-dihydro-3-methyl-7-nitro-2H-1,4-benzodiazepin-2-on, welches bei 130—134° schmilzt; $[\alpha]^{D}_{25}$ = +377,2° (in Methylenchlorid; 1%ig).

c) 57,5 g (0,18 M) (S)-5-(o-Fluorphenyl)-1,3-dihydro-3-methyl-7-nitro-2H-1,4-benzodiazepin-2-on werden in 600 ml abs. Aceton gelöst, worauf mit 48 g Kaliumcarbonat (gemahlen) und 21 ml Methyljodid versetzt und 5 Stunden bei Raumtemperatur gerührt wird. Das Reaktionsgemisch wird eingeengt, und der Rückstand wird mit Eis-Wasser versetzt und mehrmals mit Methylenchlorid extrahiert; die Methylen-chloridlösung wird mit Wasser gewaschen, über Natriumsulfat getrocknet, filtriert und eingeengt. Der Rückstand wird aus Aether-Petroläther kristallisiert, und man erhält (S)-5-(o-Fluorphenyl)-1,3-dihydro-1,3-dimethyl-7-nitro-2H-1,4-benzodiazepin-2-on, welches bei 118—126° unter Zersetzung schmilzt; $[\alpha]^{D}_{25}$ = +563,1° (in Methylenchlorid; 1%ig).

d) In 10 ml konz. Salzsäure werden unter Eiskühlung bei 0° 1 g (S)-5-(o-Fluorphenyl)-1,3-dihydro-1,3-dimethyl-7-nitro-2H-1,4-benzodiazepin-2-on portionsweise eingetragen. Die erhaltene Lösung wird bei 0° langsam mit 2 g Zinn (II)-chlorid versetzt, unter Eiskühlung 30 Minuten gerührt, auf Eis/Natriumbicarbonatlösung geschüttet und mit Methylenchlorid mehrmals extrahiert, worauf die organische Phase über Natriumsulfat getrocknet, filtriert und eingeengt wird. Man kristallisiert aus Aether und erhält (S)-7-Amino-5-(o-fluorphenyl)-1,3-dihydro-1,3-dimethyl-2H-1,4-benzodiazepin-2-on, welches mit 1 Mol Aether kristallisiert; Smp. 100—110° (unter Zersetzung), $[\alpha]^{D}_{25}$ = +77,1° (in Methylenchlorid; 1%ig).

e) In 35 ml eisgekühlter konz. Salzsäure werden bei —10° 4 g (0,011 M) (S)-7-Amino-5-(o-fluorphenyl)-1,3-dihydro-1,3-dimethyl-2H-1,4-benzodiazepin-2-on gelöst, worauf während 1 Stunde bei —10° Chlor-Gas engeleitet wird. Hierauf wird bei 0—5° unter Verwendung einer Hochvakuumkühlfalle und eines Natronkalkturms unter Hochvakuum (1—2 mmHg; 1,33—2,66 mbar) eingeengt. Der Rückstand wird mit einem Gemisch von Eis und 10%iger Natriumbicarbonatlösung alkalisch gestellt, worauf mit Methylenchlorid extrahiert und der Extrakt über Natriumsulfat getrocknet, filtriert und eingeengt wird. Das übrigbleibende Oel wird an einer 300 g-Kieselgelsäule mit Methylenchlorid und dann mit Methylenchlorid-Essigester (20:1) als Eluierungsmittel gereinigt; diese Operation wird noch einmal an einer 120 g-Kieselgelsäule mit Methylenchlorid und dann mit Methylenchlorid-Essigester (20:1) als Eluierungsmittel wiederholt. Man kristallisiert aus Essigester-Aether und erhält (S)-7-Amino-6-chlor-5-(o-fluorphenyl)-1,3-dihydro-1,3-dimethyl-2H-1,4-benzodiazepin-2-on, welches bei 222—226° schmilzt; $[\alpha]^{D}_{25}$ = +92° (in Methylenchlorid; 0.5%ig).

f) In einem Sulfierkolben wird eine Lösung von 2 g Phosgen in 20 ml eisgekühltem 1,2-Dichloräthan vorgelegt. Hierzu wird eine Lösung von 4 g (0,012 M) (S)-7-Amino-6-chlor-5-(o-fluorphenyl)-1,3-dihydro-1,3-dimethyl-2H-1,4-benzodiazepin-2-on in 40 ml 1,2-Dichloräthan unter Eiskühlung und Rühren derart zugetropft, dass die Reaktions-temperatur 10° nicht übersteigt. Anschliessend wird das Reaktionsgemisch 45 Minuten bei Raumtemperatur gerührt. Darauf wird die erhaltene Lösung von 2H-1,4-benzodiazepin-2-on in 40 ml 1,2-Dichloräthan unter Eiskühlung und Rühren derart zugetropft, (S)-]6-Chlor-5-(o-fluorphenyl)-2,3-dihydro-1,3-dimethyl-2-oxo-1H-1,4-benzodiazepin-7-yl]-isocyanat mit Eis gekühlt und mit 6 ml Triäthylamin langsam alkalisch gestellt. Eine Lösung von 2 ml 2-Aminoäthanol in 20 ml 1,2-Dichloräthan wird mit der oben vorbereiteten Lösung versetzt, worauf das Gemisch 30 Minuten bei Raumtemperatur gerührt und dann eingeengt wird. Der Rückstand wird an einer

300 g-Kieselgelsäule mit Methylenchlorid und dann mit Methylenchlorid-Aethanol (20:1) als Eluierungsmittel gereinigt und aus wenig Essigester-n-Hexan kristallisiert. Man erhält (S)-1-[6-Chlor-5-(o-fluorphenyl)-2,3-dihydro-1,3-dimethyl-2-oxo-1H-1,4-benzodiazepin-7-yl]-3-(2-hydroxyäthyl)harnstoff, welcher oberhalb von 147° unter Zersetzung schmilzt; $[\alpha]^D_{25} = +192,2°$ (in Dioxan; 1%ig).

Beispiel 27

a) Aus 120 g Carbobenzoxy-D-alanin und 100 g (0,38 M) 2-Amino-5-nitro-2'-fluorbenzophenon wird in Analogie zur Arbeitsvorschrift in Absatz a) von Beispiel 26 (R)-Benzyl-{1-/[2-(o-fluorbenzoyl)-4-nitrophenyl]carbamoyl/äthyl}-carbamat erhalten. Aus Methylenchlorid-Aether kristallisiert schmilzt das Produkt bei 158—160°; $[\alpha]^D_{25} = +16,5°$.

b) Aus 90 g (0,19 M) (R)-Benzyl-{1-/[2-(o-fluorbenzoyl)-4-nitrophenyl]carbamoyl/äthyl}carbamat wird in Analogie zur Arbeitsvorschrift in Absatz b) von Beispiel 26 (R)-5-(o-Fluorphenyl)-1,3-dihydro-3-methyl-7-nitro-2H-1,4-benzodiazepin-2-on erhalten. Aus Aether/n-Hexan kristallisiert schmilzt das Produkt bei 130—140°; $[\alpha]^D_{25} = -361,9°$ (in Methylenchlorid; 1%ig).

c) Aus 44,6 g (0,14 M) (R)-5-(o-Fluorphenyl)-1,3-dihydro-3-methyl-7-nitro-2H-1,4-benzodiazepin-2-on wird in Analogie zur Arbietsvorschrift in Absatz c) von Beispiel 26 (R)-5-(o-Fluorphenyl)-1,3-dihydro-1,3-dimethyl-7-nitro-2H-1,4-benzodiazepin-2-on erhalten. Aus Aether-Hexan kristallisiert schmilzt das Produkt bei 120°; $[\alpha]^D_{25} = -540,7°$ (in Methylenchlorid; 1%ig).

d) Aus 5 g (0,015 M) (R)-5-(o-Fluorphenyl)-1,3-dihydro-1,3-dimethyl-7-nitro-2H-1,4-benzodiazepin-2-on wird in Analogie zur Arbeitsvorschrift in Absatz d) von Beispiel 26 7-Amino-5-(o-fluorphenyl)-1,3-dihydro-1,3-dimethyl-2H-1,4-benzodiazepin-2-on erhalten. Aus Aether-Petroläther kristallisiert schmilzt das Produkt bei 95—110° unter Zersetzung (1 Mol Aether kristallisiert mit); $[\alpha]^D_{25} = -78,4°$ (in Methylenchlorid; 1%ig).

e) Aus 10 g (0,027 M) (R)-7-Amino-5-(o-fluorphenyl)-1,3-dihydro-1,3-dimethyl-2H-1,4-benzodiazepin-2-on wird in Analogie zur Arbeitsvorschrift in Absatz e) von Beispiel 26 (R)-7-Amino-6-chlor-5-(o-fluorphenyl)-1,3-dihydro-1,3-dimethyl-2H-1,4-benzodiazepin-2-on erhalten, welches aus Aether umkristallisiert wird und dann bei 224—226° schmilzt; $[\alpha]^D_{25} = -95,4°$ (in Dioxan; 1%ig).

f) Aus 4 g (0,012 M) (R)-7-Amino-6-chlor-5-(o-fluorphenyl)-1,3-dihydro-1,3-dimethyl-2H-1,4-benzodiazepin-2-on wird in Analogie zur Arbeitsvorschrift in Absatz f) von Beispiel 26 (R)-1-[6-Chlor-5-(o - fluorphenyl) - 2,3-dihydro-1,3-dimethyl-2-oxo-1H-1,4-benzodiazepin-7-yl]-3-(2-hydroxyäthyl)harnstoff erhalten, welcher oberhalb von 100° unter Zersetzung schmilzt (aus wenig Essigester-n-Hexan umkristallisiert); $[\alpha]^D_{25} = -188,8°$ (in Dioxan; 1%ig).

Beispiel 28

a) Aus 56 g Carbobenzoxy-DL-$\alpha$-aminobuttersäure und 54 g (0,21 M) 2-Amino-5-nitro-2'-fluorbenzophenon erhält man in Analogie zur Arbeitsvorschrift in Absatz a) von Beispiel 25 rac. Benzyl-{1-/[2-(o-fluorbenzoyl)-4-nitrophenyl]carbamoyl/propyl}carbamat, welches aus Aether kristallisiert wird und dann bei 135—136°C schmilzt.

b) Aus 60 g (0,125 M) rac. Benzyl-{1-/[2-(o-fluorbenzoyl)-4-nitrophenyl]-carbamoyl/propyl}carbamat erhält man in Analogie zur Arbeitsvorschrift in Absatz b) von Beispiel 25 rac-3-Aethyl-5-(o-fluorphenyl)-1,3-dihydro-7-nitro-2H-1,4-benzodiazepin-2-on, welches aus Methylenchlorid kristallisiert wird und dann bei 260° unter Zersetzung schmilzt.

c) 30 g (0,092 M) rac. 3-Aethyl-5-(o-fluorphenyl)-1,3-dihydro-7-nitro-2H-1,4-benzodiazepin-2-on werden in 200 ml abs. Aceton gelöst und mit 16 g gemahlenem Kaliumcarbonat und 8 ml Methyljodid versetzt, worauf das Gemisch bei Raumtemperatur gerührt wird. Nach einer Stunde werden nocheinmal 4 ml Methyljodid dazugegeben und noch zusätzlich 2 1/2 Stunden gerührt. Die ungelösten anorganischen Salze werden abgesaugt, und die Lösung wird eingeengt. Der Rückstand wird mit Wasser versetzt, worauf man mit Methylenchlorid extrahiert und den Extrakt mit Natriumsulfat trocknet, filtriert und einengt. Man kristallisiert aus Essigester-Petroläther und erhält rac. 3-Aethyl-5-(o-fluorphenyl)-1,3-dihydro-1-methyl-7-nitro-2H-1,4-benzodiazepin-2-on, welches bei 175—176° schmilzt.

d) Aus 4 g (0,012 M) rac. 3-Aethyl-5-(o-fluorphenyl)-1,3-dihydro-1-methyl-7-nitro-2H-1,4-benzodiazepin-2-on erhält man in Analogie zur Arbeitsvorschrift in Absatz d) von Beispiel 25 rac . 7-Amino-3-äthyl-5-(o-fluorphenyl)-1,3-dihydro-1-methyl-2H-1,4-benzodiazepin-2-on, welches aus Essigester kristallisiert wird und dann bei 185—186° schmilzt.

e) 9,3 g (0,03 M) rac. 7-Amino-3-äthyl-5-(o-fluorphenyl)-1,3-dihydro-1-methyl-2H-1,4-benzodiazepin-2-on werden in 80 ml Eissesig gelöst. Die Lösung wird auf 10° abgekühlt, tropfenweise mit 5 g (1,62 ml) Brom versetzt, nach der Zugabe von Brom noch 10 Minuten bei 10° gerührt und dann eingeengt. Der Rückstand wird mit einer Mischung von Eis und 10%iger Natriumcarbonatlösung versetzt, worauf mit Methylenchlorid extrahiert und der Extrakt über Natriumsulfat getrocknet, filtriert und eingeengt wird. Das zurückgebliebene Oel wird an einer 200 g-Kieselgelsäule mit Methylenchlorid-Essigester (10:1) gereinigt und aus Aether kristallisiert. Man erhält rac. 7-Amino-3-äthyl-6-brom-5-(o-fluorphenyl)-1,3-dihydro-1-methyl-2H-1,4-benzodiazepin-2-on, welches bei 248° schmilzt.

f) Eine Lösung von rac-[7-Amino-3-äthyl-6-brom-5-(o-fluorphenyl)-2,3-dihydro-1-methyl-2-oxo-1H-1,4-benzodiazepin-7-yl]isocyanat, erhalten aus 8,5 g (0,022 M) rac-7-Amino-3-äthyl-6-brom-(o-fluorphenyl)-1,3-dihydro-1-methyl-2H-1,4-benzodiazepin-2-on in Analogie zur Arbeitsvorschrift in Absatz a) von Beispiel 1, wird zu einer Lösung von 8,5 ml 2-Aminoäthanol in 40 ml 1,2-Dichloräthan gegeben, worauf 15 Minuten gerührt und dann eingeengt wird. Der Rückstand wird an einer 400 g-Kieselgelsäule mit Methylenchlorid/Aceton als Eluierungsmittel gereinigt und aus Aceton/Aether kristallisiert. Man erhält rac. 1-[3-Aethyl-6-brom-5-(o-fluorphenyl)-2,3-dihydro-1-methyl-2-oxo-1H-1,4-benzodiazepin-7-yl]-3-(2-hydroxyäthyl)harnstoff, welcher bei 218—222° schmilzt.

Beispiel 29

a) 13 g (0,042 M) rac. 7-Amino-3-äthyl-5-(o-fluorphenyl)-1,3-dihydro-1-methyl-2H-1,4-benzodiazepin-2-on werden in 70 ml konz. Salzsäure bei —10° gelöst, worauf so lange bei —10° Chlorgas eingeleitet wird, bis das Ausgangsprodukt dünnschichtchromatographisch nicht mehr nachgewiesen werden kann. Das Reaktionsgemisch wird langsam mit einer Mischung von Eis und 10%iger Sodalösung neutralisiert, worauf man mehrmals mit Methylenchlorid extrahiert. Die organische Lösung wird mit Wasser gewaschen, über Natriumsulfat getrocknet, filtriert und eingeengt. Der Rückstand wird an einer 300 g-Kieselgelsäule mit Methylenchlorid/Essigester (10:1) gereinigt und aus Aethanol kristallisiert. Man erhält rac. 7-Amino-3-äthyl-6-chlor-5-(o-fluorphenyl)-1,3-dihydro-1-methyl-2H-1,4-benzodiazepin-2-on, welches bei 250° schmilzt.

b) Eine Lösung von [3-Aethyl-6-chlor-5-(o-fluorphenyl)-2,3-dihydro-1-methyl-2-oxo-1H-1,4-benzodiazepin-7-yl]isocyanat, erhalten aus 3,4 g (0,01 M) rac. 7-Amino-3-äthyl-6-chlor-5-(o-fluorphenyl)-1,3-dihydro-1-methyl-2H-1,4-benzodiazepin-2-on in Analogie zu den Angaben in Absatz a) von Beispiel 1, wird zu einer Lösung von 2 ml 2-Aminoäthanol in 50 ml 1,2-Dichloräthan gegeben, worauf 15 Minuten bei Raumtemperatur gerührt und dann eingeengt wird. Der Rückstand wird an einer 200 g-Kieselgelsäule mit Methylenchlorid-Essigester (1:1) und dann mit Essigester als Eluierungsmittel gereinigt und aus Aceton/Aether kristallisiert. Man erhält rac. 1-[3-Aethyl-6-chlor-5-(o-fluorphenyl)-2,3-dihydro-1-methyl-2-oxo-1H-1,4-benzodiazepin-7-yl]-3-(2-hydroxyäthyl)harnstoff, welcher bei 243—244° schmilzt

Beispiel 30

a) 19,7 g N-Carbobenzoxy-DL-norleucin werden in 200 ml abs. Tetrahydrofuran gelöst, worauf unter Eiskühlung tropfenweise mit 10 g Thionylchlorid versetzt und dann 1 Stunde gerührt wird. Zu dieser Lösung wird schnell eine Lösung von 17 g (0,065 M) 2-Amino-5-nitro-2'-fluorbenzophenon in 130 ml abs. Tetrahydrofuran zugetropft und anschliessend bei Raumtemperatur 60 Stunden gerührt. Die Lösung wird eingeengt, worauf der Rückstand mit Eis und 10%iger Natriumbicarbonatlösung versetzt und mit Methylenchlorid extrahiert wird. Die organische Lösung wird mit Natriumsulfat getrocknet, filtriert und eingeengt. Der Rückstand wird an einer 450 g-Kieselgelsäule mit Methylenchlorid als Eluierungsmittel gereinigt und aus Aethanol kristallisiert. Man erhält rac. Benzyl-{-/[2-(o-fluorbenzoyl)-4-nitrophenyl]carbamoyl]butyl}carbamat welches bei 148—150° schmilzt.

b) Aus 24,5 g (0,05 M) rac. Benzyl-{1-/[2-(o-fluorbenzoyl)-4-nitrophenyl]-carbamoyl/butyl}-carbamat wird in Analogie zu den Angaben in Absatz b) von Beispiel 25 rac. 5-(o-Fluorphenyl)-1,3-dihydro-7-nitro-3-propyl-2H-1,4-benzodiazepin-2-on erhalten, welches aus Methylenchlorid/Aethanol kristallisiert wird und dann bei 245—246° schmilzt.

c) Aus 13,6 g (0,04 M) rac-5-(o-Fluorphenyl)-1,3-dihydro-7-nitro-3-propyl-2H-1,4-benzodiazepin-2-on wird in Analogie zu den Angaben in Absatz c) von Beispiel 28 rac. 5-(o-Fluorphenyl)-1,3-dihydro-1-methyl-7-nitro-3-propyl-2H-1,4-benzodiazepin-2-on erhalten, welches aus Aether/Petroläther kristallisiert wird und dann bei 128—130° schmilzt.

d) Aus 11,7 g (0,033 M) rac. 5-(o-Fluorphenyl)-1,3-dihydro-1-methyl-7-nitro-3-propyl-2H-1,4-benzodiazepin-2-on wird in Analogie zu den Angaben in Absatz d) von Beispiel 25 rac. 7-Amino-5-(o-fluorphenyl)-1,3-dihydro-1-methyl-3-propyl-2H-1,4-benzodiazepin-2-on erhalten, welches aus Aether kristallisiert wird und dann bei 112—116° schmilzt.

e) Eine Lösung von rac. [5-(o-Fluorphenyl)-2,3-dihydro-1-methyl-2-oxo-3-propyl-1H-1,4-benzodiazepin-7-yl]isocyanat, erhalten aus 4 g (0,012 M) rac-7-Amino-5-(o-fluorphenyl)-1,3-dihydro-1-methyl-3-propyl-2H-1,4-benzodiazepin-2-on in Analogie zu den Angaben in Absatz a) von Beispiel 1, wird zu einer Lösung von 2 ml 2-Amino-äthanol in 50 ml 1,2-Dichloräthan gegeben, worauf 15 Minuten bei Raumtemperatur gerührt und dann eingeengt wird. Der Rückstand wird an einer 250 g-Kieselgelsäule mit Methylenchlorid und dann mit Methylenchlorid/Aethanol (20:1) gereinigt und aus Essigester/n-Hexan kristallisiert. Man erhält rac. 1-[5-(o-Fluorphenyl)-2,3-dihydro-1-methyl-2-oxo-3-propyl-1H-1,4-benzodiazepin-7-yl]-3-(2-hydroxyäthyl)harnstoff, welcher bei 105—110° unter Zersetzung schmilzt.

Beispiel 31

a) Aus 94 g N-Carbobenzoxy-DL-$\alpha$-Aminobuttersäure und 93 g (0,34 M) 2-Amino-5-nitro-2'-chlorbenzophenon wird in Analogie zu den Angaben in Absatz a) von Beispiel 30 rac. Benzyl-{1-/[2-(o-chlorbenzoyl)-4-nitrophenyl]carbamoyl/propyl}carbamat erhalten, welches aus Aether/n-Hexan kristallisiert wird und dann bei 142° schmilzt.

b) Aus 70 g (0,14 M) rac. Benzyl-{1-/[2-(o-chlorbenzoyl)-4-nitrophenyl]-carbamoyl/propyl}carbamat wird in Analogie zu den Angaben in Absatz b) von Beispiel 25 rac. 3-Aethyl-5-(o-chlorphenyl)-1,3-dihydro-7-nitro-2H-1,4-benzodiazepin-2-on erhalten, welches aus Methylenchlorid/Aether kristallisiert wird und dann bei 242—243° schmilzt.

c) Aus 46,9 g (0,14 M) rac. 3-Aethyl-5-(o-chlorphenyl)-1,3-dihydro-7-nitro-2H-1,4-benzodiazepin-2-on wird in Analogie zu den Angaben in Absatz c) von Beispiel 28, jedoch nach 16 stündigem Rühren bei Raumtemperatur und anschliessendem 3-stündigem Rühren bei 40°, rac. 3-Aethyl-5-(o-chlorphenyl)-1,3-dihydro-1-methyl-7-nitro-2H-1,4-benzodiazepin-2-on erhalten. Aus Essigester/-Aether umkristallisiert schmilzt das Produkt bei 161—162°.

d) 7,7 g (0,021 M) rac. 3-Aethyl-5-(o-chlorphenyl)-1,3-dihydro-1-methyl-7-nitro-2H-1,4-benzodiazepin-2-on werden in 85 ml konz. Salzsäure gelöst, mit 18 g Zinn(II)chlorid versetzt und 30 Minuten bei Raumtemperatur gerührt. Das Reaktionsgemisch wird auf eine Mischung von Eis und Sodalösung geschüttet, worauf mit Methylenchlorid extrahiert und der Extrakt über Natriumsulfat getrocknet, filtriert und eingeengt wird. Nach Kristallisation aus Essigester/Aether erhält man rac. 7-Amino-3-äthyl-5-(o-chlorphenyl)-1,3-dihydro-1-methyl-2H-1,4-benzodiazepin-2-on, welches bei 198—199° schmilzt.

e) 9,6 g (0,029 M) rac. 7-Amino-3-äthyl-5-(o-chlorphenyl)-1,3-dihydro-1-methyl-2H-1,4-benzodiazepin-2-on, gelöst in 50 ml konz. wässriger Bromwasserstoffsäure, werden zwischen 0—5° langsam mit 5,2 g (1,65 ml) Brom versetzt, wonach 1 Stunde bei 0° gerührt wird. Anschliessend wird das Reaktionsgemisch auf eine Mischung von Eis und Sodalösung geschüttet, worauf mit Methylenchlorid extrahiert und der Extract über Natriumsulfat getrocknet, filtriert und eingeengt wird. Man kristallisiert aus Methylenchlorid/Aether/Petroläther und erhält rac. 7-Amino-3-äthyl-6-brom-5-(o-chlorphenyl)-1,3-dihydro-1-methyl-2H-1,4-benzodiazepin-2-on, welches bei 251° unter Zersetzung schmilzt.

f) Eine Lösung von rac. [3-Aethyl-6-brom-5-(o-chlorphenyl)-1-methyl-2-oxo-2,3-dihydro-1H-1,4-benzodiazepin-7-yl]isocyanat, erhalten aus 4 g (0,01 M) rac-7-Amino-3-äthyl-6-brom-5-(o-chlorphenyl)-1,3-dihydro-1-methyl-2H-1,4-benzodiazepin-2-on in Analogie zu den Angaben in Absatz a) von Beispiel 1, wird zu einer Lösung von 2,5 ml 2-Amino-äthanol in 50 ml 1,2-Dichloräthan gegeben. Hierauf wird 15 Minuten bei Raumtemperatur gerührt und dann eingeengt. Der Rückstand wird an einer 250 g-Kieselgelsäule mit Methylenchlorid und dann mit Methylenchlorid/Aceton (10:1) als Eluierungsmittel gereinigt us aus Aceton kristallisiert. Man erhält rac. 1-[3-Aethyl-6-brom-5-(o-chlorphenyl)-2,3-dihydro-1-methyl-2-oxo-1H-1,4-benzodiazepin-7-yl]-3-(2-hydroxyäthyl)harnstoff, welcher bei 236—237° schmilzt.

Beispiel 32

4,2 g (0,015 M) 7-Amino-5-(o-fluorphenyl)-1,3-dihydro-1-methyl-2H-1,4-benzodiazepin-2-on werden in 60 ml abs. Methylenchlorid suspendiert und mit 2,1 g gemahlenem Kaliumcarbonat und 1,6 ml Dimethylcarbamoylchlorid versetzt, worauf aus Gemisch 14 Tage bei 20° gerührt wird. Das Reaktionsgemisch wird danach auf eine Mischung von eis und Wasser gegossen, worauf mit Methylenchlorid extrahiert und die organische Phase 2—3 mal mit Wasser gewaschen, mit Natriumsulfat getrocknet, filtriert und eingeengt wird. Der Rückstand wird an einer 200 g-Kieselgelsäule mitr Essigester als Eluierungsmittel gereinigt und aus Aethanol/Petroläther kristallisiert. Man erhält 3-[5-(o-Fluorphenyl)-2,3-dihydro-1-methyl-2-oxo-1H-1,4-benzodiazepin-7-yl]-1,1-dimethylharnstoff, welcher mit 0,5 Mol Aethanol kristallisiert und bei 97° unter Zersetzung schmilzt.

Beispiel 33

4,2 g (0,015 M) 7-Amino-5-(o-fluorphenyl)-1,3-dihydro-1-methyl-2H-1,4-benzodiazepin-2-on werden in 50 ml abs. Methylenchlorid suspendiert und mit 2,5 ml p-Methoxyphenylisocyanat und 2 Tropfen Triäthylamin versetzt, worauf über Nacht bei Raumtemperatur gerührt wird. Das Reacktionsgemisch wird teilweise eingeengt und mit Essigester versetzt, wobei 1-[5-(o-Fluorphenyl)-2,3-dihydro-1-methyl-2-oxo-1H-1,4-benzodiazepin-7-yl]-3-(p-methoxyphenyl)harnstoff auskristallisiert. Das Produkt schmilzt bei 240° unter Zersetzung.

Beispiel 34

5 g (0,016 M) 7-Amino-6-chlor-5-(o-fluorphenyl)-1,3-dihydro-1-methyl-2H-1,4-benzodiazepin-2-on werden mit 15 ml Aethylisocyanat unter Stickstoff 7 Stunden am Rückfluss erhitzt. Das Reaktionsgemisch wird auf Methanol/Eiswasser gegossen, worauf man mit Methylenchlorid extrahiert. Die organische Phase wird abgetrennt, mit Natriumsulfat getrocknet, filtriert und eingeengt. Der Rückstand wird an einer 300 g-Kieselgelsäule mit Essigester als Eluierungsmittel gereinigt und aus Essigester/Aether kristallisiert. Man erhält 1-[6-Chlor-5-(o-fluorphenyl)-2,3-dihydro-1-methyl-2-oxo-1H-1,4-benzodiazepin7-yl]-3-äthylharnstoff, welcher bei 155—160° unter Zersetzung schmilzt.

Beispiel 35

5 g (0,018 M) 7-Amino-1,3-dihydro-1-methyl-5-(o-fluorphenyl)-2H-1,4-benzodiazepin-2-on werden in 50 ml abs. Tetrahydrofuran mit 15 ml Isopropylisocyanat und einer katalytischen Menge Triäthylamin während 5 Stunden am Rückfluss erhitzt. Das Reaktionsgemisch wird 2 1/2 Tage bei Raumtemperatur stehengelassen, wobei das Endprodukt teilweise kristallisiert. Das Lösungsmittel wird

teilweise eingeengt, worauf mit Eis gekühlt und das kristallisierte Produkt abfiltriert wird. Man erhält nach Umkristallisieren aus Methylenchlorid/Essigester 1-[5-(o-Fluorphenyl)-2,3-dihydro-1-methyl-2-oxo-1H-1,4-benzodiazepin-7-yl]-3-isopropylharnstoff, welcher bei 185—188° unter Zersetzung schmilzt.

Beispiel 36

11,1 g (0,03 M) 1-[5-(o-Fluorphenyl)-2,3-dihydro-1-methyl-2-oxo-1H-1,4-benzodiazepin-7-yl]3-(2-hydroxyaethyl)harnstoff werden in 150 ml Acetonitril mit 3,9 ml Acetylchlorid und 9 g gemahlenem Kaliumcarbonat 18 Stunden bei Raumtemperatur gerührt. Das Reaktionsgemisch wird am Rotationsverdampfer eingeengt und der Rückstand an einer 500 g-Kieselgelsäule mit Methylenchlorid/Aethanol als Eluierungsmittel gereinigt. Man kristallisiert aus Essigester und erhält 2-/3-[5-(o-Fluorphenyl)-2,3-dihydro-1-methyl-2-oxo-1H-1,4-benzodiazepin-7-yl]ureido/äthylacetat, welches bei 196—198° schmilzt.

Beispiel 37

a) 45 g Carbobenzoxy-glycin, gelöst in 1 l abs. Tetrahydrofuran, werden unter Eiskühlung tropfenweise mit 20 ml Thionylchlorid versetzt, worauf 1 Stunde unter Eiskühlung gerührt wird. Hierauf wird eine Aufschlämmung von 50 g 2-Amino-5-nitro-2'-fluorbenzophenon in 200 ml abs. Tetrahydrofuran schnell zugetropft und auschliessend bei Raumtemperatur 18 Stunden gerührt. Die erhaltene Lösung wird eingeengt; der Rückstand wird mit Eis und 10%-iger Natriumbicarbonatlösung versetzt und mit Methylenchlorid extrahiert. Die organische Lösung wird über Natriumsulfat getrocknet, filtriert und eingeengt. Der Rückstand wird in wenig Essigester heiss gelöst, etwas eingeengt und mit Aether versetzt. Man erhält Benzyl {[/2-(o-fluorbenzoyl)-4-nitrophenyl/carbamoyl]methyl}carbamat.

Die Mutterlaugen werden an einer 100 g-Kieselgelsäule mit Methylenchlorid als Eluierungsmittel gereinigt, wobei noch eine zusätzliche Menge des obigen Produktes gewonnen wird.

b) 4,5 g (0,01 M) Benzyl{[/2-(o-fluorbenzoyl)-4-nitrophenyl/carbamoyl]methyl}carbamat, gelöst in 50 ml abs. Aceton, werden mit 4,5 g Kaliumcarbonat (gemahlen) und 2 ml Methyljodid versetzt, worauf man das Gemisch 6 Tage bei Raumtemperatur rührt. Hierauf wird das unlösliche Material abfiltriert und mit Aceton gewaschen; die vereinigten Aceton-Lösungen werden eingeengt. Der Rückstand wird an einer 250 g-Kieselgelsäule mit Methylenchlorid-Essigester (10:1) und dann mit Methylenchlorid-Essigester (5:1) als Eluierungsmittel gereinigt und aus Aether-Petroläther kristallisiert. Man erhält Benzyl{[/2/(o-fluorbenzoyl)-4-nitrophenyl/methylcarbamoyl]methyl}carbamat, welches bei 112° schmilzt.

c) 10,5 g (0,0225 M) Benzyl{[/2 - (o - fluorbenzoyl) - 4 - nitrophenyl/methylcarbamoyl]methyl}carbamat, gelöst in 150 ml Eisessig, werden mit 23 g Zinn(II)chlorid und 30 ml einer Mischung von konz. Salsäure und Wasser (1:1) versetzt. Das Reaktionsgemisch wird 20 Stunden bei Raumtemperatur gerührt, auf Eis-Ammoniak gegossen und mehrmals mit Essigester extrahiert. Die organische Lösung wird über Natriumsulfat getrocknet, filtriert und eingeengt. Der Rückstand wird an einer 350 g-Kieselgelsäule mit Methylenchlorid-Essigester als Eluierungsmittel gereinigt und aus Methylenchlorid-Aether kristallisiert. Man erhalt Benzyl{[/4 - amino - 2 - (o - fluorbenzoyl) - phenyl/methylcarbamoyl]methyl}carbamat, welches bei 83° schmiltzt.

d) 0,8 g (0,0018 M) Benzyl{[/4 - amino - 2 - (o - fluorbenzoyl)phenyl/methylcarbamoyl]methyl}-carbamat, gelöst in 10 ml abs Tetrahydrofuran, werden mit 0,2 ml Chloräthylisocyanat versetzt, worauf das Gemisch 24 Stunden bei Raumtemperatur gerührt wird. Das Reaktionsgemisch wird nochmals mit Chloräthylisocyanat (0,1 ml) versetzt und 12 Stunden gerührt. Das Reaktionsgemisch wird danach eingeengt und der Rückstand an einer 70 g-Kieselgelsäule mit 5% Essigester in Methylenchlorid als Eluierungsmittel gereinigt. Das erhaltene Benzyl{[/2 - (o - fluorbenzoyl) - 4 - [3 - (2 - chloräthyl)-ureido]phenyl/methylcarbamoyl]methyl}carbamat wird ohne Isolierung weiterverarbeitet.

e) 0,7 g rohes Benzyl{[/2 - (o - fluorbenzoyl) - 4 - [3 - (2 - chloräthyl)ureido]phenyl/methylcarbamoyl]methyl}carbamat, gelöst in 15 ml Acetonitril, werden mit 400 mg Kaliumacetat und 700 mg 18-crown-6 versetzt worauf man 5 Tage bei Raumtemperatur rührt. Das Reaktionsgemisch engt man daraufhin ein, versetzt den Rückstand mit Wasser-Methylenchlorid und extrahiert die wässerige Lösung mehrmals mit Methylenchlorid. Die organische Lösung wird mit Natriumsulfat getrocknet, filtriert und eingeengt. Der Rückstand wird an einer 30 g-Kieselgelsäule mit Methylenchlorid-Essigester (3:1) als Eluierungsmittel gereinigt. Das erhaltene Benzyl{[/2 - (o - fluorbenzoyl) - 4 - [3 - (2 - acetoxyäthyl)ureido]phenyl/methylcarbamoyl]methyl}carbamat wird ohne Isolierung weiterverarbeitet.

f) 250 mg Benzyl{[/2 - (o - fluorbenzoyl) - 4 - [3 - (2 - acetoxyäthyl)ureido]phenyl/methylcarbamoyl]methyl}carbamat, gelöst in 10 ml abs. Methanol, werden mit 50 mg Natriummethylat versetzt und ein Stunde bei Raumtemperatur gerührt, worauf das Gemisch mit wenig Essigsäure gepuffert wird. Man giesst das Reaktionsgemisch auf Eis — 10% Natriumbicarbonat und extrahiert mit Methylenchlorid. Die organische Lösung wird über Natriumsulfat getrocknet, filtriert und eingeengt. Aus Aether-Aethanol kristallisiert man Benzyl{[/2 - (o - fluorbenzoyl) - 4 - [3 - (2 - hydroxyäthyl)ureido]phenyl/-methylcarbamoyl]methyl}carbamat, welches bei 117—120° schmilzt.

g) 200 mg (0,0004 M) Benzyl{[/2 - (o - fluorbenzoyl) - 4 - [3 - (2 - hydroxyäthyl)ureido]phenyl/-methylcarbamoyl]methyl}carbamat werden in 20 ml Aethanol gelöst, worauf man 20 mg 5%-iges

Palladium auf Aktivkohle zugibt und mit Wasserstoff bei Raumtemperatur und Normaldruck hydriert. Nach 5 Stunden wird der Katalysator abfiltriert, mit Methylenchlorid gewaschen und das Lösungsmittel eingeengt. Der Rückstand wird aus Aceton kristallisiert. Man erhält 1 - [5 - (o - Fluorphenyl)-2,3 - dihydro - 1 - methyl - 2 - oxo - 1H - 1,4 - benzodiazepin - 7 - yl] - 3 - (2 - hydroxyäthyl)harnstoff, welcher bei 156—160° unter Zersetzung schmilzt.

Beispiel 38

a) 1 g (0,0023 M) Benzyl{[/4-amino-2-(o-fluorbenzoyl)phenyl/methylcarbamoyl]methyl}-carbamat, gelöst in 100 ml 1,2-Dichloräthan, werden mit 320 mg gemahlenem Kaliumcarbonat und 0,25 ml Dimethylcarbamoylchlorid versetzt, worauf das Gemisch 60 Stunden am Rückfluss erhitzt, abgekühlt, mit Wasser versetzt und dreimal mit Methylenchlorid extrahiert wird. Die organische Lösung wird einmal mit Wasser gewaschen, mit Natriumsulfat getrocknet und eingeengt; der Rückstand wird an einer 200 g-Kieselgelsäule mit Essigester als Eluierungsmittel gereinigt und dann am Hochvakuum bis zum konstanten Gewicht eingeengt. Das so erhaltene ölige Benzyl{[/2-(o-fluorbenzoyl)-4-[3-(2-dimethyl)ureido]phenyl/methylcarbamoyl]methyl}carbamat wird ohne Isolierung weiterverarbeitet.

b) 0,8 g Benzyl{[/2-(o-fluorbenzoyl)-4-[3-(2-dimethyl)-ureido]phenyl/methylcarbamoyl]methyl}-carbamat werden in 5 ml 30—33%-iger Bromwasserstofflösung in Eisessig gelöst, worauf 15 Minuten bei Raumtemperatur gerührt, mit 10%-iger Natriumbicarbonatlösung versetzt und dreimal mit Methylenchlorid extrahiert wird. Die Extrakte werden über Magnesiumsulfat getrocknet, filtriert und eingeengt. Ein Teil des Rückstandes wird an einer Kieselgelpräparativplatte mit Essigester-Aceton als Eluierungsmittel gereinigt. Das so gewonnene Produkt erweist sich gemäss IR-Spektrum als identisch mit auf anderem Wege (Beispiel 32) hergestelltem 3-[5-(o-Fluorphenyl)-2,3-dihydro-1-methyl-2-oxo-1H-1,4-benzodiazepin-7-yl]-1,1-dimethyl-harnstoff.

Beispiel 39

a) 2 g (0,0057 M) N-[5-(o-fluorphenyl)-2,3-dihydro-1-methyl-2-oxo-1H-1,4-benzodiazepin-7-yl]-1-aziridin-carboxamid in 50 ml tert.-Butanol werden mit 3 ml 25%-iger Schwefelsäure versetzt. Das Gemisch wird nach 20 Minuten mit 10%-iger Natriumbicarbonatlösung alkalisch gestellt und mehrmals mit Methylenchlorid extrahiert. Die Methylenchloridlösung wird über Natriumsulfat getrocknet, filtriert und eingeengt, worauf der Rückstand an einer 80 g-Kieselgelsäule mit Essigester als Eluierungsmittel gereinigt und aus Essigester umkristallisiert wird. Man erhält 1-(2-tert.-Butoxyäthyl)-3-[5-(o-fluorphenyl)-2,3-dihydro-1-methyl-2-oxo-1H-1,4-benzodiazepin-7-yl]harnstoff, welcher bei 177° schmilzt.

b) 50 mg (0,12 mM) 1-(2-tert.-Butoxyäthyl)-3-[5-(o-fluorphenyl)-2,3-dihydro-1-methyl-2-oxo-1H-1,4-benzodiazepin-7-yl]harnstoff werden in 1 ml Trifluoressigsäure gelöst, worauf die Lösung 2 Stunden bei Raumtemperatur stehengelassen und dann eingeengt wird. Der Rückstand wird mit 10%-igem Natriumcarbonat aufgenommen; man extrahiert mehrmals mit Methylenchlorid und sehr wenig Methanol, und die organische Lösung wird über Natriumsulfat getrocknet, filtriert und eingeengt. Der Rückstand wird aus Aceton-Aether kristallisiert; das erhaltene Produkt ist gemäss IR-Spektrum identisch mit auf anderem Wege (vgl. Beispiel 7) hergestelltem 1-[5-(o-Fluorphenyl)-2,3-dihydro-1-methyl-2-oxo-1H-1,4-benzodiazepin-7-yl]-3-(2-hydroxyäthyl)harnstoff.

Beispiel 40

Eine Lösung von 1 g (0,0028 M) N-[5-(o-fluorphenyl)-2,3-dihydro-1-methyl-2-oxo-1H-1,4-benzodiazepin-7-yl]-1-aziridincarboxamid in 50 ml Dioxan wird mit einigen Tropfen 25%-iger Schwefelsäure angesäuert, nach 20 Minuten mit Ammoniak basisch gestellt und mit Methylenchlorid und sehr wenig Methanol mehrmals extrahiert. Die organische Lösung wird über Natriumsulfat getrocknet und filtriert; der Rückstand wird an einer 80 g-Keiselgelsäule mit Aceton als Eluierungsmittel gereinigt und aus Aceton-Aether kristallisiert. Gemäss IR-Spektrum, Mischschmelzpunkt und Schmelzpunkt ist das isolierte Produkt identisch mit auf anderem Wege (vgl. Beispiel 7) hergestelltem 1-[5-(o-Fluorphenyl)-2,3-dihydro-1-methyl-2-oxo-1H-1,4-benzodiazepin-7-yl]-3-(2-hydroxyäthyl)harnstoff.

Beispiel 41

a) 4,2 g (0,015 M) 7-Amino-5-(o-fluorphenyl)-1,3-dihydro-1-methyl-2H-1,4-benzodiazepin-2-on werden in 80 ml abs. Tetrahydrofuran mit 3,5 ml Chloräthylisocyanat und einer katalytischen Menge Triäthylamin während 3 Stunden am Rückfluss erhitzt. Das Reaktionsgemisch wird über Nacht bei Raumtemperatur stehengelassen. Die Lösung wird am Dampfbad eingeengt, worauf der Rückstand aus Essigester kristallisiert wird. Man erhält 1-(2-Chloräthyl)-3-[5-(o-fluorphenyl)-2,3-dihydro-1-methyl-2-oxo-1H-1,4-benzodiazepin-7-yl]harnstoff, welcher bei 240° unter Zersetzung schmilzt.

b) 760 mg (1,95 mM) 1-(2-Chloräthyl)-3-[5-(o-fluorphenyl)-2,3-dihydro-1-methyl-2-oxo-1H-1,4-benzodiazepin-7-yl]harnstoff, gelöst in 15 ml Acetonitril, werden mit 200 mg Kaliumacetat und 520 mg 18-Crown-6 versetzt. Das Reaktionsgemisch wird daraufhin 5 Tage bei Raumtemperatur gerührt, eingeengt und mit Wasser-Methylenchlorid versetzt, worauf die wässerige Lösung mehrmals mit Methylenchlorid extrahiert wird. Die organische Lösung wird mit Natriumsulfat getrocknet, filtriert und eingeengt. Der Rückstand wird an einer 70 g-Kieselgelsäule mit Essigester als Eluierungsmittel gereinigt und aus Essigester-Aether kristallisiert. Gemäss Schmelzpunkt und Mischschmelzpunkt ist

das isolierte Produkt identisch mit auf anderem Wege (vgl. Beispiel 36) hergestelltem 2-[3-/5-(o-Fluorphenyl)-2,3-dihydro-1-methyl-2-oxo-1H-1,4-benzodiazepin-7-yl/ureido]äthylacetat.

Beispiel 42

300 mg (0,73 mM) 2-[3-/5-(o-fluorphenyl)-2,3-dihydro-1-methyl-2-oxo-1H-1,4-benzodiazepin-7-yl/ureido]äthylacetat, gelöst in 20 ml abs. Methanol, werden mit 500 mg Natriummethyltat versetzt und 1 Stunde bei Raumtemperatur gerührt, worauf das Gemisch mit wenig Essigsäure gepuffert und eingeengt wird. Der Rückstand wird mit Methylenchlorid/10%-igem Natriumbicarbonat versetzt; die wässerige Lösung wird mit Methylenchlorid mehrmals extrahiert, und die organische Lösung wird über Natriumsulfat getrocknet, filtriert und eingeengt. Aus Aceton kristallisiert man 1-[5-(o-Fluorphenyl)-2,3-dihydro-1-methyl-2-oxo-1H-1,4-benzodiazepin-7-yl]-3-(2-hydroxyäthyl)harnstoff, welcher gemäss IR-Spektrum mit einer auf anderem Wege (vgl. Beispiel 7) hergestellten Probe dieser Substanz identisch ist.

Beispiel 43

a. 5 g (0,016 M) 7-Amino-6-chlor-5-(o-fluorphenyl)-1,3-dihydro-1-methyl-2H-1,4-benzodiazepin-2-on in 85 ml abs. Tetrahydrofuran werden mit 11,1 ml Chloräthylisocyanat und 1 ml Triäthylamin versetzt, worauf 1 Woche bei Raumtemperatur gerührt und 5 Stunden am Rückfluss erhitzt wird. Das Reaktionsgemisch wird mit 50 ml Aethanol versetzt und eingeengt; der Rückstand wird mit Methylenchlorid-Wasser behandelt und mit Methylenchlorid mehrmals extrahiert. Die organische Phase wird abgetrennt, mit Natriumsulfat getrocknet, filtriert und eingeengt. Der Rückstand wird an einer 300 g-Keiselgelsäule mit Methylenchlorid-Essigester (3:1) als Eluierungsmittel gereinigt; man erhält 1-(2-Chloräthyl)-3-[6-chlor-5-(o-fluorphenyl)-2,3-dihydro-1-methyl-2-oxo-1H-1,4-benzodiazepin-7-yl]-harnstoff, welcher direkt weiterverarbeitet wird.

b) 3,7 g 1-(2-Chloräthyl)-3-[6-chlor-5-(o-fluorphenyl)-2,3-dihydro-1-methyl-2-oxo-1H-1,4-benzodiazepin-7-yl]harnstoff, gelöst in 100 ml Acetonitril, werden mit 1,83 g Kaliumacetat und 4,21 g 18-Crown-6 versetzt. Das Reaktionsgemisch wird daraufhin 5 Tage bei Raumtemperatur gerührt, eingeengt und mit Wasser-Methylenchlorid versetzt, und die wässerige Lösung wird mehrmals Methylenchlorid extrahiert. Die organische Lösung wird mit Natriumsulfat getrocknet, filtriert und eingeengt. Der Rückstand wird an einer 350 g-Kieselgelsäule mit Essigester als Eluierungsmittel gereinigt und aus Aethanol-Aether kristallisiert. Man erhält rohes 2-[3-/6-Chlor-5-(o-fluorphenyl)-2,3-dihydro-1-methyl-2-oxo-1H-1,4-benzodiazepin-7-yl/ureido]äthylacetat.

Beispiel 44

600 mg 2-[3-/6-Chlor-5-(o-fluorphenyl)-2,3-dihydro-1-methyl-2-oxo-1H-1,4-benzodiazepin-7-yl/ureido]äthylacetat, gelöst in 35 ml abs. Methanol, werden mit 830 mg Natriummethylat versetzt und 1 Stunde bei Raumtemperatur gerührt, worauf das Gemisch mit wenig Essigsäure gepuffert und eingeengt wird. Der Rückstand wird mit Methylenchlorid-10%-igem Natriumbicarbonat versetzt. Die wässerige Lösung wird mit Methylenchlorid extrahiert; die organische Lösung wird über Natriumsulfat getrocknet, filtriert und eingeengt. Der Rückstand wird an einer 30 g-Kieselgelsäule mit Essigester-Methanol (10:1) als Eluierungsmittel gereinigt und aus Aethanol-Aether kristallisiert. Man erhält 1-[6-Chlor-5-(o-fluorphenyl)-2,3-dihydro-1-methyl-2-oxo-1H-1,4-benzodiazepin-7-yl]-3-(2-hydroxyäthyl)-harnstoff, welcher sich gemäss Schmelzpunkt, Mischschmelzpunkt und IR-Spektrum als mit einer auf anderem Wege (vgl. Beispiel 13) hergestellter Probe identisch erweist.

Beispiel 45

100 mg (0,26 mMol) 1-(2-Chloräthyl)-3-[5-(o-fluorphenyl)-2,3-dihydro-1-methyl-2-oxo-1H-1,4-benzodiazepin-7-yl]harnstoff werden in 6 ml Benzol und 4 ml Wasser mit 150 mg Tetrabutylammoniumbromid 16 Stunden bei 80° gerührt. Dann wird das Gemisch eingeengt, und der Rückstand wird in einer Drucksäule (0,2—0,4 atm. $N_2$) an 15 g Kieselgel (Korngrösse 0,04—0,063 mm) mit Essigester und Essigester + 3% Methanol als Eluierungsmittel chromatographiert, wobei ein 1 - [5 - (o - Fluorphenyl) - 2,3 - dihydro - 1 - methyl - 2 - oxo - 1H - 1,4 - benzodiazepin - 7 - yl] - 3 - (2 - hydroxyäthyl)harnstoff und polare Verunreinigung enthaltendes Rohprodukt erhalten wird.

Dieses Rohprodukt (60 mg) wird in Methylenchlorid-Wasser aufgenommen, worauf extrahiert und die Methylenchlorid-Lösung über Natriumsulfat getrocknet, filtriert und eingeengt wird.

Der Rückstand wird in einer Drucksäule (0,2—0,4 atm. $N_2$) an 15 g Kieselgel (Korngrösse 0,04—0,063 mm) mit abs. Aceton als Eluierungsmittel chromatographiert, worauf man aus Aceton-Aether kristallisiert; das so gewonnene Produkt ist gemäss JR-Spektrum identisch mit einer auf anderem Wege (vgl. Beispiel 7) hergestellten Probe von 1 - [5 - (o - Fluorphenyl) - 2,3 - dihydro - 1 - methyl - 2 - oxo - 1H - 1,4 - benzodiazepin - 7 - yl] - 3 - (2 - hydroxyäthyl)harnstoff.

Beispiel 46

a) Eine Dichloräthanlösung von [5-(o-Fluorphenyl)-2,3-dihydro-1-methyl-2-oxo-1H-1,4-benzodiazepin-7-yl]-isocyanat, erhalten gemäss Angaben in Absatz a) von Beispiel 1 aus 10,4 g (0,037 M) 7-Amino-5-(o-fluorphenyl)-1,3-dihydro-1-methyl-2H-1,4-benzodiazepin-2-on, wird mit 9,1 g (0,044 M)

N-Benzyl-O-tert.butyl-äthanolamin in 90 ml Dichloräthan versetzt. Die organische Phase wird zweimal mit Wasser gewaschen, über Natriumsulfat getrocknet, filtriert und eingeengt. Der Rückstand wird an einer 600 g-Keiselgelkolonne gereinigt (Laufmittel, Essigester) und aus Methylenchlorid-n-Hexan kristallisiert. Man erhält 1 - Benzyl - 1 - (2 - tert. - butoxyäthyl) - 3 - [5 - (o - fluorphenyl) - 2,3 - dihydro - 1 - methyl - 2 - oxo - 1H - 1,4 - benzodiazepin - 7 - yl]harnstoff, welcher bei 82—86° schmiltzt.

b) 200 mg (0,4 mMol) 1-Benzyl-1-(2-tert.butoxyäthyl)-3-[5-(o-fluorphenyl)-2,3-dihydro-1-methyl-2-oxo-1H-1,4-benzodiazepin-7-yl]harnstoff werden in 3 ml einer 33%igen Lösung von Bromwasserstoff in Eisessig auf dem Dampfbad 10 Minuten erhitzt and dann während drei Tagen bei Raumtemperatur stehengelassen. Das Reaktionsgemisch wird mit Natriumbicarbonat neutralisiert und mit Methylenchlorid mehrmals extrahiert; die Extrakte werden vereinigt, über Natriumsulfat getrocknet, filtriert und eingeengt. Der Rückstand wird in einer Drucksäule (0,2—0,4 atm. $N_2$) an 15 g Kieselgel (Korngrösse 0,04—0,06 mm) mit Essigester als Eluierungsmittel gereinigt und aus Essigester-Aether kristallisiert; man erhält 2 - [3 - {5 - (o - Fluorphenyl) - 2,3 - dihydro - 1 - methyl - 2 - oxo - 1H - 1,4 - benzodiazepin - 7 - yl]}ureido]äthylacetat, welches bei 196—198° schmiltzt.

c) 20 mg (0,048 mMol) 2-[3-{5-(o-Fluorphenyl)-2,3-dihydro-1-methyl-2-oxo-1H-1,4-benzodiazepin-7-yl}ureido]äthylacetat werden in 2 ml abs. Methanol gelöst und mit 13 mg Natriummethylat 1 Stunde bei 30—40° gerührt. Daraufhin wird das Reaktionsgemisch mit einigen Tropfen Eisessig gepuffert, mit Methylenchlorid verdünnt, mit 10%igem Natriumbicarbonat versetzt und mehrmals mit Methylenchlorid extrahiert, worauf die organischen Phasen vereinigt, über Natriumsulfat getrocknet, filtriert und eingeengt werden. Der Rückstand wird aus Aceton-Aether kristallisiert; man erhält 1 - [5 - (o - Fluorphenyl) - 2,3 - dihydro - 1 - methyl - 2 - oxo - 1H - 1,4 - benzodiazepin - 7 - yl] - 3 - (2 - hydroxyäthyl)harnstoff, welcher gemäss IR-Spektrum mit einer auf anderem Wege (vgl. Beispiel 7) hergestellten Probe dieser Substanz identisch ist.

Beispiel 47

1,5 g (0,003 M) 1-Benzyl-1-(2-tert.butoxyäthyl)-3-[5-(o-fluorphenyl)-2,3-dihydro-1-methyl-2-oxo-1H-1,4-benzodiazepin-7-yl]harnstoff werden in 15 ml 48%iger wässeriger Bromwasserstofflösung gelöst, worauf man 10 Minuten am Dampfbad erhitzt, mit Natriumbicarbonat alkalisch stellt, wenig Methanol zugibt und dreimal mit Methylenchlorid extrahiert. Die Extrakte werden vereinigt, über Natriumsulfat getrocknet, filtriert und eingeengt. Der Rückstand wird in einer Drucksäule (0,2—0,4 atm. $N_2$) an 300 g Kieselgel (Korngrösse 0,04—0,06 mm) mit Aceton als Eluierungsmittel gereinigt und aus Aceton-Aether kristallisiert; man erhält 1-[5-(o-Fluorphenyl)-2,3-dihydro-1-methyl-2-oxo-1H-1,4-benzodiazepin-7-yl]-3-(2-hydroxyäthyl)harnstoff, welcher gemäss IR-Spektrum mit einer auf anderem Wege (vgl. Beispiel 7) hergestellten Probe dieser Substanz identisch ist.

Beispiel 48

a) Aus 40 g (0,116 M) rac. 5-(o-Chlorphenyl)-1,3-dihydro-1,3-dimethyl-7-nitro-2H-1,4-benzodiazepin-2-on wird in Analogie zu den Angaben in Absatz d) von Beispiel 31 rac. 7-Amino-5-(o-chlorphenyl)-1,3-dihydro-1,3-dimethyl-2H-1,4-benzodiazepin-2-on erhalten, welches aus Essigester/Petroläther kristallisiert wird und bei 180—190° schmilzt.

b) Aus 4,7 g (0,015 M) rac. 7-Amino-5-(o-chlorphenyl)-1,3-dihydro-1,3-dimethyl-2H-1,4-benzodiazepin-2-on wird in Analogie zu den Angaben in Absatz e) von Beispiel 31 rac. 7-Amino-6-brom-5-(o-chlorphenyl)-1,3-dihydro-1,3-dimethyl-2H-1,4-benzodiazepin-2-on erhalten, welches aus Essigester/Aether kristallisiert wird und bei 254—256° unter Zersetzung schmilzt.

c) Aus 3 g (0,0076 M) rac. 7-Amino-6-brom-5-(o-chlorphenyl)-1,3-dihydro-1,3-dimethyl-2H-1,4-benzodiazepin-2-on wird in Analogie zu den Angaben in Absatz f) von Beispiel 31 rac. 1-[6-Brom-5-(o-chlorphenyl)-2,3-dihydro-1,3-dimethyl-2-oxo-2H-1,4-benzodiazepin-7-yl]-3-(2-hydroxyäthyl)harnstoff erhalten, welcher aus Aceton/Aether kristallisiert wird und bei 204—206° unter Zersetzung schmilzt.

Beispiel 49

a) Aus 15 g (0,045 M) rac. 7-Amino-5-(o-chlorphenyl)-1,3-dihydro-1,3-dimethyl-2H-1,4-benzodiazepin-2-on wird in Analogie zu den Angaben in Absatz a) von Beispiel 29 rac-7-Amino-6-chlor-5-(o-chlorphenyl)-1,3-dihydro-1,3-dimethyl-2H-1,4-benzodiazepin-2-on erhalten, welches aus Essigester kristallisiert wird und bei 240—242° unter Zersetzung schmilzt.

b) Aus 7 g (0,02 M) rac. 7-Amino-6-chlor-5-(o-chlorphenyl)-1,3-dihydro-1,3-dimethyl-2H-1,4-benzodiazepin-2-on wird in Analogie zu den Angaben in Absatz b) von Beispiel 29 rac - 1 - [6 - chlor - 5 - (o - chlorphenyl) - 2,3 - dihydro - 1,3 - dimethyl - 2 - oxo - 1H - 1,4 - benzodiazepin - 7 - yl] - 3 - (2 - hydroxyäthyl)harnstoff erhalten, welcher aus Aceton kristallisiert wird und bei 233—236° unter Zersetzung schmilzt.

Beispiel 50

a) Aus 15 g (0,05 M) 7 - Amino - 5 - (o - fluorphenyl) - 1,3 - dihydro - 1,3 - dimethyl - 2H - 1,4 - benzodiazepin - 2 - on wird in Analogie zu den Angaben in Absatz e) von Beispiel 31 rac. 7 - Amino - 6 - brom - 5 - (o - fluorphenyl) - 1,3 - dihydro - 1,3 - dimethyl - 2H - 1,4 - benzodiazepin - 2 - on erhalten, welches aus Essigester/Aether kristallisiert wird und bei 255° unter Zersetzung schmilzt.

b) Aus 4,4 g (0,012 M) rac. 7 - Amino - 6 - brom - 5 - (o - fluorphenyl) - 1,3 - dihydro - 1,3 - dimethyl - 2H - 1,4 - benzodiazepin - 2 - on wird in Analogie zu den Angaben in Absatz f) von Beispiel 31 rac. 1 - [6 - Brom - 5 - (o - fluorphenyl) - 2,3 - dihydro - 1,3 - dimethyl - 2 - oxo - 1H - 1,4 - benzodiazepin - 7 - yl] - 3 - (2 - hydroxyäthyl)harnstoff erhalten, welcher aus Aceton/Aether kristallisiert wird und bei 210—212° schmilzt.

Beispiel 51

Aus 6 g (0,019 M) 7 - Amino - 6 - chlor - 5 - (o - fluorphenyl) - 1,3 - dihydro - 1 - methyl - 2H - 1,4 - benzodiazepin - 2 - on wird in Analogie zu den Angaben in Beispiel 17, jedoch mit Morpholin anstatt mit N-Methylpiperazin, N - [6 - Chlor - 5 - (o - fluorphenyl) - 2,3 - dihydro - 1 - methyl - 2 - oxo - 1H - 1,4 - benzodiazepin - 7 - yl] - 4 - morpholincarboxamid erhalten, welches aus Essigester/Aether umkristallisiert wird und bei 126° schmilzt.

Beispiel A

1 - [5 - (o - Fluorphenyl) - 2,3 - dihydro - 1 - methyl - 2 - oxo - 1H - 1,4 - benzodiazepin - 7 - yl] - 3 - (2 - hydroxyäthyl)harnstoff kann wie folgt als Wirkstoff zur Herstellung von pharmazeutischen Präparaten verwendet werden:

a)

| Tabletten | 1 Tablette enthält |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Der Wirkstoff wird mit der Hälfte der mikrokristallinen Cellulose gemischt und mit einer 10%igen Lösung von Hydroxypropylmethylcellulose in einem Gemisch von Isopropanol und Methylenchlorid granuliert. Das Granulat wird getrocknet, gesiebt und mit dem Rest der Hilfsstoffe gemischt. Alsdann wird es auf einer Presse zu biplanen Tabletten von 12 mm Durchmesser mit Kreuzbruchrille verpresst.

b)

| Kapseln | 1 Kapsel enthält |
|---|---|
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

Der Wirkstoff wird mit den Hilfsstoffen gemischt und gesiebt. Nach erneutem Mischen wird die erhaltene Kapselfüllmasse auf einer vollautomatischen Kapselabfüllmaschine in Gelatinesteckkapseln geeigneter Grösse abgefüllt.

**Patentansprüche für die Vertragsstaaten: BE CH DE FR GB IT LU NL SE**

1. Benzodiazepin-Derivate der allgemeinen Formel (I)

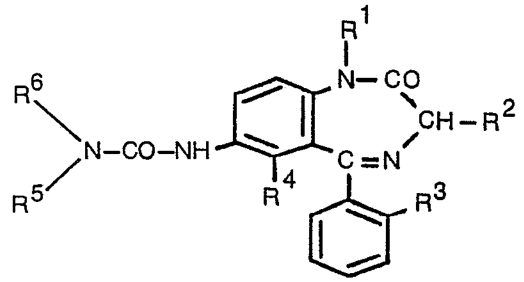

I

worin $R^1$ ($C_1$—$C_7$)-Alkyl, $R^2$ Wasserstoff oder ($C_1$—$C_7$)-Alkyl, $R^3$ Halogen und $R^4$ Wasserstoff oder Halogen bedeuten und entweder $R^5$ Wasserstoff oder ($C_1$—$C_7$)-Alkyl und $R^6$ ($C_1$—$C_7$)-Alkyl, Hydroxy-($C_2$—$C_7$)-alkyl oder ($C_1$—$C_7$)-Alknoyloxy-($C_2$—$C_7$)-alkyl bedeuten oder $R^5$ Wasserstoff und $R^6$ einen gegebenenfalls durch Halogen oder ($C_1$—$C_7$)-Alkoxy monosubstituierten Phenyl- oder Phenyl-($C_1$—$C_7$)-alkylrest bedeuten oder $R^5$ und $R^6$ zusammen mit dem Stickstoffatom einen 3- bis 7-gliedrigen Heterocyclus ohne zusätzliches Heteroatom oder einen N-Methylpiperazin-, Thiazolidin-, Morpholin- oder Thiomorpholinrest bedeuten, und pharmazeutisch akzeptable Säureadditionssalze von Benzodiazepin-Derivaten der Formel I.

2. Verbindungen gemäss Anspruch 1, dadurch gekennzeichnet, dass $R^2$ Wasserstoff, $R^3$ Fluor und $R^4$ Wasserstoff oder Chlor bedeuten.

3. Verbindungen gemäss Anspruch 1, dadurch gekennzeichnet, dass $R^4$ und $R^5$ Wasserstoff und $R^6$ ($C_2$—$C_7$)-Alkyl oder Hydroxy-($C_2$—$C_7$)-alkyl bedeuten.

4. Verbindungen gemäss Anspruch 1, dadurch gekennzeichnet, dass $R^4$ Halogen bedeutet und entweder $R^5$ Wasserstoff und $R^6$ ($C_2$—$C_7$)-Alkyl oder Hydroxy-($C_2$—$C_7$)-alkyl bedeuten oder $R^5$ und $R^6$ je ($C_1$—$C_7$)-Alkyl bedeuten oder $R^5$ und $R^6$ zusammen mit dem Stickstoffatom einen 3-bis 7-gliedriegen Heterocyclus ohne zusätzliches Heteroatom oder einen N-Methylpiperazin-, Thiazolidin-, Morpholin- oder Thiomorpholinrest bedeuten.

5. Verbindungen gemäss Anspruch 4, dadurch gekennzeichnet, dass $R^4$ Brom oder Chlor bedeutet.

6. Verbindungen gemäss einem der Ansprüche 3 bis 5, dadurch gekennzeichnet, dass $R^3$ Fluor oder Chlor bedeutet.

7. 1 - [5 - (o - Fluorphenyl) - 2,3 - dihydro - 1 - methyl - 2 - oxo - 1H - 1,4 - benzodiazepin - 7 - yl] - 3 - (2 - hydroxyäthyl)harnstoff.

8. 1 - [5 - (o - Fluorphenyl) - 2,3 - dihydro - 1 - methyl - 2 - oxo - 1H - 1,4 - benzodiazepin - 7 - yl] - 3 - methylharnstoff.

9. 1 - [5 - (o - Fluorphenyl) - 2,3 - dihydro - 1 - methyl - 2 - oxo - 1H - 1,4 - benzodiazepin - 7 - yl] - 3 - (2 - hydroxy - 1 - methyläthyl)harnstoff.

10. N - [5 - (o - Fluorphenyl) - 2,3 - dihydro - 1 - methyl - 2 - oxo - 1H - 1,4 - benzodiazepin - 7 - yl] - 1 - pyrrolidincarboxamid.

11. 1 - [6 - Chlor - 5 - (o - fluorphenyl) - 2,3 - dihydro - 1 - methyl - 2 - oxo - 1H - 1,4 - benzodiazepin - 7 - yl] - 3 - methylharnstoff.

12. 1 - n - Butyl - 3 - [6 - chlor - 5 - (o - fluorphenyl) - 2,3 - dihydro - 1 - methyl - 2 - oxo - 1H - 1,4 - benzodiazepin - 7 - yl]harnstoff.

13. 3 - [6 - Chlor - 5 - (o - fluorphenyl) - 2,3 - dihydro - 1 - methyl - 2 - oxo - 1H - 1,4 - benzodiazepin - 7 - yl] - 1 - äthyl - 1 - methylharnstoff.

14. 1 - [6 - Brom - 5 - (o - chlorphenyl) - 2,3 - dihydro - 1,3 - dimethyl - 2 - oxo - 1H - 1,4 - benzodiazepin - 7 - yl] - 3 - (2 - hydroxyäthyl)harnstoff.

15. N - [6 - Chlor - 5 - (o - fluorphenyl) - 2,3 - dihydro - 1 - methyl - 2 - oxo - 1H - 1,4 - benzodiazepin - 7 - yl] - 4 - morpholincarboxamid.

16. 1 - [6 - Brom - 5 - (o - fluorphenyl) - 2,3 - dihydro - 1,3 - dimethyl - 2 - oxo - 1H - 1,4 - benzodiazepin - 7 - yl] - 3 - (2 - hydroxyäthyl)harnstoff.

17. 1 - [6 - Chlor - 5 - (o - chlorphenyl) - 2,3 - dihydro - 1,3 - dimethyl - 2 - oxo - 1H - 1,4 - benzodiazepin - 7 - yl] - 3 - (2 - hydroxyäthyl)harnstoff.

18. 1 - tert. - Butyl - 3 - [5 - (o - fluorphenyl) - 2,3 - dihydro - 1 - methyl - 2 - oxo - 1H - 1,4 - benzodiazepin - 7 - yl]harnstoff,

1 - [5 - (o - Fluorphenyl) - 2,3 - dihydro - 1 - methyl - 2 - oxo - 1H - 1,4 - benzodiazepin - 7 - yl] - 3 - (p - methoxyphenyl)harnstoff,

1 - Benzyl - 3 - [5 - (o - fluorphenyl) - 2,3 - dihydro - 1 - methyl - 2 - oxo - 1H - 1,4 - benzodiazepin - 7 - yl]harnstoff,

3 - [5 - (o - Fluorphenyl) - 2,3 - dihydro - 1 - methyl - 2 - oxo - 1H - 1,4 - benzodiazepin - 7 - yl] - 1,1 - dimethylharnstoff.

1 - Aethyl - 3 - [5 - (o - fluorphenyl) - 2,3 - dihydro - 1 - methyl - 2 - oxo - 1H - 1,4 - benzodiazepin - 7 - yl] - 1 - methylharnstoff und

N - [5 - (o - Fluorphenyl) - 2,3 - dihydro - 1 - methyl - 2 - oxo - 1H - 1,4 - benzodiazepin - 7 - yl] - 1 - aziridincarboxamid.

19. 1 - [6 - Chlor - 5 - (o - fluorphenyl) - 2,3 - dihydro - 1 - methyl - 2 - oxo - 1H - 1,4 - benzodiazepin - 7 - yl] - 3 - äthylharnstoff,

1 - [6 - Chlor - 5 - (o - fluorphenyl) - 2,3 - dihydro - 1 - methyl - 2 - oxo - 1H - 1,4 - benzodiazepin - 7 - yl] - 3 - (2 - hydroxyäthyl)harnstoff und

3 - [6 - Chlor - 5 - (o - fluorphenyl) - 2,3 - dihydro - 1 - methyl - 2 - oxo - 1H - 1,4 - benzodiazepin - 7 - yl] - 1,1 - diäthylharnstoff.

20. Verbindungen gemäss einem der Ansprüche 1 bis 19 zur Anwendung als pharmazeutische Wirkstoffe.

21. Verbindungen gemäss einem der Ansprüche 1, 2, 7 bis 13, 18 und 19 zur Anwendung als aldosteronantagonistische Wirkstoffe.

22. Verbindungen gemäss einem der Ansprüche 3 bis 6 und 14 bis 17 zur Anwendung als die intestinale Resorption von Cholesterin hemmende Wirkstoffe.

23. Verfahren zur Herstellung von Benzodiazepin-Derivaten gemäß Anspruch 1, dadurch gekennzeichnet, das man

a) ein in situ hergestelltes Benzodiazepin-Derivat der allgemeinen Formel (II)

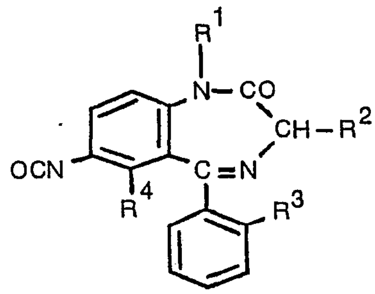

II

worin $R^1$, $R^2$, $R^3$ und $R^4$ die in Anspruch 1 angegebene Bedeutung besitzen, mit einer Aminoverbindung der allgemeinen Formel (III)

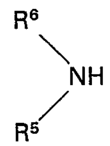

III

worin $R^5$ und $R^6$ die in Anspruch 1 angegebene Bedeutung besitzen, umsetzt, oder

b) eine in situ hergestellte Verbindung der allgemeinen Formel (IV)

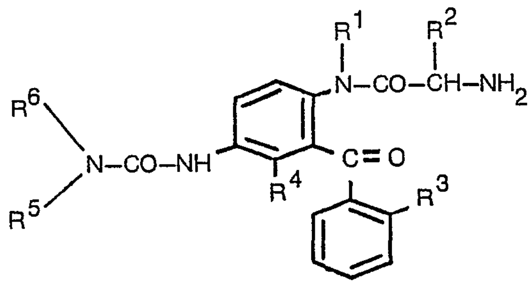

IV

worin $R^1$, $R^2$, $R^3$, $R^4$, $R^5$ und $R^6$ die in Anspruch 1 angegebene Bedeutung besitzen, in Lösung cyclisiert, oder

c) ein Benzodiazepin-Derivat der allgemeinen Formel (V)

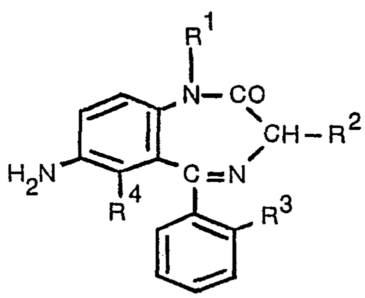

V

worin $R^1$, $R^2$, $R^3$ und $R^4$ die in Anspruch 1 angegebene Bedeutung besitzen, mit einem Halogenid der allgemeinen Formel (VI)

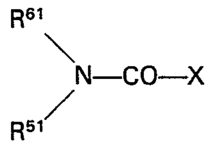

VI

worin X Halogen bedeutet und entweder $R^{51}$ ($C_1$—$C_7$)-Alkyl und $R^{61}$ ($C_1$—$C_7$)-Alkyl oder ($C_1$—$C_7$)-Alkanoyloxy-($C_2$—$C_7$)-alkyl bedeuten oder $R^{51}$ und $R^{61}$ zusammen mit dem Stickstoffatom einen 3-bis 7-gliedriegen Heterocyclus ohne zusätzliches Heteroatom oder einen N-Methylpiperazin-, Thiazolidin-, Morpholin- oder Thiomorpholinrest bedeuten, umsetzt, oder

d) ein Benzodiazepin-Derivat der obigen allgemeinen Formel (V) mit einem Isocyanat der allgemeinen Formel (VIII)

$$R^{62}—NCO \qquad VII$$

worin $R^{62}$ ($C_1$—$C_7$)-Alkyl, ($C_1$—$C_7$)-Alkanoyloxy-($C_2$—$C_7$)-alkyl oder einen gegebenenfalls durch Halogen oder ($C_1$—$C_7$)-Alkoxy monosubstituierten Phenyl- oder Phenyl-($C_1$—$C_7$)-alkylrest bedeutet, umsetzt, oder

e) aus einem Benzodiazepin-Derivat der allgemeinen Formel (VIII)

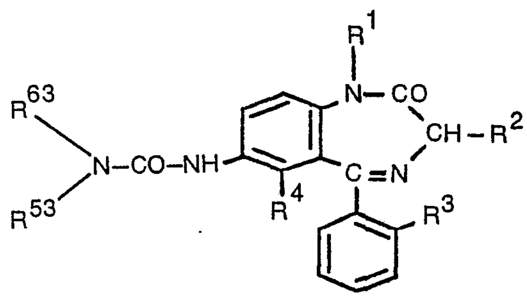

VIII

worin $R^1$, $R^2$, $R^3$ und $R^4$ die in Anspruch 1 angegebene Bedeutung besitzen und entweder $R^{53}$ eine Schutzgruppe und $R^{63}$ ($C_1$—$C_7$)-Alkyl, einen gegebenenfalls durch Halogen oder ($C_1$—$C_7$)-Alkoxy monosubstituierten Phenyl- oder Phenyl($C_1$—$C_7$)-alkylrest oder einen Rest der Formel (IX)

$$—A—O—Y \qquad IX$$

bedeuten, wobei A ($C_2$—$C_7$)-Alkylen und Y eine Schutzgruppe bedeuten, oder $R^{53}$ Wasserstoff oder ($C_1$—$C_7$)-Alkyl und $R^{63}$ einen Rest der obigen Formel (IX) bedeuten, die Schutzgruppe(n) entfernt, oder

f) ein Benzodiazepin-Derivat der allgemeinen Formel X

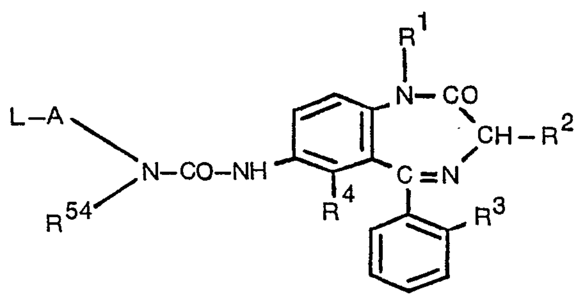

X

worin $R^1$, $R^2$, $R^3$ und $R^4$ die in Anspruch 1 angegebene Bedeutung und A obige Bedeutung besitzen und $R^{54}$ Wasserstoff oder ($C_1$—$C_7$)-Alkyl und L ein Halogenatom, eine Arylsulfonyloxy-, Alkylsulfonyloxy- oder eine quartäre Ammoniumgruppe bedeuten, in die entsprechende Hydroxy- oder ($C_1$—$C_7$)-Alkanoyloxy-verbindung überführt, oder

g) ein Benzodiazepin-Derivat der allgemeinen Formel (Ia)

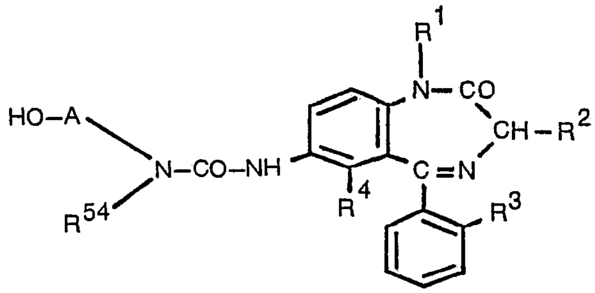

Ia

worin $R^1$, $R^2$, $R^3$ und $R^4$ die in Anspruch 1 angegebene Bedeutung und $R^{54}$ und A obige Bedeutung besitzen, ($C_1$—$C_7$)-alkanoyliert, oder

h) in einem Benzodiazepin-Derivat der allgemeinen Formel (Ib)

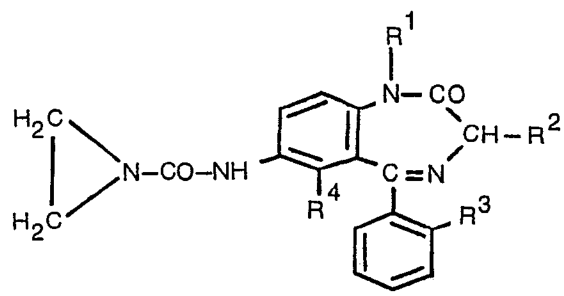

Ib

worin $R^1$, $R^2$ $R^3$ und $R^4$ die in Anspruch 1 angegebene Bedeutung besitzen, den Aziridinring hydrolytisch öffnet, oder

i) ein Benzodiazepin-Derivat der allgemeinen Formel (I) in ein pharmazeutisch akzeptables Säureadditionssalz überführt.

24. Verfahren nach Anspruch 23, dadurch gekennzeichnet, dass man nach den Varianten a) bis g) oder i) arbeitet.

25. Arzneimittel, enthaltend eine Verbindung gemäss einem der Ansprüche 1 bis 19.

26. Aldosteronantagonistisches Mittel, enthaltend eine Verbindung gemäss einem der Ansprüche 1, 2, 7 bis 13, 18 und 19.

27. Die intestinale Resorption von Cholesterin hemmendes Mittel, enthaltend eine Verbindung gemäss einem der Ansprüche 3 bis 6 und 14 bis 17.

**Patentansprüche für den Vertragsstaat: AT**

1. Verfahren zur Herstellung von Benzodiazepin-Derivaten der allgemeinen Formel (I)

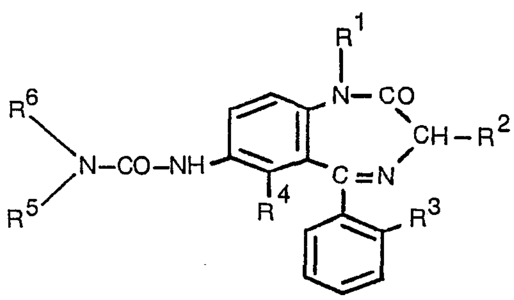

I

worin $R^1$ ($C_1$—$C_7$)-Alkyl, $R^2$ Wasserstoff oder ($C_1$—$C_7$)-Alkyl, $R^3$ Halogen und $R^4$ Wasserstoff oder Halogen bedeuten und entweder $R^5$ Wasserstoff oder ($C_1$—$C_7$)-Alkyl und $R^6$ ($C_1$—$C_7$)-Alkyl, Hydroxy-($C_2$—$C_7$)-alkyl oder ($C_1$—$C_7$)-Alkanoyloxy-($C_2$—$C_7$)-alkyl bedeuten oder $R^5$ Wasserstoff und $R^6$ einen gegebenenfalls durch Halogen oder ($C_1$—$C_7$)-Alkoxy monosubstituierten Phenyl- oder Phenyl-($C_1$—$C_7$)-alkylrest bedeuten oder $R^5$ und $R^6$ zusammen mit dem Stickstoffatom einen 3- bis 7-gliedrigen Heterocyclus ohne zusätzliches Heteroatom oder einen N-Methylpiperazin-, Thiazolidin-, Morpholin- oder Thiomorpholinrest bedeuten und von pharmazeutisch akzeptablen Säureadditionssalzen von Benzodiazepin-Derivaten der Formel I, dadurch gekennzeichnet, dass man

a) ein in situ hergestelltes Benzodiazepin-Derivat der allgemeinen Formel (I)

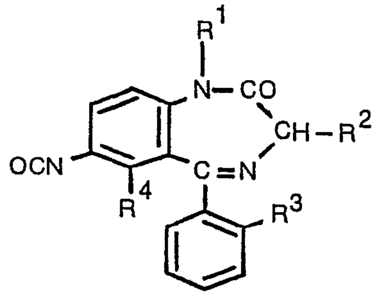

II

worin $R^1$, $R^2$, $R^3$ und $R^4$ obige Bedeutung besitzen, mit einer Aminoverbindung der allgemeinen Formel (III)

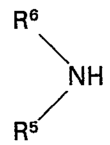

III

worin $R^5$ und $R^6$ obige Bedeutung besitzen, umsetzt, oder
b) eine in situ hergestellte Verbindung der allgemeinen Formel (IV)

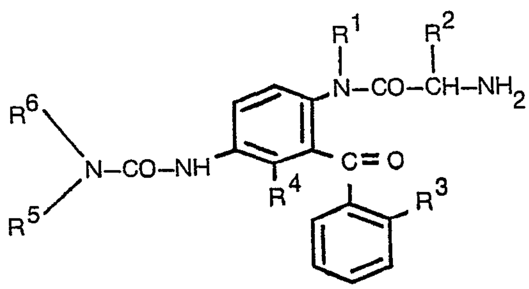

IV

worin $R^1$, $R^2$, $R^3$, $R^4$, $R^5$ und $R^6$ obige Bedeutung besitzen, in Lösung cyclisiert, oder
c) ein Benzodiazepin-Derivat der allgemeinen Formel (V)

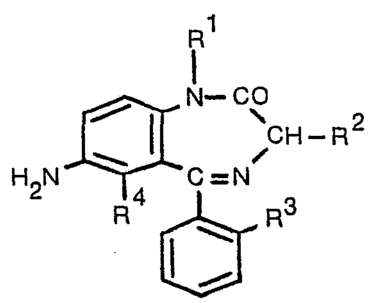

V

worin $R^1$, $R^2$, $R^3$ und $R^4$ obige Bedeutung besitzen, mit einem Halogenid der allgemeinen Formel (VI)

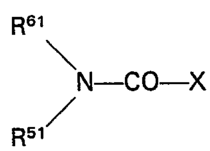

VI

worin X Halogen bedeutet und entweder $R^{51}$ ($C_1$—$C_7$)-Alkyl und $R^{61}$ ($C_1$—$C_7$)-Alkyl oder ($C_1$—$C_7$)-Alkanoyloxy-($C_2$—$C_7$)-alkyl bedeuten oder $R^{51}$ und $R^{61}$ zusammen mit dem Stickstoffatom einen 3- bis 7-gliedrigen Heterocyclus ohne zusätzliches Heteroatom oder einen N-Methylpiperazin-, Thiazolidin-, Morpholin- oder Thiomorpholinrest bedeuten, umsetzt, oder
d) ein Benzodiazepin-Derivat der obigen allgemeinen Formel (V) mit einem Isocyanat der allgemeinen Formel (VII)

$R^{62}$—NCO VII

worin $R^{62}$ ($C_1$—$C_7$)-Alkyl, ($C_1$—$C_7$)-Alkanoyloxy-($C_2$—$C_7$)-alkyl oder einen gegebenenfalls durch

Halogen oder ($C_1$—$C_7$)-Alkoxy monosubstituierten Phenyl- oder Phenyl-($C_1$—$C_7$)-alkylrest bedeutet, umsetzt, oder

e) aus einem Benzodiazepin-Derivat der allgemeinen Formel VIII

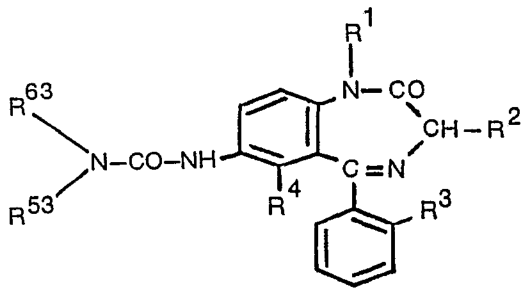

VIII

worin $R^1$, $R^2$, $R^3$ und $R^4$ obige Bedeutung besitzen und entweder $R^{53}$ eine Schutzgruppe und $R^{63}$ ($C_1$—$C_7$)-Alkyl einen gegebenenfalls durch Halogen oder ($C_1$—$C_7$)-Alkoxy monosubstituierten Phenyl- oder Phenyl-($C_1$—$C_7$)-alkylrest oder einen Rest der Formel (IX)

$$—A—O—Y \qquad IX$$

bedeuten, wobei A ($C_1$—$C_7$)-Alkylen und Y eine Schutzgruppe bedeuten, oder $R^{53}$ Wasserstoff oder ($C_1$—$C_7$)-Alkyl und $R^{63}$ einen Rest der obigen Formel IX bedeuten, die Schutzgruppe(n) entfernt, oder

f) ein Benzodiazepin-Derivat der allgemeinen Formel X

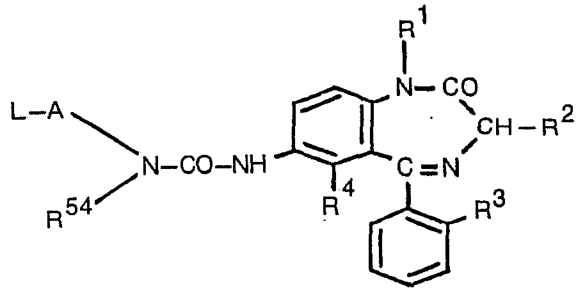

X

worin $R^1$, $R^2$, $R^3$, $R^4$ und A obige Bedeutung besitzen und $R^{54}$ Wasserstoff oder ($C_1$—$C_7$)-Alkyl und L ein Halogenatom, eine Arylsulfonyloxy-, Alkylsulfonyloxy- oder eine quartäre Ammoniumgruppe bedeuten, in die entsprechende Hydroxy- oder ($C_1$—$C_7$)Alkanoyloxy verbindung überführt, oder

g) ein Benzodiazepin-Derivat der allgemeinen Formel (Ia)

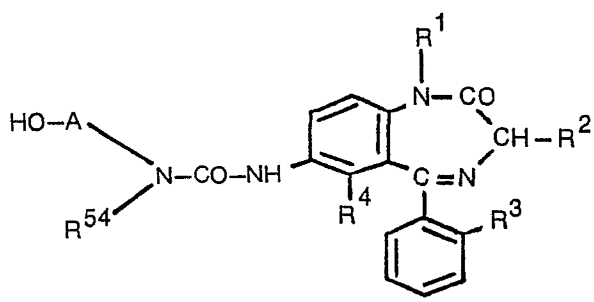

Ia

worin $R^1$, $R^2$, $R^3$ $R^4$, $R^{54}$ und A obige Bedeutung besitzen, ($C_1$—$C_7$) -alkanoyliert, oder

h) in einem Benzodiazepin-Derivat der allgemeinen Formel (Ib)

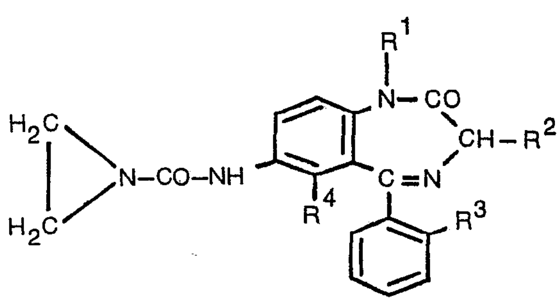

Ib

worin $R^1$, $R^2$, $R^3$ und $R^4$ obige Bedeutung besitzen, den Aziridinring hydrolytisch öffnet, oder i) ein Benzodiazepin-Derivat der allgemeinen Formel (I) in ein pharmazeutisch akzeptables Säureadditionssalz überführt.

2. Verfahren nach Anspruch 1, dadurch gekennzeichnet, dass man nach den Varianten a) bis g) oder i) arbeitet.

3. Verfahren nach Anspruch 1 oder 2, dadurch gekennzeichnet, dass $R^2$ Wassserstoff, $R^3$ Fluor und $R^4$ Wasserstoff oder Chlor bedeuten.

4. Verfahren nach Anspruch 1 oder 2, dadurch gekennzeichnet, dass $R^4$ und $R^5$ Wasserstoff und $R^6$ ($C_2$—$C_7$)-Alkyl oder Hydroxy-($C_2$—$C_7$)-alkyl bedeuten.

5. Verfahren nach Anspruch 1 oder 2, dadurch gekennzeichnet, dass $R^4$ Halogen bedeutet und entweder $R^5$ Wasserstoff und $R^6$ ($C_2$—$C_7$)-Alkyl oder Hydroxy-($C_2$—$C_7$)-alkyl bedeuten oder $R^5$ und $R^6$ je ($C_1$—$C_7$)-Alkyl bedeuten oder $R^5$ und $R^6$ zusammen mit dem Stickstoffatom einen 3- bis 7-gliedrigen Heterocyclus ohne zusätzliches Heteroatom oder einen N-Methylpiperazin-, Thiazolidin-, Morpholin- oder Thiomorpholinrest bedeuten.

6. Verfahren nach Anspruch 5, dadurch gekennzeichnet, dass $R^4$ Brom oder Chlor bedeutet.

7. Verfahren nach einem der Ansprüche 4 bis 6, dadurch gekennzeichnet, dass $R^3$ Fluor oder Chlor bedeutet.

8. Verfahren nach Anspruch 3, dadurch gekennzeichnet, dass man 1 - [5 - (o - Fluorphenyl) - 2,3 - dihydro - 1 - methyl - 2 - oxo - 1H - 1,4 - benzodiazepin - 7 - yl] - 3 - (2 - hydroxyäthyl)harnstoff herstellt.

9. Verfahren nach Anspruch 3, dadurch gekennzeichnet, dass man 1 - [5 - (o - Fluorphenyl) - 2,3 - dihydro - 1 - methyl - 2 - oxo - 1H - 1,4 - benzodiazepin - 7 - yl] - 3 - methylharnstoff herstellt.

10. Verfahren nach Anspruch 3, dadurch gekennzeichnet, dass man 1 - [5 - (o - Fluorphenyl) - 2,3 - dihydro - 1 - methyl - 2 - oxo - 1H - 1,4 - benzodiazepin - 7 - yl] - 3 - (2 - hydroxy - 1 - methyläthyl)harnstoff herstellt.

11. Verfahren nach Anspruch 3, dadurch gekennzeichnet, dass man N - [5 - (o - Fluorphenyl) - 2,3 - dihydro - 1 - methyl - 2 - oxo - 1H - 1,4 - benzodiazepin - 7 - yl] - 1 - pyrrolidincarboxamid herstellt.

12. Verfahren nach Anspruch 3, dadurch gekennzeichnet, dass man 1 - [6 - Chlor - 5 - (o - fluorphenyl) - 2,3 - dihydro - 1 - methyl - 2 - oxo - 1H - 1,4 - benzodiazepin - 7 - yl] - 3 - methylharnstoff herstellt.

13. Verfahren nach Anspruch 3, dadurch gekennzeichnet, dass man 1 - n - Butyl - 3 - [6 - chlor - 5 - (o - fluorphenyl) - 2,3 - dihydro - 1 - methyl - 2 - oxo - 1H - 1,4 - benzodiazepin - 7 - yl]harnstoff herstellt.

14. Verfahren nach Anspruch 3, dadurch gekennzeichnet, dass man 3 - [6 - Chlor - 5 - (o - fluorphenyl) - 2,3 - dihydro - 1 - methyl - 2 - oxo - 1H - 1,4 - benzodiazepin - 7 - yl] - 1 - äthyl - 1 - methylharnstoff herstellt.

15. Verfahren nach Anspruch 6, dadurch gekennzeichnet, dass man 1 - [6 - Brom - 5 - (o - chlorphenyl) - 2,3 - dihydro - 1,3 - dimethyl - 2 - oxo - 1H - 1,4 - benzodiazepin - 7 - yl] - 3 - (2 - hydroxyäthyl)harnstoff herstellt.

16. Verfahren nach Anspruch 6, dadurch gekennzeichnet, dass man N - [6 - Chlor - 5 - (o - fluorphenyl) - 2,3 - dihydro - 1 - methyl - 2 - oxo - 1H - 1,4 - benzodiazepin - 7 - yl] - 4 - morpholincarboxamid herstellt.

17. Verfahren nach Anspruch 6, dadurch gekennzeichnet, dass man 1 - [6 - Brom - 5 - (o - fluorphenyl) - 2,3 - dihydro - 1,3 - dimethyl - 2 - oxo - 1H - 1,4 - benzodiazepin - 7 - yl] - 3 - (2 - hydroxyäthyl)harnstoff herstellt.

18. Verfahren nach Anspruch 6, dadurch gekennzeichnet, dass man 1 - [6 - Chlor - 5 - (o - chlorphenyl) - 2,3 - dihydro - 1,3 - dimethyl - 2 - oxo - 1H - 1,4 - benzodiazepin - 7 - yl] - 3 - (2 - hydroxyäthyl)harnstoff herstellt.

**Revendications pour les Etats contractants: BE CH DE FR GB IT LU NL SE**

1. Dérivés de benzodiazépine de formule générale (I)

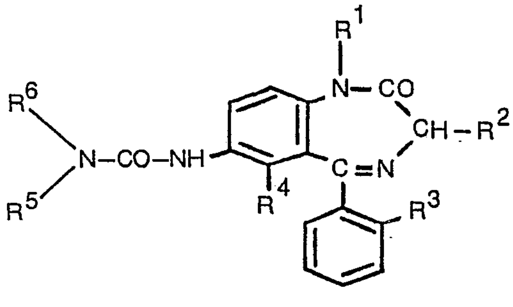

I

où $R^1$ représente un alcoyle en $C_1$ à $C_7$; $R^2$ représente un hydrogène ou un alcoyle en $C_1$ à $C_7$; $R^3$ représente un halogène et $R^4$ un hydrogène ou un halogène et où ou bien $R^5$ représente un hydrogène ou un alcoyle en $C_1$ à $C_7$ et $R^6$ représente un alcoyle en $C_1$ à $C_7$, un hydroxy-alcoyle en $C_2$ à $C_7$ ou un alcanoyloxy en $C_1$ à $C_7$-alcoyle en $C_2$ à $C_7$, ou bien $R^5$ représente un hydrogène et $R^6$ un radical phényle ou phényl-alcoyle en $C_1$ à $C_7$ éventuellement substitué par un halogène ou un alcoxy en $C_1$ à $C_7$ ou bien $R^5$ et $R^6$ représentent ensemble avec l'atome d'azote un hétérocycle à 3 à 7 chaînons sans heteroatome supplémentaire ou un radical N-méthylpipérazine, thiazolidine, morpholine ou thiomorpholine, et les sels d'addition d'acides pharmaceutiquement acceptables des dérivés de benzodiazépine de formule (I).

2. Composés selon la revendication 1, caractérisés en ce que $R^2$ représente un hydrogène, $R^3$ un fluor et $R^4$ un hydrogène ou un chlore.

3. Composés selon la revendication 1, caractérisés en ce que $R^4$ et $R^5$ représente un hydrogène et $R^6$ un alcoyle en $C_2$ à $C_7$ ou un hydroxyalcoyle en $C_2$ à $C_7$.

4. Composés selon la revendication 1, caractérisés en ce que $R^4$ représente un halogène et ou bien $R^5$ représente un hydrogène et $R^6$ un alcoyle en $C_2$ à $C_7$ ou un hydroxyalcoyle en $C_2$ à $C_7$, ou bien $R^5$ et $R^6$ représentent chacun un alcoyle en $C_1$ à $C_7$ ou bien $R^5$ et $R^6$ représentent ensemble avec l'atome d'azote un hétérocycle à 3 à 7 chaînons sans hétéroatome supplémentaire ou un radical N-méthylpipérazine, thiazolidine, morpholine ou thiomorpholine.

5. Composés selon la revendication 4, caractérisés en ce que $R^4$ représente un brome ou un chlore.

6. Composés selon l'une des revendications 3 à 5, caractérisés en ce que $R^3$ représente un fluor ou un chlore.

7. 1 - [5 - (o - Fluorophényl) - 2,3 - dihydro - 1 - méthyl - 2 - oxo - 1H - 1,4 - benzodiazépin - 7 - yl] - 3 - (2 - hydroxyéthyl)urée.

8. 1 - [5 - (o - Fluorophényl) - 2,3 - dihydro - 1 - méthyl - 2 - oxo - 1H - 1,4 - benzodiazépin - 7 - yl] - 3 - méthylurée.

9. 1 - [5 - (o - Fluorophényl) - 2,3 - dihydro - 1 - méthyl - 2 - oxo - 1H - 1,4 - benzodiazépin - 7 - yl] - 3 - (2 - hydroxy - 1 - méthyléthyl)urée.

10. N - [5 - (o - Fluorophényl) - 2,3 - dihydro - 1 - méthyl - 2 - oxo - 1H - 1,4 - benzodiazépin - 7 - yl] - 1 - pyrrolidinecarboxamide.

11. 1 - [6 - Chloro - 5 - (o - fluorophényl) - 2,3 - dihydro - 1 - méthyl - 2 - oxo - 1H - 1,4 - benzodiazépin - 7 - yl] - 3 - méthylurée.

12. 1 - n - Butyl - 3 - [6 - chloro - 5 - (o - fluorophényl) - 2,3 - dihydro - 1 - méthyl - 2 - oxo - 1H - 1,4 - benzodiazépin - 7 - yl]urée.

13. 3 - [6 - Chloro - 5 - (o - fluorophényl) - 2,3 - dihydro - 1 - méthyl - 2 - oxo - 1H - 1,4 - benzodiazépin - 7 - yl] - 1 - éthyl - 1 - méthylurée.

14. 1 - [6 - Bromo - 5 - (o - chlorophényl) - 2,3 - dihydro - 1,3 - diméthyl - 2 - oxo - 1H - 1,4 - benzodiazépin - 7 - yl] - 3 - (2 - hydroxyéthyl)urée.

15. N - [6 - Chloro - 5 - (o - fluorophényl) - 2,3 - dihydro - 1 - méthyl - 2 - oxo - 1H - 1,4 - benzodiazépin - 7 - yl] - 4 - morpholinecarboxamide.

16. 1 - [6 - Bromo - 5 - (o - fluorophényl) - 2,3 - dihydro - 1,3 - diméthyl - 2 - oxo - 1H - 1,4 - benzodiazépin - 7 - yl] - 3 - (2 - hydroxyéthyl)urée.

17. 1 - [6 - Chloro - 5 - (o - chlorophényl) - 2,3 - dihydro - 1,3 - diméthyl - 2 - oxo - 1H - 1,4 - benzodiazépin - 7 - yl] - 3 - (2 - hydroxyéthyl)urée.

18. 1 - Tert. - butyl - 3 - [5 - (o - fluorophényl) - 2,3 - dihydro - 1 - méthyl - 2 - oxo - 1H - 1,4 - benzodiazépin - 7 - yl]urée;

1 - [5 - (o - fluorophényl) - 2,3 - dihydro - 1 - méthyl - 2 - oxo - 1H - 1,4 - benzodiazépin - 7 - yl] - 3 - (p - méthoxyphényl)urée;

1 - benzyl - 3 - [5 - (o - fluorophényl) - 2,3 - dihydro - 1 - méthyl - 2 - oxo - 1H - 1,4 - benzodiazépin - 7 - yl]urée;

3 - [5 - (o - fluorophényl) - 2,3 - dihydro - 1 - méthyl - 2 - oxo - 1H - 1,4 - benzodiazépin - 7 - yl] - 1,1 - diméthylurée;

1 - éthyl - 3 - [5 - (o - fluorophényl) - 2,3 - dihydro - 1 - méthyl - 2 - oxo - 1H - 1,4 - benzodiazépin - 7 - yl] - 1 - méthylurée; et

N - [5 - (o - fluorophényl) - 2,3 - dihydro - 1 - méthyl - 2 - oxo - 1H - 1,4 - benzodiazépin - 7 - yl] - 1 - aziridinecarboxamide.

19. 1 - [6 - Chloro - 5 - (o - fluorophényl) - 2,3 - dihydro - 1 - méthyl - 2 - oxo - 1H - 1,4 - benzodiazépin - 7 - yl] - 3 - éthylurée;

1 - [6 - chloro - 5 - (o - fluorophényl) - 2,3 - dihydro - 1 - méthyl - 2 - oxo - 1H - 1,4 - benzodiazépin - 7 - yl] - 3 - (2 - hydroxyéthyl)urée; et

3 - [6 - chloro - 5 - (o - fluorophényl) - 2,3 - dihydro - 1 - méthyl - 2 - oxo - 1H - 1,4 - bensodiazépin - 7 - yl] - 1,1 - diéthylurée.

20. Composés selon l'une des revendications 1 à 19 aux fins d'application comme substances actives pharmaceutiques.

21. Composés selon l'une des revendications 1, 2, 7 à 13, 18 et 19 aux fins d'application comme substances actives antagonistes de l'aldostérone.

22. Composés selon l'une des revendications 3 à 6 et 14 à 17 aux fins d'application comme substances actives inhibant la résorption intestinale du cholestérol.

23. Procédé de préparation de dérivés de benzodiazépine selon la revendication 1, caractérisé en ce que

a) on fait réagir un dérivé de benzodiazépine préparé in situ de formule générale (II)

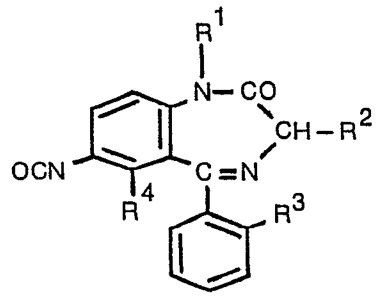

II

où $R^1$, $R^2$, $R^3$ et $R^4$ ont la signification donnée dans la revendication 1, avec un composé amino de formule générale (III)

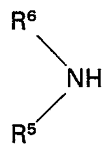

III

où $R^5$ et $R^6$ ont la signification donnée dans la revendication 1, ou

b) on cyclise en solution un composé de formule générale (IV) préparé in situ

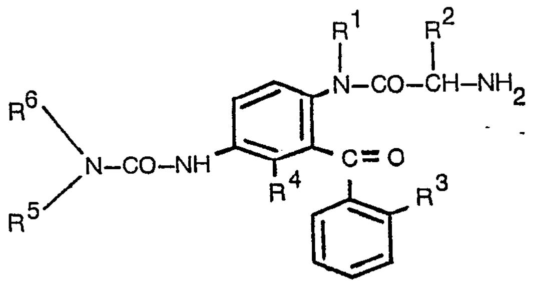

IV

où $R^1$, $R^2$, $R^3$, $R^4$, $R^5$ et $R^6$ ont la signification donnée dans la revendication 1, ou

c) on fait réagir un dérivé de benzodiazépine de formule générale (V)

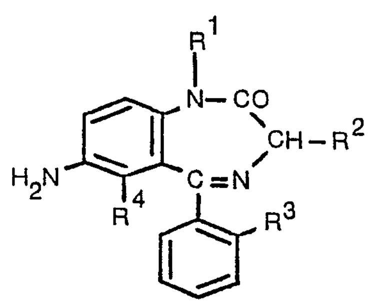

V

où $R^1$, $R^2$, $R^3$ et $R^4$ ont la signification donnée dans la revendication 1,

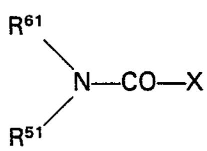

VI

où X représente un halogène et ou bien $R^{51}$ représente un alcoyle en $C_1$ à $C_7$ et $R^{61}$ représente un alcoyle en $C_1$ à $C_7$ ou un alcanoyloxy en $C_1$ à $C_7$-alcoyle en $C_2$ à $C_7$, ou bien $R^{51}$ et $R^{61}$ représentent ensemble

avec l'atome d'azote un hétérocycle à 3 à 7 chaînons sans hétéroatome supplémentaire ou un radical N-méthylpipérazine, thiazolidine, morpholine ou thiomorpholine; ou

d) on fait réagir un dérivé de benzodiazépine de formule générale (V) ci-dessus avec un isocyanate de formule générale (VII)

$$R^{62}—NCO \qquad VII$$

où $R^{62}$ représente un alcoyle en $C_1$ à $C_7$, un alcanoyloxy en $C_1$ à $C_7$-alcoyle en $C_2$ à $C_7$ ou un radical phényle ou phénylalcoyle en $C_1$ à $C_7$ éventuellement monosubstitué par un halogène ou un alcoxy en $C_1$ à $C_7$, ou

e) on retire le (les) groupe(s) protecteur(s) d'un dérivé de benzodiazépine de formule générale (VIII)

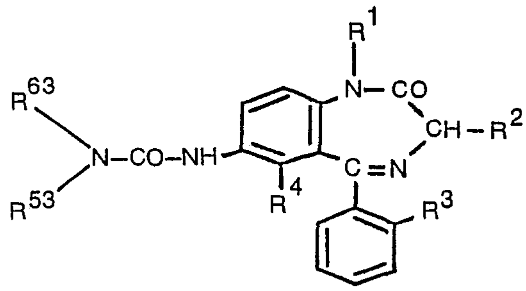

VIII

où $R^1$, $R^2$, $R^3$ et $R^4$ ont la signification donnée dans la revendication 1 et où bien $R^{53}$ représente un groupe protecteur et $R^{63}$ un alcoyle en $C_1$ à $C_7$, un radical phényle ou phénylalcoyle en $C_1$ à $C_7$ éventuellement monosubstitué par un halogène ou un alcoxy en $C_1$ à $C_7$ ou un radical de formule (IX)

$$—A—O—Y \qquad IX$$

où A représente un alcoylène en $C_2$ à $C_7$ et Y un groupe protecteur, ou bien $R^{53}$ représente un hydrogène ou un alcoyle en $C_1$ à $C_7$ et $R^{63}$ représente un radical de formule (IX) ci-dessus, ou

f) on transforme un dérivé de benzodiazépine de formule générale (X)

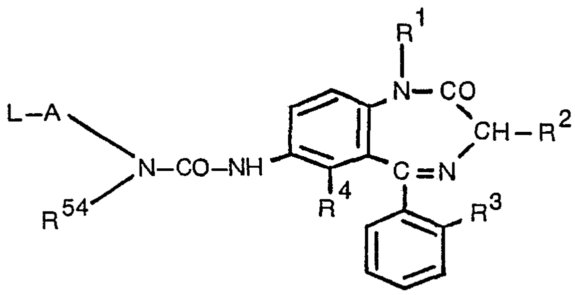

X

où $R^1$, $R^2$, $R^3$ et $R^4$ ont la signification donnée dans la revendication 1 et A a la signification donnée ci-dessus et $R^{54}$ représente un hydrogène ou un alcoyle en $C_1$ à $C_7$ et L représente un atome d'halogène, un groupe arylsulfonyloxy, alcoylsulfonyloxy ou ammonium quaternaire, en un composé hydroxy ou alcanoyloxy en $C_1$ à $C_7$ correspondant, ou

g) on traite avec un alcanoyle en $C_1$ à $C_7$ un dérivé de benzodiazépine de formule générale (Ia)

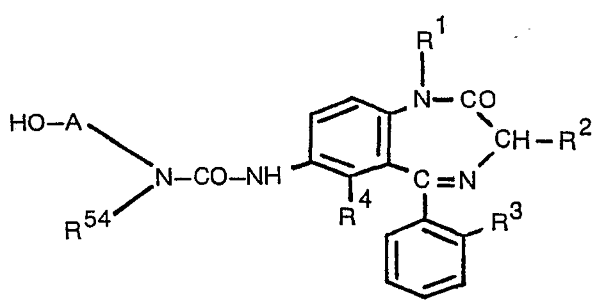

Ia

où $R^1$, $R^2$, $R^3$ et $R^4$ ont la signification donnée dans la revendication 1 et $R^{54}$ et A ont la signification donnée ci-dessus, ou

h) dans un dérivé de benzodiazépine de formule générale (Ib)

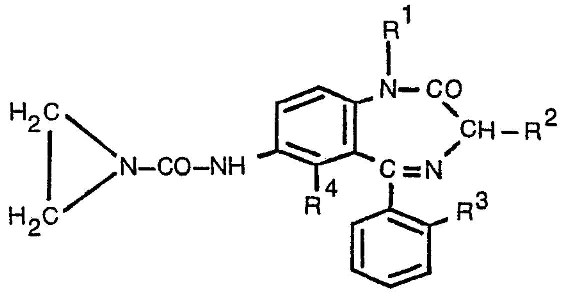

Ib

où $R^1$, $R^2$, $R^3$ et $R^4$ ont la signification donnée dans la revendication 1, on ouvre par hydrolyse le noyau aziridine, ou

i) on transforme un dérivé de benzodiazépine de formule générale (I) en un sel d'addition d'acide pharmaceutiquement acceptable.

24. Procédé selon la revendication 23, caractérisé en ce qu'on travaille selon les variantes a) à g) ou i).

25. Médicament contenant un composé selon l'une des revendications 1 à 19.

26. Agent antagoniste de l'aldostérone, contenant un composé selon l'une des revendications 1, 2, 7 à 13, 18 et 19.

27. Agent inhibant la résorption intestinale de cholestérol, contenant un composé selon l'une des revendications 3 à 6 et 14 à 17.

**Revendications pour l'Etat contractant: AT**

1. Procédé de préparation de dérivés de benzodiazépine de formule générale (I)

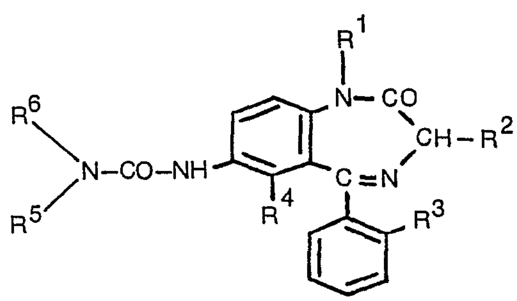

I

où $R^1$ représente un alcoyle en $C_1$ à $C_7$; $R^2$ représente un hydrogène ou un alcoyle en $C_1$ à $C_7$; $R^3$ représente un halogène et $R^4$ un hydrogène ou un halogène et où ou bien $R^5$ représente un hydrogène ou un alcoyle en $C_1$ à $C_7$ et $R^6$ représente un alcoyle en $C_1$ à $C_7$, un hydroxy-alcoyle en $C_2$ à $C_7$ ou un alcanoyloxy en $C_1$ à $C_7$-alcoyle en $C_1$ à $C_7$, ou bien $R^5$ représente un hydrogène et $R^6$ un radical phényle ou phényl-alcoyle en $C_1$ à $C_7$ éventuellement substitué par un halogène ou un alcoxy en $C_1$ à $C_7$ ou bien $R^5$ et $R^6$ représentent ensemble avec l'atome d'azote un hétérocycle à 3 à 7 chaînons sans hétéroatome supplémentaire ou un radical N-méthylpipérazine, thiazolidine, morpholine ou thiomorpholine, et des sels d'addition d'acides pharmaceutiquement acceptables de dérivés de benzodiazépine de formule (I), caractérisé en ce que:

a) on fait réagir un dérivé de benzodiazépine préparé in situ de formule générale (II)

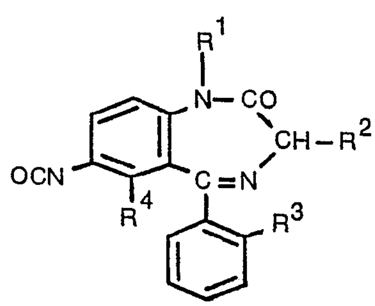

II

où $R^1$, $R^2$, $R^3$ et $R^4$ ont la signification donnée ci-dessus,

avec un composé amino de formule générale (III)

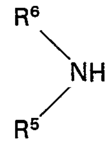

III

où $R^5$ et $R^6$ ont la signification donnée ci-dessus, ou

b) on cyclise en solution un composé de formule générale (IV) préparé in situ

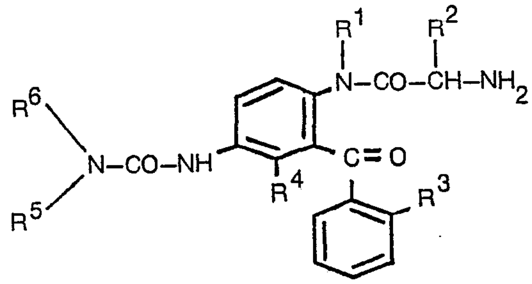

IV

où $R^1$, $R^2$, $R^3$, $R^4$, $R^5$ et $R^6$ ont la signification donnée ci-dessus, ou

c) on fait réagir un dérivé de benzodiazépine de formule générale (V)

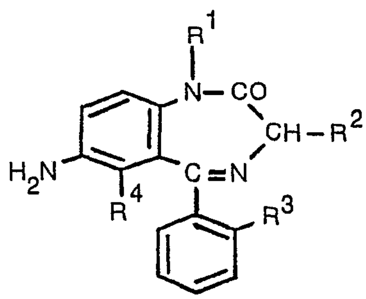

V

où $R^1$, $R^2$, $R^3$ et $R^4$ ont la signification donnée ci-dessus, avec un halogénure de formule générale (VI)

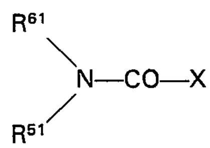

VI

où X représente un halogène et ou bien $R^{51}$ représente un alcoyle en $C_1$ à $C_7$ et $R^{61}$ représente un alcoyle en $C_1$ à $C_7$ ou un alcanoyloxy en $C_1$ à $C_7$-alcoyle en $C_2$ à $C_7$, ou bien $R^{51}$ et $R^{61}$ représentent ensemble avec l'atome d'azote un hétérocycle à 3 à 7 chaînons sans hétéroatome supplémentaire ou un radical N-méthylpipérazine, thiazolidine, morpholine ou thiomorpholine; ou

d) on fait réagir un dérivé de benzodiazépine de formule générale (V) ci-dessus avec un isocyanate de formule génerale (VII)

$$R^{62}—NCO \qquad VII$$

où $R^{62}$ représente un alcoyle en $C_1$ à $C_7$, un alcanoyloxy en $C_1$ à $C_7$-alcoyle en $C_2$ à $C_7$ ou un radical phényle ou phénylalcoyle en $C_1$ à $C_7$ éventuellement monosubstitué par un halogène ou un alcoxy en $C_1$ à $C_7$, ou

e) on retire le (les) groupe(s) protecteur(s) d'un dérivé de benzodiazépine de formule générale (VIII)

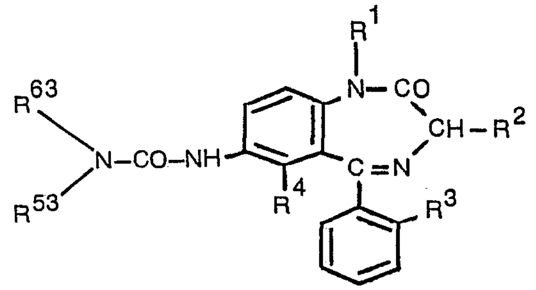

VIII

où $R^1$, $R^2$, $R^3$ et $R^4$ ont la signification donnée ci-dessus et où ou bien $R^{53}$ représente un groupe protecteur et $R^{63}$ un alcoyle en $C_1$ à $C_7$, un radical phényle ou phénylalcoyle en $C_1$ à $C_7$ éventuellement monosubstitué par un halogène ou un alcoxy en $C_1$ à $C_7$ ou un radical de formule (IX)

$$—A—O—Y \qquad IX$$

où A représente un alcoylène en $C_2$ à $C_7$ et Y un groupe protecteur, ou bien $R^{53}$ représente un hydrogène ou un alcoyle en $C_1$ à $C_7$ et $R^{63}$ représente un radical de formule (IX) ci-dessus, ou

f) on transforme un dérivé de benzodiazépine de formule générale (X)

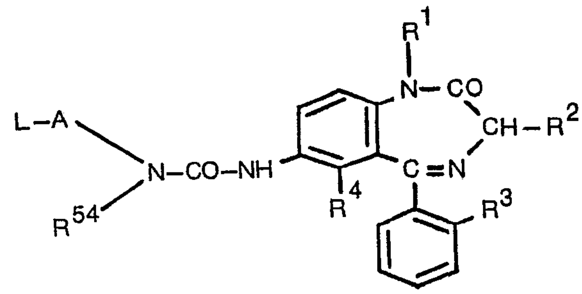

X

où $R^1$, $R^2$, $R^3$, $R^4$ et A ont la signification donnée ci-dessus et $R^{54}$ représente un hydrogène ou un alcoyle en $C_1$ à $C_7$ et L représente un atome d'halogène, un groupe arylsulfonyloxy, alcoylsulfonyloxy ou ammonium quaternaire, en un composé hydroxy ou alcanoyloxy en $C_1$ à $C_7$ correspondant, ou

g) on traite avec un alcanoyle en $C_1$ à $C_7$ un dérivé de benzodiazépine de formule générale (Ia)

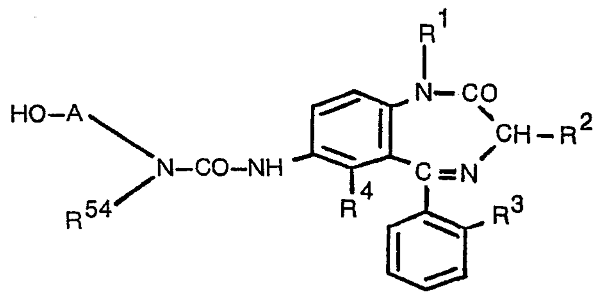

Ia

où $R^1$, $R^2$, $R^3$, $R^4$, $R^{54}$ et A ont la signification donnée ci-dessus, ou

h) dans un dérivé de benzodiazépine de formule générale (Ib)

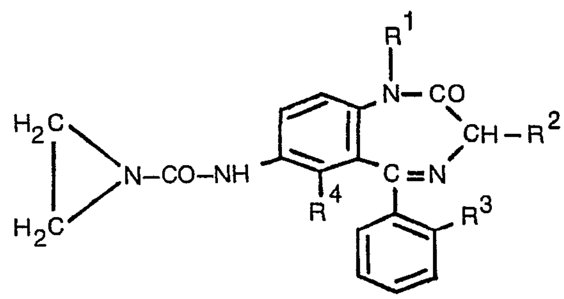

Ib

où $R^1$, $R^2$, $R^3$ et $R^4$ ont la signification donnée ci-dessus, on ouvre par hydrolyse le noyau aziridine, ou

i) on transforme un dérivé de benzodiazépine de formule générale (I) en un sel d'addition d'acide pharmaceutiquement acceptable.

2. Procédé selon la revendication 1, caractérisé en ce qu'on travaille selon les variantes a) à g) ou i).

3. Procédé selon la revendication 1 ou 2, caractérisé en ce que $R^2$ représente un hydrogène, $R^3$ un fluor et $R^4$ un hydrogène ou un chlore.

4. Procédé selon la revendication 1 ou 2, caractérisé en ce que $R^4$ et $R^5$ représentent un hydrogène et $R^6$ un alcoyle en $C_2$ à $C_7$ ou un hydroxyalcoyle en $C_2$ à $C_7$.

5. Procédé selon la revendication 1 ou 2, caractérisé en ce que $R^4$ représente un halogène et ou bien $R^5$ représente un hydrogène et $R^6$ représente un alcoyle en $C_2$ à $C_7$ ou un hydroxyalcoyle en $C_2$ à $C_7$, ou bien $R^5$ et $R^6$ représentent chacun un alcoyle en $C_1$ à $C_7$, ou bien $R^5$ et $R^6$ représentent ensemble avec l'atome d'azote un hétérocycle à 3 à 7 chaînons sans hétéroatome supplémentaire ou un radical N-méthylpipérazine, thiazolidine, morpholine ou thiomorpholine.

6. Procédé selon la revendication 5, caractérisé en ce que $R^4$ représente un brome ou un chlore.

7. Procédé selon l'une des revendications 4 à 6, caractérisé en ce que $R^3$ représente un fluor ou un chlore.

8. Procédé selon la revendication 3, caractérisé en ce qu'on prépare la 1 - [5 - (o - fluorophényl) - 2,3 - dihydro - 1 - méthyl - 2 - oxo - 1H - 1,4 - benzodiazépin - 7 - yl] - 3 - (2 - hydroxyéthyl)urée.

9. Procédé selon la revendication 3, caractérisé en ce qu'on prépare la 1 - [5 - (o - fluorophényl) - 2,3 - dihydro - 1 - méthyl - 2 - oxo - 1H - 1,4 - benzodiazépin - 7 - yl] - 3 - méthylurée.

10. Procédé selon la revendication 3, caractérisé en ce qu'on prépare la 1 - [5 - (o - fluorophényl) - 2,3 - dihydro - 1 - méthyl - 2 - oxo - 1H - 1,4 - benzodiazépin - 7 - yl] - 3 - (2 - hydroxy - 1 - méthyléthyl)urée.

11. Procédé selon la revendication 3, caractérisé en ce qu'on prépare le N - [5 - (o - fluorophényl) - 2,3 - dihydro - 1 - méthyl - 2 - oxo - 1H - 1,4 - benzodiazépin - 7 - yl] - 1 - pyrrolidinecarboxamide.

12. Procédé selon la revendication 3, caractérisé en ce qu'on prépare la 1 - [6 - chloro - 5 - (o - fluorophényl) - 2,3 - dihydro - 1 - méthyl - 2 - oxo - 1H - 1,4 - benzodiazépin - 7 - yl] - 3 - méthylurée.

13. Procédé selon la revendication 3, caractérisé en ce qu'on prépare la 1 - n - butyl - 3 - [6 - chloro - 5 - (o - fluorophényl) - 2,3 - dihydro - 1 - méthyl - 2 - oxo - 1H - 1,4 - benzodiazépin - 7 - yl[urée.

14. Procédé selon la revendication 3, caractérise' en ce qu'on prépare la 3 - [6 - chloro - 5 - (o - fluorophényl) - 2,3 - dihydro - 1 - méthyl - 2 - oxo - 1H - 1,4 - benzodiazépin - 7 - yl] - 1 - éthyl - 1 - méthylurée.

15. Procédé selon la revendication 6, caractérisé en ce qu'on prépare la 1 - [6 - bromo - 5 - (o - chlorophényl) - 2,3 - dihydro - 1,3 - diméthyl - 2 - oxo - 1H - 1,4 - benzodiazépin - 7 - yl] - 3 - (2 - hydroxyéthyl)urée.

16. Procédé selon la revendication 6, caractérisé en ce qu'on prépare le N - [6 - chloro - 5 - (o - fluorophényl) - 2,3 - dihydro - 1 - méthyl - 2 - oxo - 1H - 1,4 - benzodiazépin - 7 - yl] - 4 - morpholinecarboxamide.

17. Procédé selon la revendication 6, caractérisé en ce qu'on prépare la 1 - [6 - bromo - 5 - (o - fluorophényl) - 2,3 - dihydro - 1,3 - diméthyl - 2 - oxo - 1H - 1,4 - benzodiazépin - 7 - yl] - 3 - (2 - hydroxyéthyl)urée.

18. Procédé selon la revendication 6, caractérisé en ce qu'on prépare la 1 - [6 - chloro - 5 - (o - chlorophényl) - 2,3 - dihydro - 1,3 - diméthyl - 2 - oxo - 1H - 1,4 - benzodiazépin - 7 - yl] - 3 - (2 - hydroxyéthyl)urée.

**Claims for the Contracting States: BE CH DE FR GB IT LU NL SE**

1. Benzodiazepine derivatives of the general formula (I)

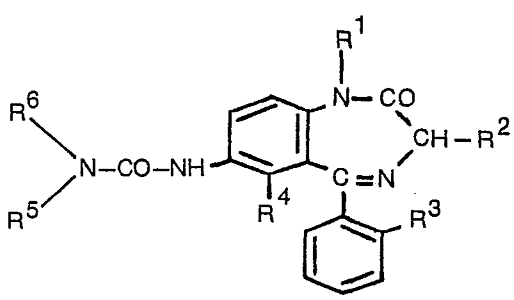

I

wherein $R^1$ signifies $(C_1-C_7)$-alkyl, $R^2$ signifies hydrogen or $(C_1-C_7)$-alkyl, $R^3$ signifies halogen and $R^4$ signifies hydrogen or halogen and either $R^5$ signifies hydrogen or $(C_1-C_7)$-alkyl and $R^6$ signifies $(C_1-C_7)$-alkyl, hydroxy-$(C_2-C_7)$-alkyl or $(C_1-C_7)$-alkanoyloxy-$(C_2-C_7)$-alkyl or $R^5$ signifies hydrogen and $R^6$ signifies a phenyl or phenyl-$(C_1-C_7)$-alkyl residue optionally mono-substituted by halogen or $(C_1-C_7)$-alkoxy or $R^5$ and $R^6$ together with the nitrogen atom signify a 3- to 7-membered heterocycle without an additional hetero atom or a N-methylpiperazine, thiazolidine, morpholine or thiomorpholine residue, and pharmaceutically acceptable acid addition salts of benzodiazepine derivatives of formula I.

2. Compounds according to claim 1, characterized in that $R^2$ signifies hydrogen, $R^3$ signifies fluorine and $R^4$ signifies hydrogen or chlorine.

3. Compounds according to claim 1, characterized in that $R^4$ and $R^5$ signify hydrogen and $R^6$ signifies $(C_2-C_7)$-alkyl or hydroxy-$(C_2-C_7)$-alkyl.

4. Compounds according to claim 1, characterized in that $R^4$ signifies halogen and either $R^5$ signifies hydrogen and $R^6$ signifies $(C_2-C_7)$-alkyl or hydroxy-$(C_2-C_7)$-alkyl or $R^5$ and $R^6$ each signify

($C_1$—$C_7$)-alkyl or $R^5$ and $R^6$ together with the nitrogen atom signify a 3- to 7-membered heterocycle without an additional hetero atom or a N-methylpiperazine, thiazolidine, morpholine or thiomorpholine residue.

5. Compounds according to claim 4, characterized in that $R^4$ signifies bromine or chlorine.

6. Compounds according to any one of claims 3 to 5, characterized in that $R^3$ signifies fluorine or chlorine.

7. 1 - [5 - (o - Fluorophenyl) - 2,3 - dihydro - 1 - methyl - 2 - oxo - 1H - 1,4 - benzodiazepin - 7 - yl] - 3 - (2 - hydroxyethyl)urea.

8. 1 - [5 - (o - Fluorophenyl) - 2,3 - dihydro - 1 - methyl - 2 - oxo - 1H - 1,4 - benzodiazepin - 7 - yl] - 3 - methylurea.

9. 1 - [5 - (o - Fluorophenyl) - 2,3 - dihydro - 1 - methyl - 2 - oxo - 1H - 1,4 - benzodiazepin - 7 - yl] - 3 - (2 - hydroxy - 1 - methylethyl)urea.

10. N - [5 - (o - Fluorophenyl) - 2,3 - dihydro - 1 - methyl - 2 - oxo - 1H - 1,4 - benzodiazepin - 7 - yl] - 1 - pyrrolidinecarboxamide.

11. 1 - [6 - Chloro - 5 - (o - fluorophenyl) - 2,3 - dihydro - 1 - methyl - 2 - oxo - 1H - 1,4 - benzodiazepin - 7 - yl] - 3 - methylurea.

12. 1 - n - Butyl - 3 - [6 - chloro - 5 - (o - fluorophenyl) - 2,3 - dihydro - 1 - methyl - 2 - oxo - 1H - 1,4 - benzodiazepin - 7 - yl]urea.

13. 3 - [6 - Chloro - 5 - (o - fluorophenyl) - 2,3 - dihydro - 1 - methyl - 2 - oxo - 1H - 1,4 - benzodiazepin - 7 - yl] - 1 - ethyl - 1 - methylurea.

14. 1 - [6 - Bromo - 5 - (o - chlorophenyl) - 2,3 - dihydro - 1,3 - dimethyl - 2 - oxo - 1H - 1,4 - benzodiazepin - 7 - yl] - 3 - (2 - hydroxyethyl)urea.

15. N - [6 - Chloro - 5 - (o - fluorophenyl) - 2,3 - dihydro - 1 - methyl - 2 - oxo - 1H - 1,4 - benzodiazepin - 7 - yl] - 4 - morpholinecarboxamide.

16. 1 - [6 - Bromo - 5 - (o - fluorophenyl) - 2,3 - dihydro - 1,3 - dimethyl - 2 - oxo - 1H - 1,4 - benzodiazepin - 7 - yl] - 3 - (2 - hydroxyethyl)urea.

17. 1 - [6 - Chloro - 5 - (o - chlorophenyl) - 2,3 - dihydro - 1,3 - dimethyl - 2 - oxo - 1H - 1,4 - benzodiazepin - 7 - yl] - 3 - (2 - hydroxyethyl)urea.

18. 1 - Tert. - butyl - 3 - [5 - (o - fluorophenyl) - 2,3 - dihydro - 1 - methyl - 2 - oxo - 1H - 1,4 - benzodiazepin - 7 - yl]urea,

1 - [5 - (o - fluorophenyl) - 2,3 - dihydro - 1 - methyl - 2 - oxo - 1H - 1,4 - benzodiazepin - 7 - yl] - 3 - (p - methoxyphenyl)urea,

1 - benzyl - 3 - [5 - (o - fluorophenyl) - 2,3 - dihydro - 1 - methyl - 2 - oxo - 1H - 1,4 - benzodiazepin - 7 - yl]urea,

3 - [5 - (o - fluorophenyl) - 2,3 - dihydro - 1 - methyl - 2 - oxo - 1H - 1,4 - benzodiazepin - 7 - yl] - 1,1 - dimethylurea,

1 - ethyl - 3 - [5 - (o - fluorophenyl) - 2,3 - dihydro - 1 - methyl - 2 - oxo - 1H - 1,4 - benzodiazepin - 7 - yl] - 1 - methylurea and

N - [5 - (o - fluorophenyl) - 2,3 - dihydro - 1 - methyl - 2 - oxo - 1H - 1,4 - benzodiazepin - 7 - yl] - 1 - aziridinecarboxamide.

19. 1 - [6 - Chloro - 5 - (o - fluorophenyl) - 2,3 - dihydro - 1 - methyl - 2 - oxo - 1H - 1,4 - benzodiazepin - 7 - yl] - 3 - ethylurea,

1 - [6 - chloro - 5 - (o - fluorophenyl) - 2,3 - dihydro - 1 - methyl - 2 - oxo - 1H - 1,4 - benzodiazepin - 7 - yl] - 3 - (2 - hydroxyethyl)urea and

3 - [6 - chloro - 5 - (o - fluorophenyl) - 2,3 - dihydro - 1 - methyl - 2 - oxo - 1H - 1,4 - benzodiazepin - 7 - yl] - 1,1 - diethylurea.

20. Compounds according to any one of claims 1 to 19 for use as pharmaceutically active substances.

21. Compounds according to any one of claims 1, 2, 7 to 13, 18 and 19 for use as aldosterone-antagonistic active substances.

22. Compounds according to any one of claims 3 to 6 and 14 to 17 for use as active substances which inhibit the intestinal resorption of cholesterol.

23. Process for the manufacture of benzodiazepine derivatives according to claim 1, characterized by a) reacting a benzodiazepine derivative, prepared in situ, of the general formula (II)

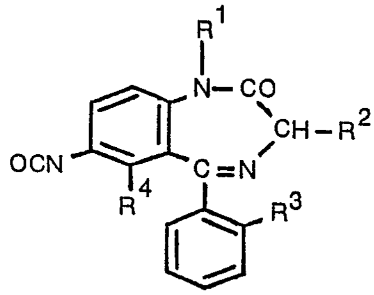

II

wherein $R^1$, $R^2$, $R^3$ and $R^4$ have the significance given in claim 1, with an amino compound of the general formula (III)

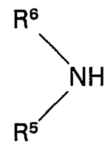

III

wherein $R^5$ and $R^6$ have the significance given in claim 1, or
b) cyclizing a compound, prepared in situ, of the general formula (IV)

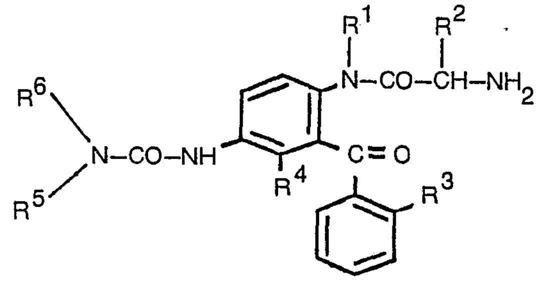

IV

wherein $R^1$, $R^2$, $R^3$, $R^4$, $R^5$ and $R^6$ have the significance given in claim 1, in solution, or
c) reacting a benzodiazepine derivative of the general formula (V)

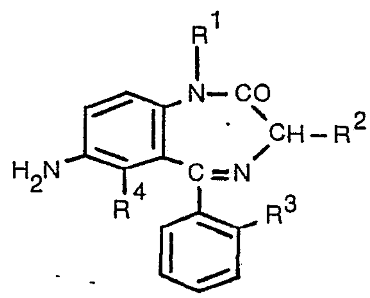

V

wherein $R^1$, $R^2$, $R^3$ and $R^4$ have the significance given in claim 1, with a halide of the general formula (VI)

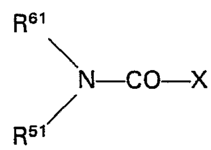

VI

wherein X signifies halogen and either $R^{51}$ signifies $(C_1$—$C_7)$-alkyl and $R^{61}$ signifies $(C_1$—$C_7)$-alkyl or $(C_1$—$C_7)$-alkanoyloxy-$(C_2$—$C_7)$-alkyl or $R^{51}$ and $R^{61}$ together with the nitrogen atom signify a 3- to 7-membered heterocycle without an additional hetero atom or a N-methylpiperazine, thiazolidine, morpholine or thiomorpholine residue, or
d) reacting a benzodiazepine derivative of general formula (V) above with an isocyanate of the general formula (VII)

$R^{62}$—NCO VII

wherein $R^{62}$ signifies $(C_1$—$C_7)$-alkyl, $(C_1$—$C_7)$-alkanoyloxy-$(C_2$—$C_7)$-alkyl or a phenyl or phenyl-$(C_1$—$C_7)$-alkyl residue optionally monosubstituted by halogen or a $(C_1$—$C_7)$-alkoxy, or
e) removing the protecting group(s) from a benzodiazepine derivative of the general formula (VIII)

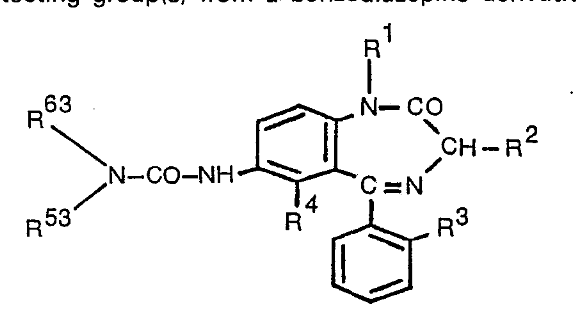

VIII

wherein $R^1$, $R^2$, $R^3$ and $R^4$ have the significance given in claim 1 and either $R^{53}$ signifies a protecting group and $R^{63}$ signifies ($C_1$—$C_7$)-alkyl, a phenyl or phenyl-($C_1$—$C_7$)-alkyl residue optionally mono-substituted by halogen or ($C_1$—$C_7$)-alkoxy or a residue of the formula (IX)

$$—A—O—Y \qquad IX$$

in which A signifies ($C_2$—$C_7$)-alkylene and Y signifies a protecting group, or $R^{53}$ signifies hydrogen or ($C_1$—$C_7$)-alkyl and $R^{63}$ signifies a residue of formula (IX) above, or
f) converting a benzodiazepine derivative of the general formula (X)

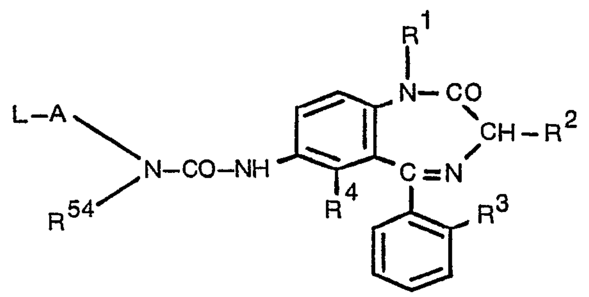

X

wherein $R^1$, $R^2$, $R^3$ and $R^4$ have the significance given in claim 1 and A has the above significance and $R^{54}$ signifies hydrogen or ($C_1$—$C_7$)-alkyl and L signifies a halogen atom, an arylsulphonyloxy, alkylsulphonyloxy or a quaternary ammonium group, into the corresponding hydroxy or ($C_1$—$C_7$)-alkanoyloxy compound, or
g) ($C_1$—$C_7$) alkanoylating a benzodiazepine derivative of the general formula (Ia)

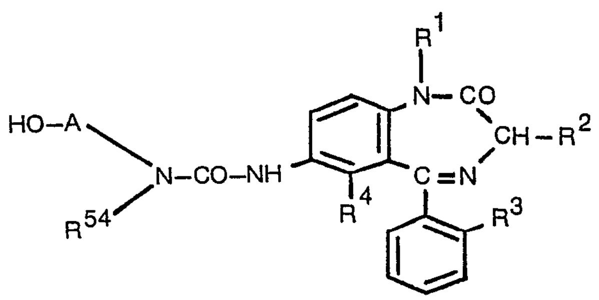

Ia

wherein $R^1$, $R^2$, $R^3$ and $R^4$ have the significance given in claim 1 and $R^{54}$ and A have the above significance, or
h) hydrolytically opening the aziridine ring in a benzodiazepine derivative of the general formula (Ib)

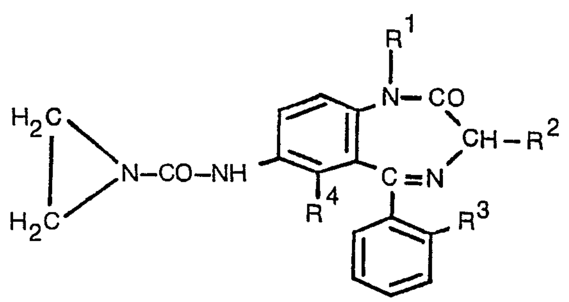

Ib

wherein $R^1$, $R^2$, $R^3$ and $R^4$ have the significance given in claim 1, or
i) converting a benzodiazepine derivative of general formula (I) into a pharmaceutically acceptable acid addition salt.

24. Process according to claim 23, characterized in that variants a) to g) or i) are carried out.

25. Medicament, containing a compound according to any one of claims 1 to 19.

26. Aldosterone-antagonistic medicament, containing a compound according to any one of claims 1, 2, 7 to 13, 18 and 19.

27. Medicament which inhibits the intestinal resorption of cholesterol, containing a compound according to any one of claims 3 to 6 and 14 to 17.

**Claims for the Contracting State: AT**

1. Process for the manufacture of benzodiazepine derivatives of the general formula (I)

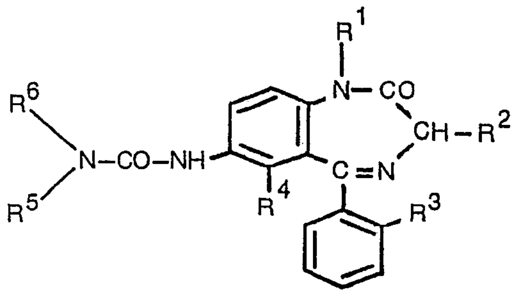

I

wherein $R^1$ signifies $(C_1—C_7)$-alkyl, $R^2$ signifies hydrogen or $(C_1—C_7)$-alkyl, $R^3$ signifies halogen and $R^4$ signifies hydrogen or halogen and either $R^5$ signifies hydrogen or $(C_1—C_7)$-alkyl and $R^6$ signifies $(C_1—C_7)$-alkyl, hydroxy-$(C_2—C_7)$-alkyl or $(C_1—C_7)$-alkanoyloxy-$(C_2—C_7)$-alkyl or $R^5$ signifies hydrogen and $R^6$ signifies a phenyl or phenyl-$(C_1—C_7)$-alkyl residue optionally monosubstituted by halogen or $(C_1—C_7)$-alkoxy or $R^5$ and $R^6$ together with the nitrogen atom signify a 3- to 7-membered heterocycle without an additional hetero atom or a N-methylpiperazine, thiazolidine, morpholine or thiomorpholine residue, and of pharmaceutically acceptable acid addition salts of benzodiazepine derivatives of formula I, characterized by

a) reacting a benzodiazepine derivative, prepared in situ, of the general formula (II)

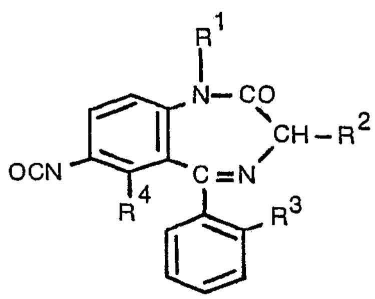

II

wherein $R^1$, $R^2$, $R^3$ and $R^4$ have the above significance, with an amino compound of the general formula (III)

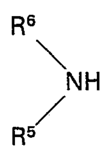

III

wherein $R^5$ and $R^6$ have the above significance, or

b) cyclizing a compound, prepared in situ, of the general formula (IV)

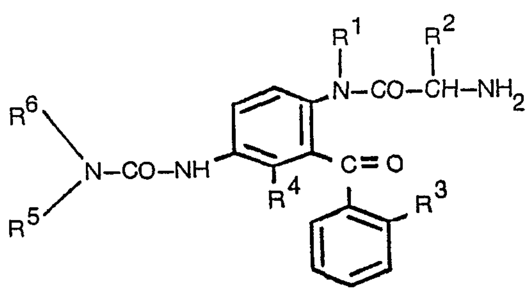

IV

wherein $R^1$, $R^2$, $R^3$, $R^4$, $R^5$ and $R^6$ have the above significance, in solution, or

c) reacting a benzodiazepine derivative of the general formula (V)

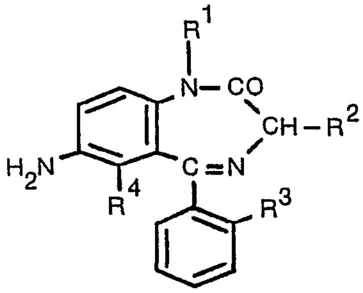

V

wherein $R^1$, $R^2$, $R^3$ and $R^4$ have the above significance, with a halide of the general formula (VI)

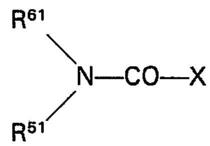

VI

wherein X signifies halogen and either $R^{51}$ signifies ($C_1$—$C_7$)-alkyl and $R^{61}$ signifies ($C_1$—$C_7$)-alkyl or ($C_1$—$C_7$)-alkanoyloxy-($C_2$—$C_7$)-alkyl or $R^{51}$ and $R^{61}$ together with the nitrogen atom signify a 3- to 7-membered heterocycle without an additional hetero atom or a N-methylpiperazine, thiazolidine, morpholine or thiomorpholine residue, or

d) reacting a benzodiazepine derivative of general formula (V) above with an isocyanate of the general formula (VII)

$$R^{62}\text{—NCO} \qquad \text{VII}$$

wherein $R^{62}$ signifies ($C_1$—$C_7$)-alkyl, ($C_1$—$C_7$)-alkanoyloxy-($C_2$—$C_7$)-alkyl or a phenyl or phenyl-($C_1$—$C_7$)-alkyl residue optionally monosubstituted by halogen or ($C_1$—$C_7$)-alkoxy, or

e) removing the protecting group(s) from a benzodiazepine derivative of the general formula (VIII)

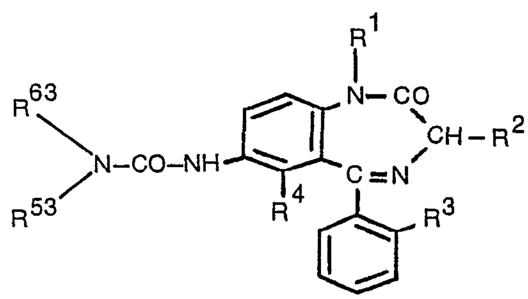

VIII

wherein $R^1$, $R^2$, $R^3$ and $R^4$ have the above significance and either $R^{53}$ signifies a protecting group and $R^{63}$ signifies ($C_1$—$C_7$)-alkyl, a phenyl or phenyl-($C_1$—$C_7$)-alkyl residue optionally monosubstituted by halogen or ($C_1$—$C_7$)-alkoxy or a residue of the formula (IX)

$$\text{—A—O—Y} \qquad \text{IX}$$

in which A signifies ($C_2$—$C_7$)-alkylene and Y signifies a protecting group, or $R^{53}$ signifies hydrogen or ($C_1$—$C_7$)-alkyl and $R^{63}$ signifies a residue of formula (IX) above, or

f) converting a benzodiazepine derivative of the general formula (X)

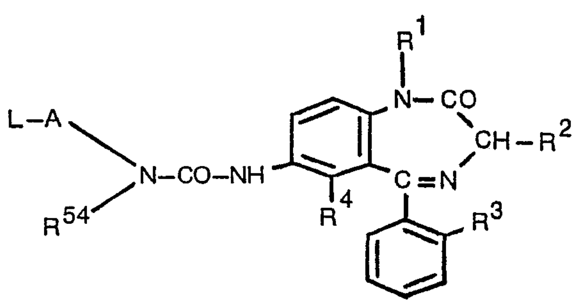

X

wherein $R^1$, $R^2$, $R^3$ and $R^4$ have the above significance and $R^{54}$ signifies hydrogen or ($C_1$—$C_7$)-alkyl and L

signifies a halogen atom, an arylsulphonyloxy, alkylsulphonyloxy or a quaternary ammonium group, into the corresponding hydroxy or ($C_1$—$C_7$)-alkanoyloxy compound, or

g) ($C_1$—$C_7$) alkanoylating a benzodiazepine derivative of the general formula (Ia)

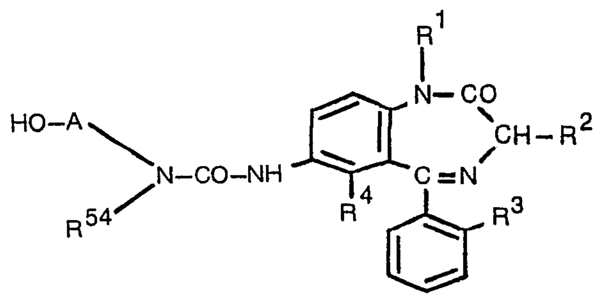

Ia

wherein $R^1$, $R^2$, $R^3$, $R^4$, $R^{54}$ and A have the above significance, or

h) hydrolytically opening the aziridine ring in a benzodiazepine derivative of the general formula (Ib)

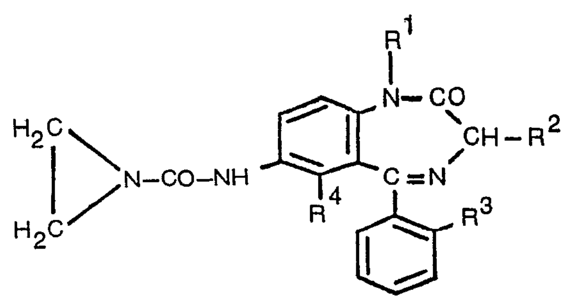

Ib

wherein $R^1$, $R^2$, $R^3$ and $R^4$ have the above significance, or

i) converting a benzodiazepine derivative of general formula (I) into a pharmaceutically acceptable acid addition salt.

2. Process according to claim 1, characterized in that variants a) to g) or i) are carried out.

3. Process according to claim 1 or 2, characterized in that $R^2$ signifies hydrogen, $R^3$ signifies fluorine and $R^4$ signifies hydrogen or chlorine.

4. Process according to claim 1 or 2, characterized in that $R^4$ and $R^5$ signify hydrogen and $R^6$ signifies ($C_2$—$C_7$)-alkyl or hydroxy-($C_2$—$C_7$)-alkyl.

5. Process according to claim 1 or 2, characterized in that $R^4$ signifies halogen and either $R^5$ signifies hydrogen and $R^6$ signifies ($C_2$—$C_7$)-alkyl or hydroxy-($C_2$—$C_7$)-alkyl or $R^5$ and $R^6$ each signify ($C_1$—$C_7$)-alkyl or $R^5$ and $R^6$ together with the nitrogen atom signify a 3- to 7-membered heterocycle without an additional hetero atom or a N-methylpiperazine, thiazolidine, morpholine or thiomorpholine residue.

6. Process according to claim 5, characterized in that $R^4$ signifies bromine or chlorine.

7. Process according to any one of claims 4 to 6, characterized in that $R^3$ signifies fluorine or chlorine.

8. Process according to claim 3, characterized in that 1 - [5 - (o - fluorophenyl) - 2,3 - dihydro- 1 - methyl - 2 - oxo - 1H - 1,4 - benzodiazepin - 7 - yl] - 3 - (2 - hydroxyethyl)urea is manufactured.

9. Process according to claim 3, characterized in that 1 - [5 - (o - fluorophenyl) - 2,3 - dihydro - 1 - methyl - 2 - oxo - 1H - 1,4 - benzodiazepin - 7 - yl] - 3 - methylurea is manufactured.

10. Process according to claim 3, characterized in that 1 - [5 - (o - fluorophenyl) - 2,3 - dihydro - 1 - methyl - 2 - oxo - 1H - 1,4 - benzodiazepin - 7 - yl] - 3 - (2 - hydroxy - 1 - methylethyl)urea is manufactured.

11. Process according to claim 3, characterized in that N - [5 - (o - fluorophenyl) - 2,3 - dihydro - 1 - methyl - 2 - oxo - 1H - 1,4 - benzodiazepin - 7 - yl] - 1 - pyrrolidinecarboxamide is manufactured.

12. Process according to claim 3, characterized in that 1 - [6 - chloro - 5 - (o - fluorophenyl) - 2,3 - dihydro - 1 - methyl - 2 - oxo - 1H - 1,4 - benzodiazepin - 7 - yl] - 3 - methylurea is manufactured.

13. Process according to claim 3, characterized in that 1 - n - butyl - 3 - [6 - chloro - 5 - (o - fluorophenyl) - 2,3 - dihydro - 1 - methyl - 2 - oxo - 1H - 1,4 - benzodiazepin - 7 - yl]urea is manufactured.

14. Process according to claim 3, characterized in that 3 - [6 - chloro - 5 - (o - fluorophenyl) - 2,3 - dihydro - 1 - methyl - 2 - oxo - 1H - 1,4 - benzodiazepin - 7 - yl] - 1 - ethyl - 1 - methylurea is manufactured.

15. Process according to claim 6, characterized in that 1 - [6 - Bromo - 5 - (o - chlorophenyl) - 2,3 - dihydro - 1,3 - dimethyl - 2 - oxo - 1H - 1,4 - benzodiazepin - 7 - yl] - 3 - (2 - hydroxyethyl)urea is manufactured.

16. Process according to claim 6, characterized in that N - [6 - chloro - 5 - (o - fluorophenyl) - 2,3 - dihydro - 1 - methyl - 2 - oxo - 1H - 1,4 - benzodiazepin - 7 - yl] - 4 - morpholinecarboxamide is manufactured.

17. Process according to claim 6, characterized in that 1 - [6 - bromo - 5 - (o - fluorophenyl) - 2,3 - dihydro - 1,3 - dimethyl - 2 - oxo - 1H - 1,4 - benzodiazepin - 7 - yl] - 3 - (2 - hydroxyethyl)urea is manufactured.

18. Process according to claim 6, characterized in that 1 - [6 - chloro - 5 - (o - chlorophenyl) - 2,3 - dihydro - 1,3 - dimethyl - 2 - oxo - 1H - 1,4 - benzodiazepin - 7 - yl] - 3 - (2 - hydroxyethyl)urea is manufactured.